(19) Europäisches Patentamt
European Patent Office
Office européen des brevets

(11) EP 2 068 379 B1

(12) EUROPEAN PATENT SPECIFICATION

(45) Date of publication and mention of the grant of the patent:
**26.12.2012 Bulletin 2012/52**

(21) Application number: **07807084.4**

(22) Date of filing: **11.09.2007**

(51) Int Cl.:
***H01L 51/30*** *(2006.01)* ***C07D 333/72*** *(2006.01)*
***C09K 11/06*** *(2006.01)* ***H01L 21/336*** *(2006.01)*
***H01L 29/786*** *(2006.01)* ***H01L 51/05*** *(2006.01)*
***H01L 51/40*** *(2006.01)* ***H01L 51/50*** *(2006.01)*

(86) International application number:
**PCT/JP2007/067675**

(87) International publication number:
**WO 2008/032715 (20.03.2008 Gazette 2008/12)**

# (54) ORGANIC SEMICONDUCTOR MATERIAL, ORGANIC SEMICONDUCTOR DEVICE USING THE SAME, AND THEIR PRODUCTION METHODS

ORGANISCHES HALBLEITERMATERIAL, ORGANISCHES HALBLEITERBAUELEMENT DAMIT UND HERSTELLUNGSVERFAHREN DAFÜR

MATÉRIAU SEMI-CONDUCTEUR ORGANIQUE, DISPOSITIF SEMI-CONDUCTEUR ORGANIQUE UTILISANT CELUI-CI ET LEURS PROCÉDÉS DE FABRICATION

(84) Designated Contracting States:
**DE FR GB**

(30) Priority: **12.09.2006 JP 2006247383**

(43) Date of publication of application:
**10.06.2009 Bulletin 2009/24**

(73) Proprietors:
- **Hiroshima University**
  **Hiroshima 739-8511 (JP)**
- **TOKAI UNIVERSITY EDUCATIONAL SYSTEM**
  **Shibuya-ku,**
  **Tokyo 151-0063 (JP)**
- **NIPPON KAYAKU KABUSHIKI KAISHA**
  **Tokyo 102-8172 (JP)**

(72) Inventors:
- **TAKIMIYA, Kazuo,**
  **c/o Graduate School of Engineering,**
  **Hiroshima University**
  **Horoshima 739-8527 (JP)**
- **KUNUGI, Yoshihito,**
  **c/o Tokai University**
  **Kanagawa 259-1292 (JP)**

(74) Representative: **Bentz, Jean-Paul et al**
**Novagraaf Technologies**
**122 Rue Edouard Vaillant**
**92593 Levallois-Perret Cedex (FR)**

(56) References cited:
**WO-A1-2006/095634 JP-A- 2005 140 948**
**JP-B2- 07 039 477**

- **T.M. PAPPENFUS: "A ¶-Stacking Terthiophene-Based Quinodimethane Is an n-Channel Conductor in a Thin Film Transistor", J. AM. CHEM. SOC., vol. 124, 29 March 2002 (2002-03-29), pages 4184-4185, XP7918080,**
- **S. HANDA ET AL.: "Solution-Processible n-Channel Organic Field-Effect Transistors Based on Dicyanomethylene-Substituted Terthienoquinoid Derivative", J. AM. CHEM. SOC., vol. 129, 31 August 2007 (2007-08-31), pages 11684-11685, XP7918082,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 068 379 B1

**Description**

Technical Field

The present invention relates to an organic semiconductor material, an organic semiconductor device using the organic semiconductor material, and production methods of the organic semiconductor material and the organic semiconductor device using the organic semiconductor material. More particularly, the present invention relates to (i) an organic semiconductor material to which a welding process is applicable, and which enables stable n-type transistor operation in the atmosphere, (ii) an organic semiconductor device using the organic semiconductor material, and (iii) production methods of the organic semiconductor material and the organic semiconductor device using the organic semiconductor material.

Background art

In recent years, thin film devices using an organic semiconductor material, such as organic EL devices, organic FET (Field Effect Transistor) devices, and organic thin film photoelectric converting devices have been considered noteworthy and these thin film devices have been put into practical use.

In basic properties of the organic semiconductor material used in the thin film devices, carrier mobility is important. For example, in the organic EL devices, the carrier mobility is important for high-efficient light emission and driving at a low voltage since the carrier mobility affects efficiency of charge transport. Further, in the organic FET devices, the carrier mobility is important for practical uses of the organic FET devices since the carrier mobility directly affects a switching speed and performances of a device to be driven.

Moreover, in order that characteristics of the organic semiconductor material used in the thin film devices is effectively utilized, application of a welding process is important in which method the organic semiconductor material dissolved in an organic solvent of various kinds is applied by use of an applying method, a spin-coating method, an inkjet method, or the like method so as to produce a thin film device. With the use of the welding process, it is possible to use paper, plastic, or the like as a substrate, which allows a resultant organic semiconductor device to be lightweight and flexible. Further, the use of the welding process enables (i) a simplification of production processes, (ii) a significant decrease in production cost of devices and the like necessary for production, and (iii) production of a large-area organic semiconductor device, compared with a case where conventionally-used vacuum vapor deposition is applied. The use of the welding process requires an organic semiconductor material that is soluble in a solvent and is stably in the atmosphere.

Conventionally, an organic semiconductor material (hereinafter referred to as a "p-type material") using a p-type (hole transport) transistor and an organic semiconductor material (hereinafter referred to as an "n-type material") using an n-type (electron transport) transistor have been known as organic semiconductor materials, similarly to inorganic semiconductor materials. For example, the p-type material and the n-type material are necessary for forming a logical circuit such as a CMOS (Complementary Metal Oxide Semiconductor) or the like.

Until now, a lot of studies on the p-type material have been conducted and materials that are soluble and allow stable driving in the atmosphere have been reported. On the other hand, in regard to the n-type material, with the use of vapor deposition, it is possible to obtain carrier mobility that is not so much different from that of the p-type material. From this reason, the n-type material for the vapor deposition has been studied a lot.

Non Patent Literature 1 discloses an organic semiconductor material having a structure represented by the following general formula (11).

Chem. 1

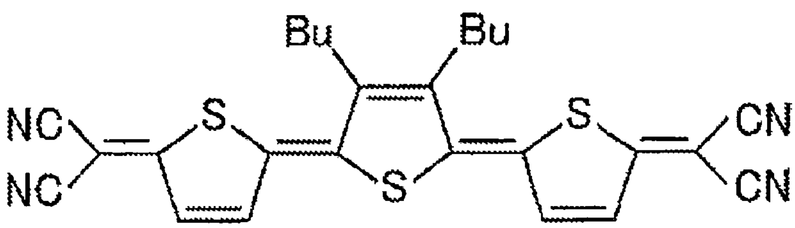

...(11)

The organic semiconductor material can be used in an organic FET device. The vapor deposition is applied to the organic semiconductor material and the organic semiconductor material has characteristics of both p-type and n-type materials.

Further, Non Patent Literature 2 discloses an organic semiconductor material to which the vapor deposition is applied and which has characteristics of both p-type and n-type materials.

Moreover, Non Patent Literature 3 discloses an organic semiconductor material having a structure represented by the following general formula (12).

Chem. 2

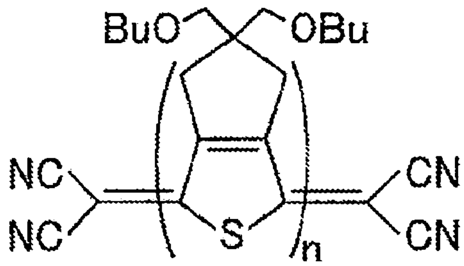

...(12)

The organic semiconductor material has solubility and characteristics of both p-type and n-type materials.

Citation List

Non Patent Literature 1

Pappenfus, T.M.: Chesterfield, R.J.; Friesbie, C.D.; Mann, K.R.; Casado, J.; Raff, J.D.; Miller, L.L.; J. Am. Chem. Soc. 2002, 124,4184-4185.

Non Patent Literature 2

Chesterfield, R.J.; Newman, C.R.; Pappenfus, T.M.; Ewbank, P.C.; Haukaas, M.H.; Mann, K.R.; Miller, L.L.; Frisbie, C.D. Adv. Mater. 2003, 15, 1278-1282.

Non Patent Literature 3

Extensive Quinoidal Oligothiophenes with Dicyanomethylene Groups at Terminal Positions as Highly Amphoteric Redox Molecules, T. Takahashi, K. Matsuoka, K. Takimiya, T. Otsubo, Y. Aso, J. Am. Chem. Soc. 2005, 127(25), 8928-8929.

Summary of Invention

However, there are few organic semiconductor materials that can be subjected to a welding process and allows a steady n-type transistor operation even in an atmosphere. In addition, it is extremely difficult to develop such a material. Therefore, such a material is not practically provided.

Organic semiconductor materials described in Non-Patent Literatures 1 and 2 have a quinoid structure, and therefore decrease in solubility in response to expansion of conjugation. This makes it difficult to purify and process a compound. Therefore, these organic semiconductor materials cannot serve as such a material that can be subjected to the welding process and allows a steady n-type transistor operation even in an atmosphere.

An organic semiconductor material described in Non-Patent Literature 3 includes a hexamer of heterocycles. Compared with the trimer of heterocycles described in Non-Patent Literature 1, the hexamer of heterocycles has a higher solubility but is poorer in FET characteristic (field-effect transistor characteristic) and conductivity. Therefore, the organic semiconductor material described in Non-Patent Literature 3 can serve as such a material that can be subjected to the welding process, however, does not sufficiently fulfill a characteristic of semiconductor material.

The present invention has been accomplished in view of the problems above, and an object of the present invention is to provide an organic semiconductor material that can be subjected to a welding process and allows a steady n-type transistor operation even in an atmosphere, an organic semiconductor device using the same, and their production methods.

As a result of diligent work on the object, which is to provide the organic semiconductor material that can be subjected to the welding process and allow a steady n-type transistor operation even in an atmosphere, the inventor of

the present invention uniquely found a new usage of a compound represented by the general formula (1). By this, the inventor accomplished the present invention.

In order to attain the object, an organic semiconductor material according to the present invention comprises a compound represented by the general formula (1):

Chem. 3

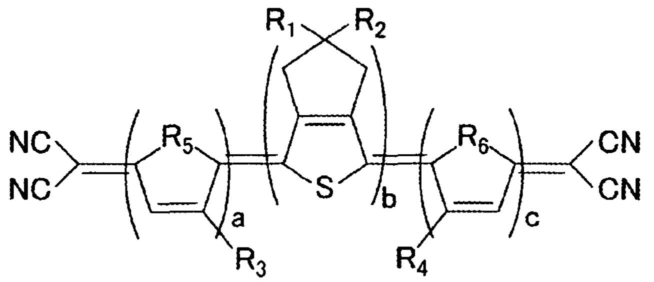

$\cdots$ (1)

where each of $R_1$ through $R_4$ independently is an atom or functional group selected from the group consisting of (i) a hydrogen atom, (ii) a halogen atom, (iii) an alkyl group in which one or more carbon atoms may be substituted by an oxygen atom and/or a sulfur atom and in which one or more hydrogen atoms may be substituted by a halogen atom, (iv) an alkyloxy group, (v) an alkylthio group, (vi) an aryl group, and (vii) an alkyl group in which one or more hydrogen atoms are substituted by an aryl group; each of $R_5$ and $R_6$ independently is a sulfur atom or a selenium atom; and a, b, and c are integers.

With the arrangement, in which the compound includes a cyclopentane ring-fused thiophene, it is possible to prevent a decrease in solubility caused by conjugation expansion of a quinoid structure. Further, with the arrangement, in which a non-cyclopentane ring-fused thiophene or selenophene is introduced to an end of the cyclopentane ring-fused thiophene, it is possible to prevent a decrease in intermolecular force caused by a large-size functional group included in the cyclopentane ring-fused thiophene. This makes it possible to prevent a decrease in conductivity.

With the arrangement, in which the compound has a polarity due to including a cyano group that attracts an electron, it is possible to perform an n-type transistor operation in which an electron serves as a carrier. Further, since a substituted cyclopentane ring-fused thiophene, which is soluble, is partially introduced, it becomes possible to densely fill a thin-film with the material. This allows a stable driving even in an atmosphere.

That is to say, the arrangement includes in combination (i) a cyclopentane ring-fused thiophene, which is soluble, (ii)a selenophene or a thiophene which is not derived from a condensed cyclopentane, and which increases a molecule overlap, and (iii) a cyano group, which has a polarity. This makes it possible to provide an organic semiconductor material that can be subjected to the welding process and allows a steady n-type transistor operation even in an atmosphere.

The organic semiconductor material according to the present invention is preferably arranged such that the carbon number of the functional group is 1 or more and 18 or less; and $a + b + c \leq 6$.

In the arrangement, the carbon number of the functional group is 1 or more and 18 or less. Since the functional group has a comparatively small carbon number, it is possible to make an entire molecule small in size even in a case where the compounds includes the functional group. This makes it possible to prevent a decrease in conductivity caused by a decrease in intermolecular force. By this, it is possible to uniformly form a film.

Further, since $a + b + c \leq 6$, which means that the compound has a relatively small molecular weight, it is possible to easily synthesize the compound.

The organic semiconductor material according to the present invention is preferably arranged such that $R_1$ and $R_2$ are an identical atom or functional group; $R_3$ and $R_4$ are an identical atom or functional group; $R_5$ and $R_6$ are an identical atom; and a = c.

In the arrangement, the compound is a symmetric molecule. In the symmetric molecule, a non-substituted thiophene or a selenophene can be connected, concurrently during synthesis of a molecular frame, to both ends of a cyclopentane ring-fused thiophene located in the center of the molecule. This makes easy to extend a thiophene chain and the like. Further, it is possible to synthesize a final product through a small number of synthesizing steps. In addition, use of the symmetric molecule as a semiconductor material reduces possibility of disarray of molecular orientation in the thin-film. This structure is desirable to achieve a high mobility.

The organic semiconductor material according to the present invention is preferably arranged such that in the

general formula (1), $R_5$ and $R_6$ are a sulfur atom.

The organic semiconductor material according to the present invention is preferably arranged such that in the general formula (1): $R_1$ and $R_2$ are a butoxymethyl group; $R_3$ and $R_4$ are a hydrogen atom; and $R_5$ and $R_6$ are a sulfur atom.

With the arrangement, since the butoxymethyl group is easily dissolved in an organic solvent, it is possible to more surely prevent a decrease in solubility. Further, since the hydrogen atom is small in size, it is possible to make an entire molecule small in size. This makes it possible to prevent a decrease in conductivity caused by a decrease in intermolecular force. By this, it is possible to uniformly form a film.

The organic semiconductor material according to the present invention is preferably arranged such that in the general formula (1): $R_1$ and $R_2$ are a butoxymethyl group; $R_3$ and $R_4$ are a methyl group; and $R_5$ and $R_6$ are a sulfur atom.

The methyl group has a larger size than a hydrogen atom, but has a comparatively small size among general functional groups. Therefore, with the arrangement, it is possible to make an entire molecule small in size. This makes it possible to prevent a decrease in conductivity caused by a decrease in intermolecular force. By this, it is possible to uniformly form a film.

The organic semiconductor material according to the present invention is preferably arranged such that an electron mobility is $10^{-3}cm^2/Vs$ or more.

With the arrangement, it becomes possible to achieve a high mobility to such an extent that cannot be attained by a conventional n-type material that can be subjected to the welding process.

There has been a problem that an organic semiconductor device using a welding process is lower in electron mobility than an organic semiconductor device using a vapor deposition, and the electron mobility of the organic semiconductor device using the welding process is approximately $10^{-5}cm^2/Vs$ or more and $10^{-4}cm^2/Vs$ or less. In contrast, the organic semiconductor device using the organic semiconductor material of the present invention achieves an electron mobility of $10^{-3}cm^2/Vs$ or more. This means that the organic semiconductor device of the present invention achieves an extremely high electron mobility among n-type organic semiconductor devices that can perform in an atmosphere.

The organic semiconductor material according to the present invention is preferably an n-type transistor material.

With the arrangement, in which an electron freely moves in a transporting layer as a carrier, it becomes easy to form an electrode and the like when producing the semiconductor device.

An organic semiconductor material according to the present invention is preferably a bipolar transistor material that includes the organic semiconductor material and a p-type transistor material.

With the arrangement, it becomes possible to produce a logical circuit such as CMOS by a coating process. This allows a wide range of application of the organic semiconductor material to the semiconductor device.

The organic semiconductor material according to the present invention is preferably drivable in an atmosphere. The organic semiconductor material is preferably drivable at a normal temperature. The organic semiconductor material is preferably drivable under a normal pressure.

A conventional n-type material that can be subjected to the welding process cannot be driven in an atmosphere. In an n-type material of the present invention, in which the compound has a polarity due to a cyano group that attracts an electron, the electron is likely to transfer to the cyano group. This causes a high electron mobility. This allows driving of the material even in the atmosphere, and further, driving at a normal temperature and/or under a normal pressure, which means a normal condition of environment. Therefore, the material of the present invention can be considered as important in putting an organic transistor into practical use.

The organic semiconductor material is preferably such that annealing is applied thereto.

With the arrangement, it is possible to improve FET characteristics such as electron mobility. This allows a wide range of application of the organic semiconductor material to the semiconductor device.

An organic semiconductor device according to the present invention comprises the organic semiconductor material.

The organic semiconductor material achieves an excellent solubility in an organic solvent. This makes it possible to produce the organic semiconductor device by using a welding process. Further, the organic semiconductor material is excellent in characteristics such as conductivity. Therefore, the organic semiconductor material can provide such an organic semiconductor device that has a large area and is flexible, light in weight, and excellent in characteristics.

The organic semiconductor device according to the present invention is preferably a light-emitting device that includes an organic carrier transporting layer and/or a light-emitting layer.

The organic carrier transporting layer makes it possible for a carrier to freely move in the transporting layer. By this, it becomes easy to form an electrode and the like when producing the light-emitting device.

Further, the light-emitting layer provides a site where holes injected from a positive electrode recombine with electrons injected from a negative electrode at the time of voltage application.

Further, with the arrangement, in which the organic carrier transporting layer and the light-emitting layer are included, the carrier freely moves in the transporting layer so as to transport, to the light emitting layer, the holes injected from the positive electrode and the electrons injected from the negative electrode. This allows the organic semiconductor device to emit light.

The organic semiconductor device according to the present invention is preferably a thin-film transistor that includes an organic semiconductor layer.

The thin-film transistor, which has a thin film, can realize reduction in size and weight of the organic semiconductor device. It is also possible to simplify a producing method and to significantly reduce a cost of devices used in producing the semiconductor device.

A method according to the present invention is preferably a method for producing the organic semiconductor device, comprising providing an organic semiconductor material on a substrate by a method of application, spin coating, or inkjet.

With the arrangement, it is possible to simplify a film-forming process and reduce an amount of semiconductor material to use, compared to a case of using a vapor deposition. Further, since the organic semiconductor material of the present invention is used, it is possible to produce, at a low cost, such an organic semiconductor device that has a large area and is flexible and light in weight. Furthermore, the organic semiconductor device can be produced by a simple producing method and devices of a significant lower cost.

A method for producing an organic semiconductor material is preferably a method comprising:

producing a compound represented by the general formula (4):

Chem. 6

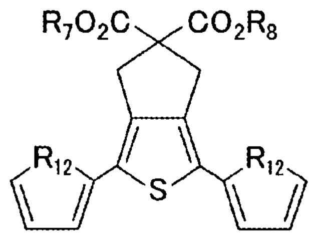

· · · (4)

where each of $R_7$ and $R_8$ independently is an alkyl group; and $R_{12}$ is a sulfur atom or selenium atom,
the compound represented by the general formula (4), being produced by reacting a compound represented by the general formula (2) and a compound represented by the general formula (3):

Chem. 4

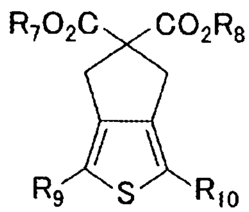

· · · (2)

where each of $R_7$ and $R_8$ independently is an alkyl group; and each of $R_9$ and $R_{10}$ independently is a halogen atom;

Chem. 5

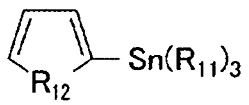

· · · (3)

where $R_{11}$ is an alkyl group; and $R_{12}$ is a sulfur atom or a selenium atom; and
thereafter reducing an ester group of the compound represented by the general formula (4). It is preferable to arrange the method so that, in the general formula (4), $R_{12}$ is a sulfur atom.

In a conventional method for production of organic semiconductor material, a cyclopentane ring-fused thiophene

ring which includes a soluble substituted group and is located in the center is synthesized at first and thereafter alcoholized by a reduction of ester group, and then a hydroxyl group of the thiophene is etherified. At the stage of the reduction, two halogen atoms in the thiophene ring are substituted with hydrogen atoms. Therefore, it is necessary to re-substitute the hydrogen atoms with halogen atoms. In the method of the present invention, which carries out at first a coupling of a compound represented by the general formula (2) and a compound represented by the general formula (3), it is unnecessary to carry out re-substituting the hydrogen atoms with the halogen atoms.

When changing a chain length of ether, a compound that is produced by a reduction of ester group of a compound represented by the general formula (4) can be used as a common intermediate. This allows an easy production of various derivatives. As a result, it becomes possible to synthesize an n-hexyl derivative, an n-octyl derivative, and n-decyl derivative in addition to an n-butyl derivative.

The method according to the present invention is preferably arranged such that in the general formula (2), $R_7$ and $R_8$ are an ethyl group; and $R_9$ and $R_{10}$ are a bromine atom; in the general formula (3), $R_{11}$ is a butyl group; and $R_{12}$ is a sulfur atom; and in the general formula (4), $R_7$ and $R_8$ are an ethyl group; and $R_{12}$ is a sulfur atom.

Additional objects, features, and strengths of the present invention will be made clear by the description below. Further, the advantages of the present invention will be evident from the following explanation in reference to the drawings.

Brief Description of Drawings

Fig. 1
Fig. 1 shows MS and IR charts of an organic semiconductor material according to one embodiment of the present invention.
Fig. 2
Fig. 2 shows a $^1$H-NMR chart of an organic semiconductor material according to one embodiment of the present invention.
Fig. 3
Fig. 3 shows structures of geometric isomers of an organic semiconductor material according to one embodiment of the present invention.
Fig. 4
Fig. 4 shows MS and IR charts of an organic semiconductor material according to one embodiment of the present invention.
Fig. 5
Fig. 5 shows a $^1$H-NMR chart of an organic semiconductor material according to one embodiment of the present invention.
Fig. 6
Fig. 6 is a graph showing relations between electron absorption spectrum and absorption wavelength in organic semiconductor materials according to one embodiment of the present invention.
Fig. 7
Fig. 7 is a graph showing energy of HOMO-LUMO in organic semiconductor materials according to one embodiment of the present invention.
Fig. 8
Fig. 8 is a side view and a top view illustrating an FET device produced for evaluating FET characteristics of an organic semiconductor material according to one embodiment of the present invention.
Fig. 9
Fig. 9 is graphs showing results of FET characteristics of an organic semiconductor material according to one embodiment of the present invention.
Fig. 10
Fig. 10 is graphs showing results of FET characteristics of an organic semiconductor material according to one embodiment of the present invention.
Fig. 11
Fig. 11 is graphs showing results of FET characteristics of an organic semiconductor material according to Non Patent Literature 3.
Fig. 12
Fig. 12 is a perspective view illustrating an OTFT device produced for evaluating FET characteristics of an organic semiconductor material according to one embodiment of the present invention.
Fig. 13
Fig. 13 is graphs showing results of FET characteristics of an organic semiconductor material according to one

embodiment of the present invention.

Description of Embodiments

The following explains about the present invention in detail.

<Organic semiconductor material>

An organic semiconductor material of the present invention includes a compound represented by the following general formula (1):

Chem. 7

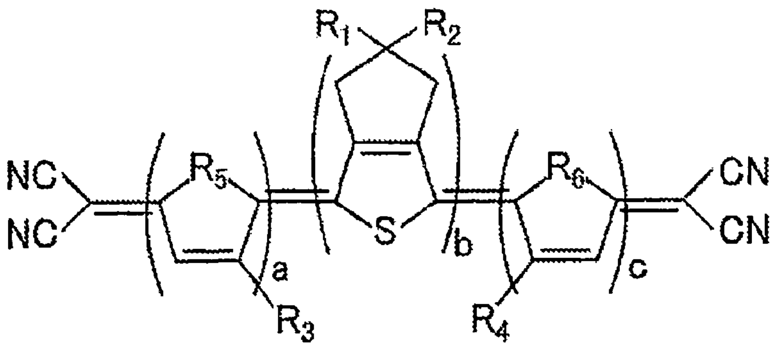

...(1)

where $R_1$ through $R_4$ independently are an atom or a functional group selected from the group consisting of (i) a hydrogen atom, (ii) a halogen atom, and (iii) an alkyl group in which one or more carbon atoms may be substituted with an oxygen atom and/or a sulfur atom and one or more hydrogen atoms may be substituted with a halogen atom, (iv) an alkyloxy group, (v) an alkylthio group, (vi) an aryl group, and (vii) an alkyl group in which one or more hydrogen atoms are substituted with an aryl group; $R_5$ and $R_6$ independently are a sulfur atom or a selenium atom; and a, b, and c respectively are integers.

The compound represented by the general formula (1) has a structure in which a cyclopentane ring-fused thiophene, thiophene or selenophene that is not fused to a cyclopentane ring, and polar cyano groups are combined. The cyclopentane ring-fused thiophene provides solubility to the compound, and thiophene or selenophene allows the compound to have a large intermolecular overlapping. This allows the use of a welding process, and further a stable n-type transistor operation in the atmosphere.

The organic semiconductor material may be made from only the compound represented by the general formula (1), or may additionally contain a substance other than the compound represented by the general formula (1), provided that characteristics of the compound represented by the general formula (1) are not disturbed. A method of forming such an organic semiconductor material additionally containing the substance other than the compound represented by the general formula (1) is not especially limited, and can be such that the compound represented by the general formula (1) and the substance other than that are stirred under heat application so that an organic semiconductor material is synthesized.

Conventionally known examples of such a substance encompass dopants such as: bases such as ammonia and organic amine; inorganic salts such as potassium iodine and sodium iodine; and metal atoms such as sodium, potassium, and the like.

The addition of the dopant adjusts electrical conductivity of the organic semiconductor material. The doping can be performed in any way, and may be carried out in a conventionally-known manner. The doping can be carried out by, for example, gas-phase doping, liquid-phase doping, solid-phase doping, or the like, but is not limited to these methods.

The cyclopentane ring-fused thiophene is a fused ring in which thiophene is fused to cyclopentane. The fused ring is a cyclic structure in which a cyclic compound has two or more rings and the rings share two or more atoms with each other. The cyclopentane ring-fused thiophene of the present invention is a bicyclic fused ring.

The thiophene or selenophene that is not fused to a cyclopentane ring is thiophene or selenophene that is not fused to cyclopentane.

Since the compound represented by the general formula (1) has the cyclopentane ring-fused thiophene and the thiophene or selenophene that is not fused to a cyclopentane ring, it is possible to prevent a decrease in resolvability and conductivity.

The compound has a geometric isomer which varies depending on how to combine the cyclopentane ring-fused thiophene and the thiophene or selenophene that is not fused to a cyclopentane ring. The geometric isomer is not limited a trans-trans conformation in which thiophenes or selenophenes placed at both ends are on the opposite side to a thiophene placed at a center in the general formula (1), and may be a trans-cis conformation in which one of the thiophenes or selenophenes at both ends is on the same side as the thiophene at the center and the other one of the thiophenes or selenophenes at both ends is on the opposite side to the thiophene at the center, or a cis-cis conformation in which the thiophenes or selenophenes at both ends are on the same side as the thiophene at the center. Any of the conformations are stable structures.

Among these conformations, the trans-trans conformation is most stable because a distance between $R_1$ and $R_3$ and a distance between $R_2$ and $R_4$ are large in the compound represented by the general formula (1). The compound may be constituted by a single isomer or a combination of any of the foregoing geometric isomers.

The cyano group is a substituent in which a carbon atom and a nitrogen atom are triple-bonded. The cyano group attracts electrons and stabilizes negative charge on molecules. Examples of a compound having the cyano group encompass nitrile and the like.

The halogen atom may be fluorine, chlorine, bromine, iodine, or the like, but is not limited to these. Among these halogens, the fluorine and the chlorine are preferably used because they have high electronegativity and a small atomic radius.

Further, the alkyl group is such that one or more carbon atoms may be substituted with an oxygen atom and/or a sulfur atom and one or more hydrogen atoms may be substituted with a halogen atom.

The alkyl group is not especially limited provided that the alkyl group is straight, branched, or cyclic. The alkyl group may be methyl, ethyl, n-propyl, isopropyl, n-butyl, n-pentyl, isopentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, or the like. However, the alkyl group is not limited to these groups. Among these alkyl groups, the n-pentyl group is preferably used because the n-pentyl group is easily dissolved in an organic solvent.

Examples of the alkyl group in which one or more carbon atoms are substituted with an oxygen atom and/or a sulfur atom encompasses an alkyl group in which one or more carbon atoms are substituted with an oxygen atom, an alkyl group in which one or more carbon atoms are substituted with a sulfur atom, and an alkyl group in which one or more carbon atoms are substituted with an oxygen atom and a sulfur atom. For example, the alkyl group may be n-butoxymethyl, n-butoxyethyl, n-butoxypropyl, n-butylthiomethyl, n-butylthioethyl, n-butylthiopropyl, or the like, but is not limited to these. Among these alkyl groups, the n-butoxymethyl group is preferably used because the n-butoxymethyl group is easily dissolved in an organic solvent.

The alkyl group in which one or more hydrogen atoms are substituted with a halogen atom may be methyl chloride group, ethyl chloride group, n-propyl chloride group, or the like, but is not limited to these. Among these alkyl groups, the ethyl chloride group is preferably used because a reagent of the ethyl chloride group is easily available, and the ethyl chloride group is easily synthesized and refined.

The alkyloxy group may be methoxy group, ethoxy group, n-propoxy group, isopropoxy group, n-butoxy group, n-pentyloxy group, or the like, but is not limited to these. Among these alkyloxy group, the n-butoxy group is preferably used because a reagent of the n-butoxy group is easily available, and the n-butoxy group is easily synthesized and refined.

The alkylthio group may be methylthio group, ethylthio group, n-propylthio group, n-butylthio group, n-pentylthio group, or the like, but is not limited to these. Among these alkylthio groups, the n-butylthio group is preferably used because a reagent of the n-butylthio group is easily available and the n-butylthio group has appropriate solubility.

The aryl group may be phenyl group, tolyl group, xylyl group, cumenyl group, naphthyl group, or the like, but is not limited to these. Among these aryl groups, the phenyl group is preferably used because a reagent of the phenyl group is easily available, and the phenyl group is easily synthesized and refined.

The alkyl group in which one or more hydrogen atoms are substituted with an aryl group may be benzyl group, benzhydryl group, trityl group, phenethyl group, or the like, but is not limited to these. Among these alkyl groups, the benzyl group is preferably used because a reagent of the benzyl group is easily available, and the benzyl group is easily synthesized and refined.

A respective of $R_1$ through $R_4$ may be different atoms or functional groups. Further, two or three of $R_1$ through $R_4$ may be the same atoms or functional groups, and all of $R_1$ through $R_4$ may be the same atoms or functional groups. Especially, it is preferable that $R_1$ and $R_2$ be the same, and $R_3$ and $R_4$ be the same. This is because the number of processes of synthesizing can be reduced, and defects in a molecular arrangement are hardly caused in a thin film. The number of carbons in the functional groups is not especially limited, but is preferably not less than 1 but not more than 18 for reducing bulkiness of entire molecules.

Further, $R_1$ and $R_2$ are preferably a butoxymethyl group or a pentyl group, especially preferably a butoxymethyl group because these groups are easily dissolved in an organic solvent. Moreover, $R_3$ and $R_4$ are preferably a hydrogen atom or a methyl group, especially preferably a hydrogen atom because the size of molecules is small. On this account, it is especially preferable that $R_1$ and $R_2$ be a butoxymethyl group, and $R_3$ and $R_4$ be a hydrogen atom. Further, it is preferable that $R_5$ and $R_6$ be a sulfur atom because this attains excellent characteristics when an FET device is formed

with the use of the organic semiconductor material.

In the general formula (1), a, b, and c are not especially limited provided that they are integers, but are preferably as large integers as possible because this increases mobility of electrons. However, from the viewpoint of easiness of synthesis as a compound, it is preferable that the number of heterocycles, i.e., the number of thiophenes (a+b+c) be not more than 6. Further, it is preferable that a and c are the same integer because this allows a compound to be efficiently synthesized and easily refined.

The organic semiconductor material of the present invention is preferably such that the mobility of electrons is not less than $10^{-3}$ $cm^2/Vs$. In this case, as an n-type material to which the welding process is applicable, the organic semiconductor material has high mobility of electrons, which cannot be attained by a conventional technique. This allows the organic semiconductor material to be applied to a semiconductor device.

The mobility of electrons can be calculated from the following expression formula (a):

$$Id = Z\mu Ci(Vg-Vt)^2/2L \text{ ...(a).}$$

This expression formula (a) shows an electric characteristic of a carrier type generated in an organic semiconductor layer when a gate electric field is applied to $SiO_2$ as a gate dielectric. In the expression formula (a), Id indicates a saturated source/drain current value, Z indicates a channel width, Ci indicates a capacitance of an insulator, Vg indicates a gate potential, Vt indicates a threshold potential, L indicates a channel length, and $\mu$ indicates mobility ($cm^2/Vs$) to be determined. Further, Ci is determined by permittivity of a $SiO_2$ insulating film, Z and L are determined by a device structure of an FET device, Id and Vg are determined when a current value of the FET device is measured, and Vt can be calculated from Id and Vg. By substituting each values in the expression formula (a), mobility is calculated out with respect to a given gate potential. In a low electric field, an average transport speed of carriers (electrons or holes) increases in proportion to the electric field, and its proportionality coefficient referred to as the mobility.

The organic semiconductor material of the present invention can work as an n-type transistor material. In this case, since electrons as carriers freely move in a transporting layer, it is easy to form electrodes and the like from the organic semiconductor material of the present invention, in order to produce a semiconductor device. Further, when an n-type transistor material is combined with a p-type transistor material, the organic semiconductor material can work as a transistor material having characteristics of both n-type and p-type materials. In this case, it is possible to form a logical circuit such as a CMOS or the like by an applying method, with the result in that the organic semiconductor material can be widely applied to a semiconductor device.

As the p-type semiconductor material, conventionally known materials can be used. Examples of the p-type semiconductor material encompass GaAs, MnAs, ZnTe, MnTe, and the like.

The combination of the n-type transistor material and the p-type transistor material can be made, for example, by forming a junction of both materials unsymmetrically. More particularly, when an n-type semiconductor material has contact with a p-type semiconductor material, electrons move from the n-type semiconductor material to the p-type semiconductor material, and holes move from the p-type semiconductor material to the n-type semiconductor material. This causes collision between the electrons and the holes, thereby causing junction of the n-type semiconductor material and the p-type semiconductor material. As such the n-type semiconductor material and the p-type semiconductor material is combined.

The n-type transistor material denotes an organic semiconductor material for use in an n-type (electron transport) transistor, and the p-type transistor material denotes an organic semiconductor material for use in a p-type (hole transport) transistor. The electron transport denotes transport of electrons as carriers, and the hole transport denotes transport of holes as carriers. The carriers denote the substrate which can carry substances to be transported and move freely in a film.

It is preferable that the organic semiconductor material of the present invention allows driving in the atmosphere, especially at a room temperature and/or a normal pressure. In the organic semiconductor material of the present invention, the compound has a high electron-accepting property due to the cyano group that absorbs electrons. This increases the mobility of electrons, thereby resulting in that the transistor operation can be carried out in the atmosphere, and further the transistor operation can be carried out at a room temperature and/or a normal pressure, i.e., under standard environmental conditions.

The transistor operation is switching (between on and off) of carrier (electron or hole) transport according to a gate voltage. What is meant by "the transistor operation can be carried out in the atmosphere" is that the carrier transport can be switched without a sealant in the atmosphere. Further, when the carrier mobility is large, it can be said that a current passing the transistor while the transistor is on is large.

Furthermore, the room temperature denotes a standard environmental temperature, and is 25°C (298.15K). The normal pressure denotes a standard environmental pressure, and is 1 bar ($10^5$ Pa). What is meant by "the transistor operation can be carried out at a room temperature" is that the carrier transport can be switched over (between on and

off) by a gate voltage at 25°C (298.15K). What is meant by " the transistor operation can be carried out at a normal pressure" is that the carrier transport can be switched over (between on and off) by a gate voltage at 1 bar ($10^5$ Pa). Further, what is meant by "the transistor operation can be carried out at a room temperature and a normal pressure" is that the carrier transport can be switched over (between on and off) under the standard environmental conditions, i.e., at 25°C (298.15K) and 1 bar ($10^5$ Pa).

<Method for synthesizing organic semiconductor material>

A conventionally known method may be used as a method for synthesizing the compound, and for example may be synthesized by the following synthesis methods. A conventionally known organic synthesis reaction is usable for any synthesis that are not described in detail in the following description in terms of how to be carried out.

A specific example of a synthesis method of an organic semiconductor material made of a compound represented by a general formula (1) is as follows: a compound represented by the general formula (1) is synthesized in a reaction path shown in reaction formulae (A) through (C), more specifically, reaction formulae (D) through (R), or a reaction path shown in reaction formula (S), (T), or (U). The organic semiconductor material (i) may be the compound represented by the general formula (1) thus synthesized, or (ii) may contain the compound represented by the general formula (1) and another material as explained earlier in a degree in which the feature of the compound represented by the general formula (1) is not inhibited. Numbers shown in the reaction paths indicate compounds which correspond to the numbers, respectively.

More specifically, for example, methyl chloroacetate is reacted with sodium sulfide nonahydrate so as to synthesize dimethyl thiodiacetate (compound 1), and from this compound, cyclopentane ring-fused thiophene (compound 7) is synthesized. Thiophene is introduced at an end of the compound, so as to synthesize the compound represented by the general formula (1) (compounds 13, 18), and subsequently an organic semiconductor material containing the compound represented by the general formula (1) (the compounds 13, 18) is synthesized.

More specifically, for example, it is possible to synthesize the organic semiconductor material by methods (II), (III), and (VII) by use of a monomer unit obtained by method (I) described below. Moreover, for example, it is possible to synthesize the organic semiconductor material by methods (IV) through (VII).

<(I) Synthesis of Monomer Unit (cyclopentane ring-fused thiophene)>

It is not particularly limited in how the monomer unit of the component is synthesized. For example, the monomer unit may be synthesized from methyl chloroacetate and sodium sulfide nonahydrate by going through seven stages of reactions; this is represented by reaction formula (A) as follows:

Chem. 8

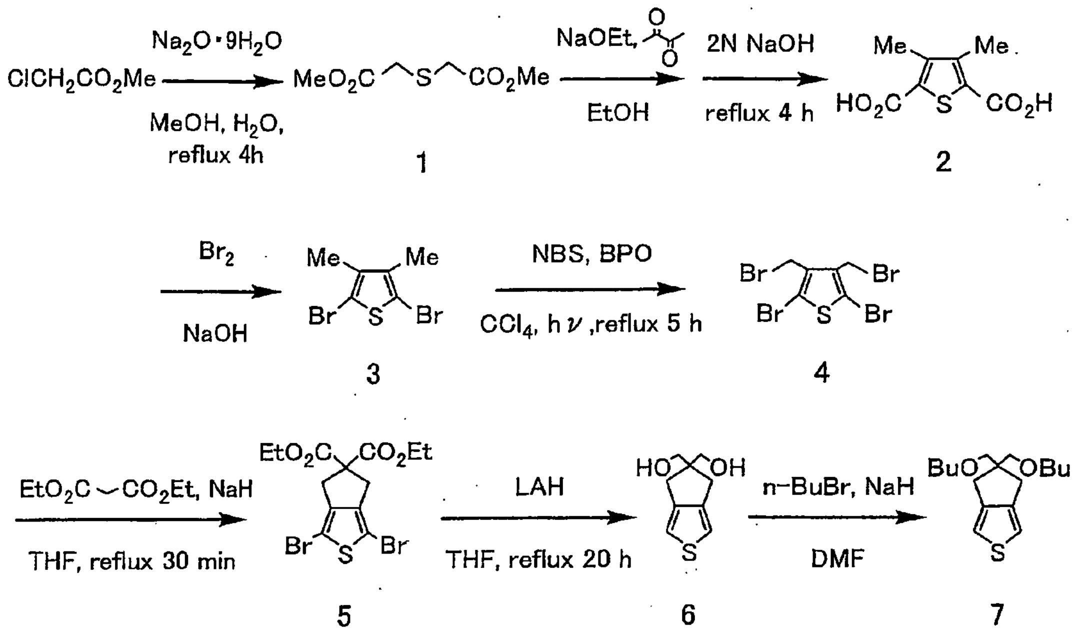


··· (A)

More specifically, a compound 1 is synthesized by reacting the methyl chloroacetate with the sodium sulfide nonahydrate as shown in reaction formula (D) below, and subsequently a conventionally known Hinsberg dehydrative cyclization reaction is carried out by use of 2,3-butandione so as to synthesize a compound 2:

Chem. 9

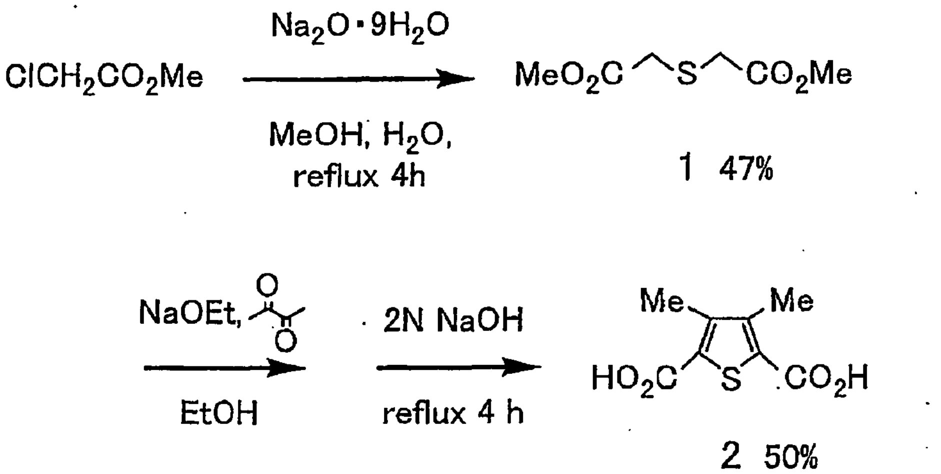


··· (D)

Thereafter, decarboxylation and bromination of a conventionally known method are carried out to the compound 2 so as to synthesize a compound 3, as shown in reaction formula (E) below, and further radical bromination is carried out by use of benzoyl peroxide as an initiator so as to synthesize a compound 4:

Chem. 10

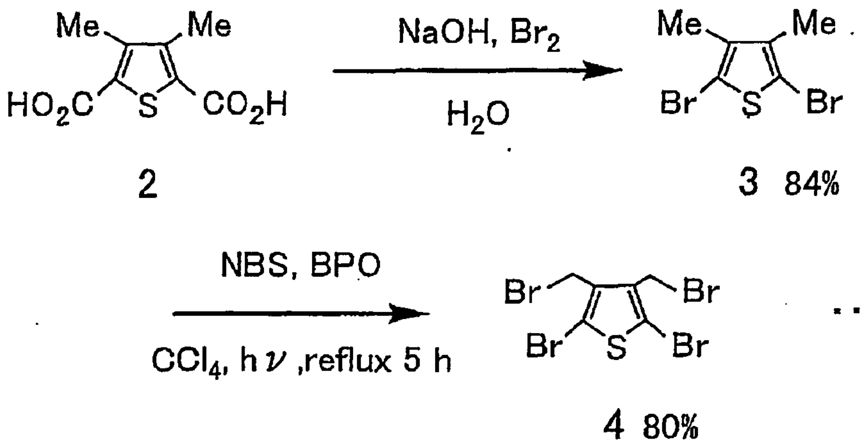


2

3 84%

4 80%

··· (E)

Subsequently, a conventionally known intramolecular cyclization of the compound 4 and diester malonate is carried out to synthesize a compound 5, as shown in reaction formula (F) as follows:

Chem. 11

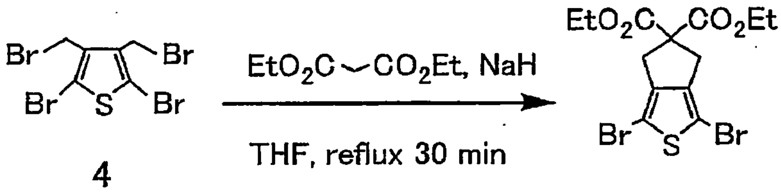


4

5

··· (F)

An ester group of the compound 5 is thereafter reduced by use of a conventionally known reducing agent of lithium aluminum hydride so as to synthesize a compound 6, as shown in reaction formula (G) below. Further, a conventionally known Williamson etherification is carried out by use of n-butylbromide so as to synthesize a compound 7, that is, cyclopentane ring-fused thiophene.

Chem. 12

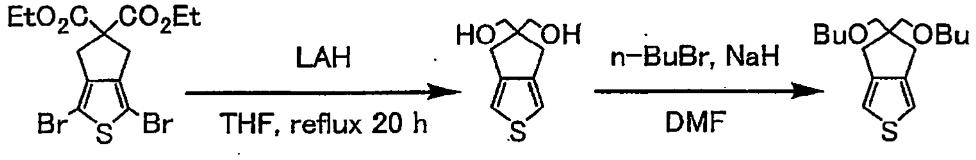


5

6 74%

7 97%

··· (G)

<(II) Synthesis of Compound represented by General Formula (1) (Compound represented by the general formula (1) where $R_1$ and $R_2$ are a butoxymethyl group; $R_3$ and $R_4$ are a hydrogen atom; $R_5$ and $R_6$ are a sulfur atom; and a, *b,* and *c* are 1) >

A compound represented by the general formula (1) where $R_1$ and $R_2$ are a butoxymethyl group; $R_3$ and $R_4$ are a hydrogen atom; $R_5$ and $R_6$ are a sulfur atom; and *a*, *b*, and *c* are 1 is synthesized by carrying out, in sequential

order, the following procedures to the compound 7, that is, the monomer unit: (i) bromination; (ii) Stille coupling; (iii) further bromination or iodination; and (iv) introduction of a dicyanomethyl group. This synthesis method is represented by the following reaction formula (B):

Chem. 13

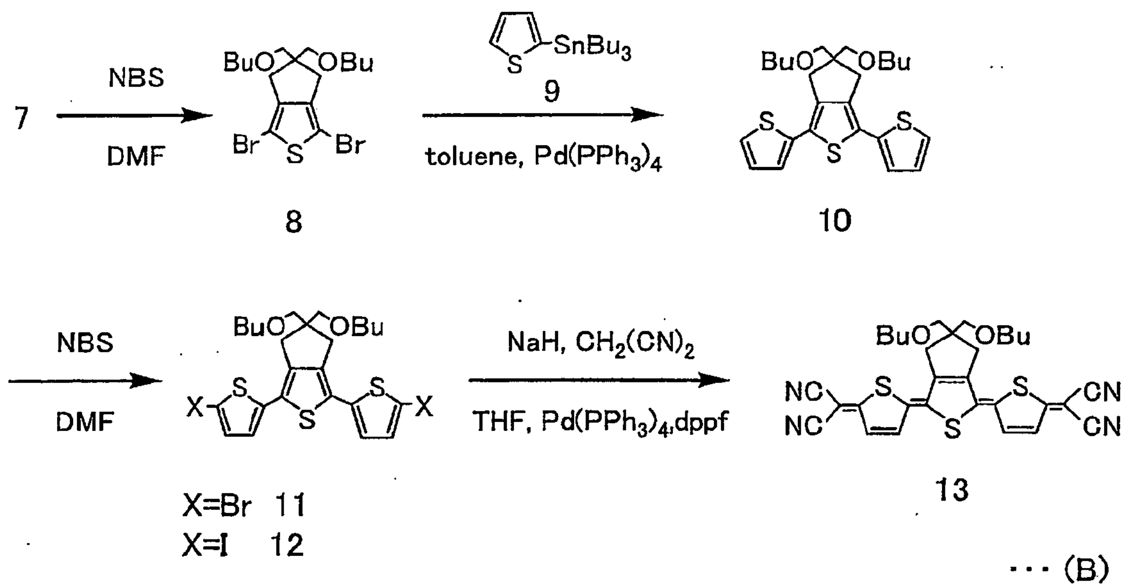


··· (B)

More specifically, the compound 7 is brominated by a conventionally known method by use of N-bromosuccinimide so as to synthesize a compound 8, as shown in reaction formula (H) as follows:

Chem. 14

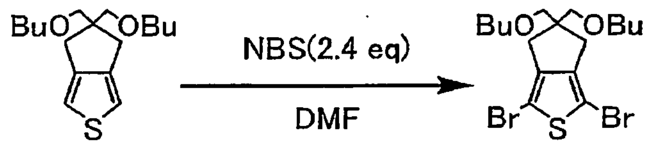


7 8 92%

··· (H)

Subsequently, as shown in reaction formula (I) below, the compound 8 is reacted with n-butyllithium and modified with tin in a conventionally known method by use of tributyltin chloride, so as to synthesize a compound 9.

Chem. 15

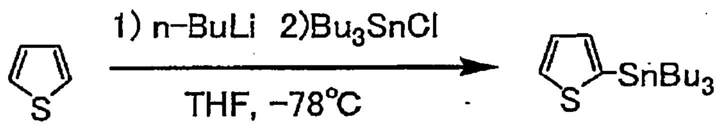


9 87%

··· (I)

As shown in reaction formula (J) below, Stille coupling reaction of the compound 8 and the compound 9 is then carried out in a conventionally known method, so as to synthesize a compound 10. Since it is difficult to separate the compound 10 and tin at this time, recrystallization is carried out in a conventionally known method by use of ethanol so as to purify the compound 10.

Chem. 16

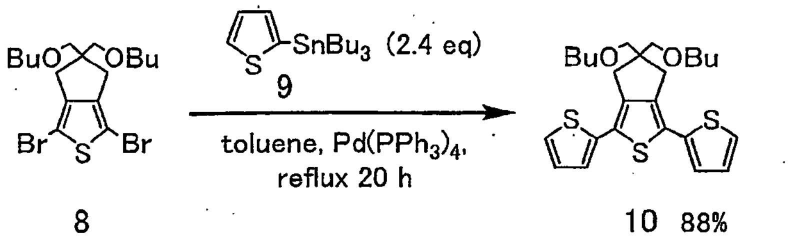


8 9 10 ··· (J)

A conventionally known bromination is thereafter carried out to the compound 10 by use of N-bromosuccinimide so that a compound 11 is synthesized, as shown in reaction formula (K) as follows:

Chem. 17

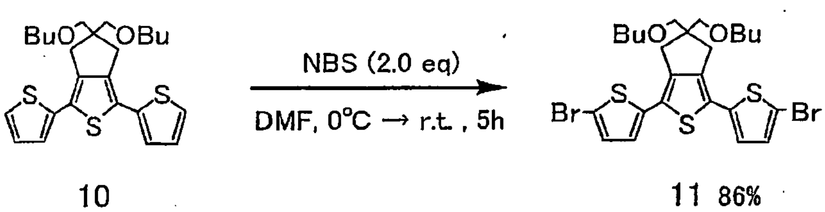


10 11 ··· (K)

Following this, a dicyanomethyl group is introduced to the compound 11 in a conventionally known method in order to synthesize a compound 13, as shown in reaction formula (L) as follows:

Chem. 18

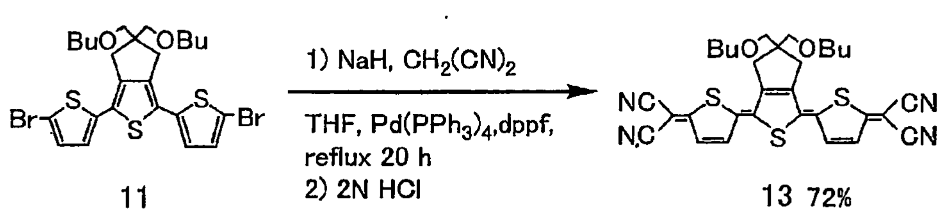


11 13 ··· (L)

Moreover, a conventionally known iodination is carried out to the compound 10 by use of N-iodosuccinimide, so as to synthesize a compound 12, as shown in reaction formula (M) as follows:

Chem. 19

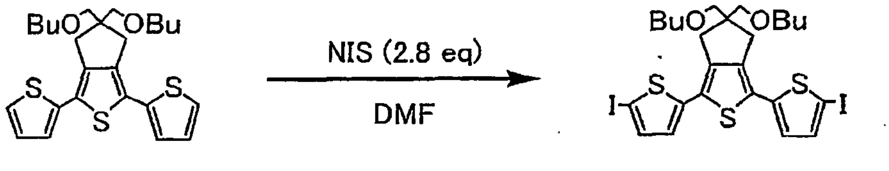


10

12 88%

··· (M)

Subsequently, as shown in reaction formula (N) below, a dicyanomethyl group is introduced to the compound 12 in a conventionally known method, which synthesizes the compound 13 which is the compound represented by the general formula (1) (the compound represented by the general formula (1) where $R_1$ and $R_2$ are a butoxymethyl group; $R_3$ and $R_4$ are a hydrogen atom; $R_5$ and $R_6$ are a sulfur atom; and *a*, *b,* and *c* are 1):

Chem. 20

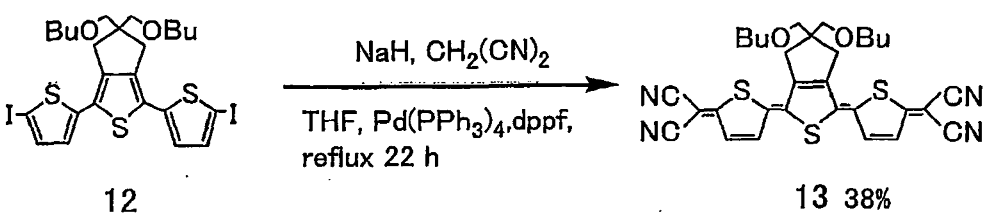


12

13 38%

··· (N)

<(III) Synthesis of Compound represented by General Formula (1) (Compound represented by the general formula (1) where $R_1$ and $R_2$ are a butoxymethyl group; $R_3$ and $R_4$ are a methyl group; $R_5$ and $R_6$ are a sulfur atom; and *a*, *b*, and *c* are 1)>

A compound represented by the general formula (1) where $R_1$ and $R_2$ are a butoxymethyl group; $R_3$ and $R_4$ are a methyl group; $R_5$ and $R_6$ are a sulfur atom; and *a*, *b*, and *c* are 1 may be synthesized via three types of synthesis routes. Synthesis methods of the compound are as shown in reaction formula (C) as follows:

Chem. 21

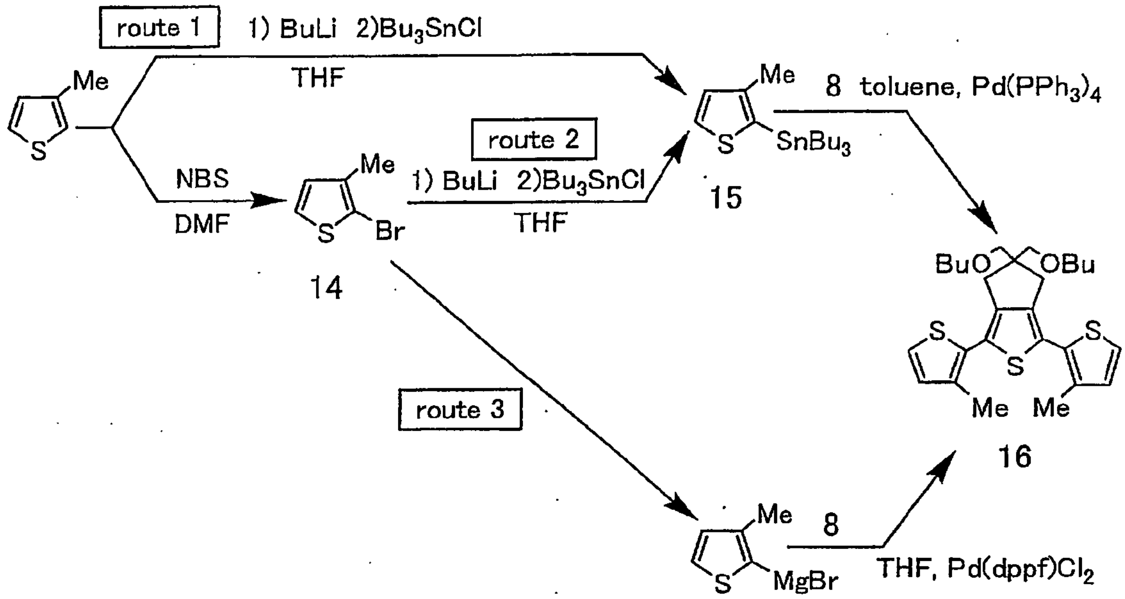


14

15

16

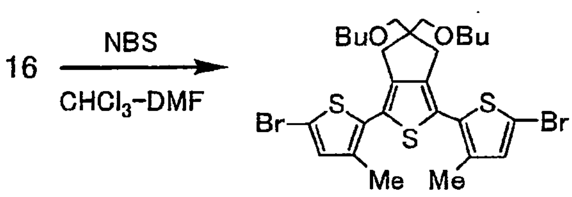


17

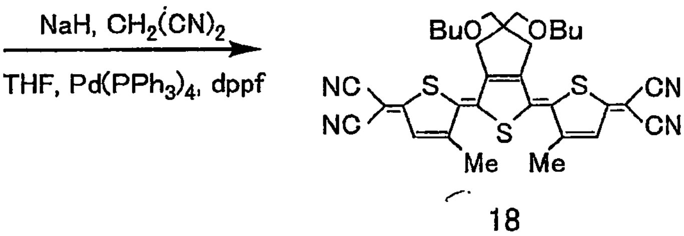


18

··· (C)

More specifically, as shown in reaction formula (O) below, route 1 synthesizes a compound 15 by first causing 3-methylthiophene to react with n-butyllithium in a conventionally known method and thereafter carrying out modification with tin by use of tributyltin chloride. Route 2 synthesizes the compound 15 by first synthesizing a compound 14, by carrying out a conventionally known bromination to 3-methylthiophene by use of N-bromosuccinimide. This compound 14 is reacted with n-butyllithium in a conventionally known method, and then modification with tin is carried out in a conventionally known method to the compound 14 thus reacted by slowly dropping tributyltin chloride, in order to synthesize the compound 15.

Chem. 22

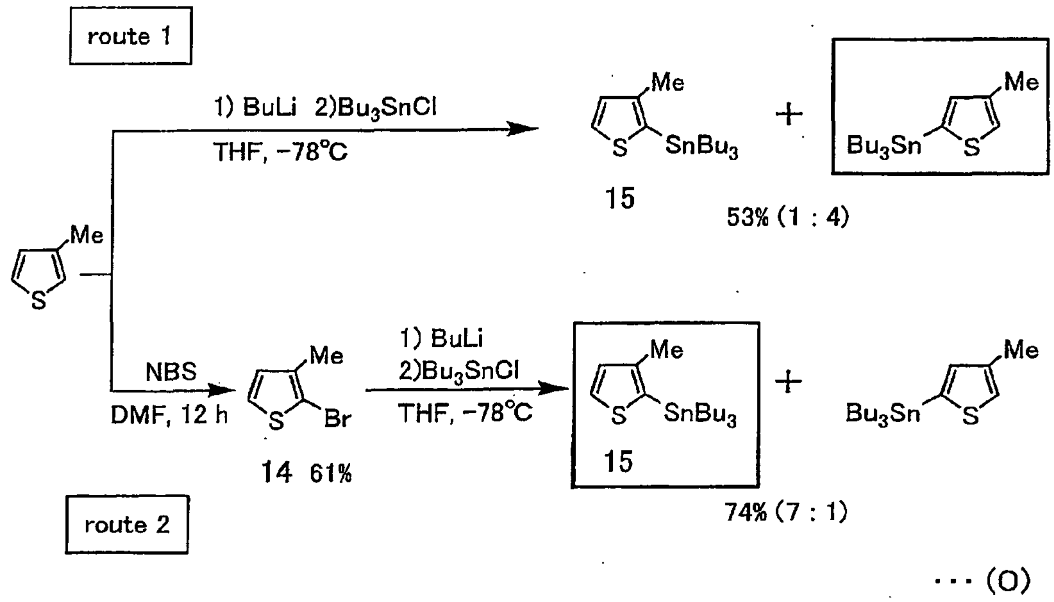


··· (O)

Route 3, as shown in reaction formula (P) below, synthesizes a compound 16 in any one of two conditions. Condition 1 is to carry out synthesis of the compound 16 by dropping, to the compound 8, Grinard reagent in which magnesium is excessively contained, with which ether is used as a solvent and Ni(dppp)$Cl_2$ is used as a catalyst. Condition 2 is to carry out synthesis of the compound 16 by dropping, to the compound 8, the Grinard reagent in which magnesium is excessively contained, with which THF is used as the solvent and Pd(dppf)$Cl_2$ is used as the catalyst.

Chem. 23

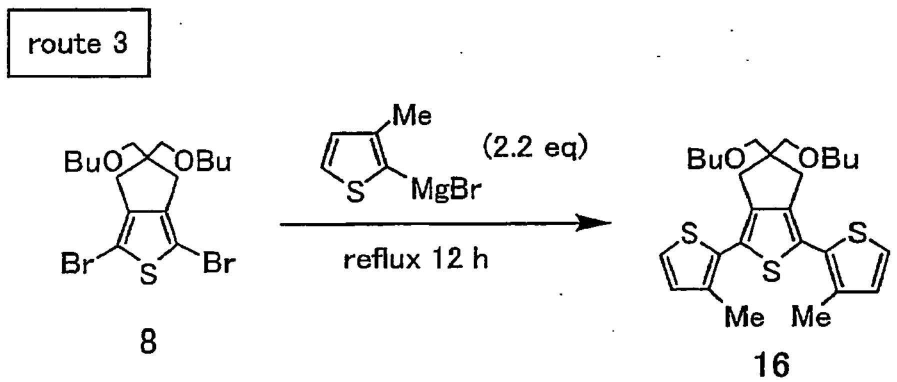


··· (P)

Thereafter, a conventionally known bromination is carried out to the compound 16 by use of N-bromosuccinimide, so as to synthesize a compound 17, as shown in reaction formula (Q) as follows:

Chem. 24

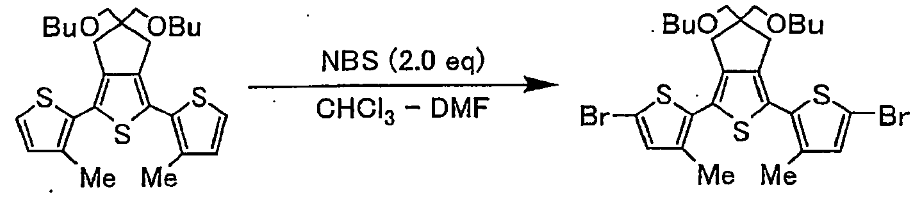


16

17 85%

··· (Q)

A dicyanomethyl group is subsequently introduced to the compound 17 in a conventionally known method as shown in reaction formula (R) below, so as to synthesize a compound 18 which is the compound represented by the general formula (1) (the compound represented by the general formula (1) where $R_1$ and $R_2$ are a butoxymethyl group; $R_3$ and $R_4$ are a methyl group; $R_5$ and $R_6$ are a sulfur atom; and *a*, *b*, and *c* are 1).

Chem. 25

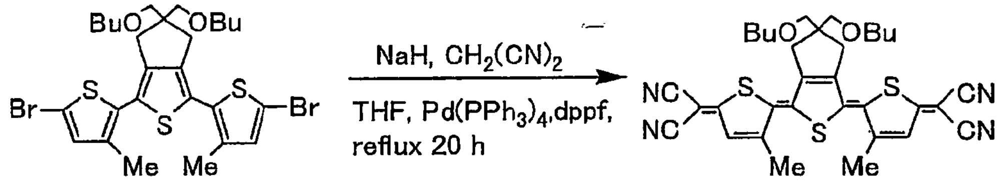


17

18 63%

··· (R)

Purification of the compound 18 may be carried by use of a conventionally known column chromatography or the like, and recrystallization of the compound 18 may be carried out by a conventionally known method by use of an acetone-acetonitrile mixed solvent. In the column chromatography, a conventionally known low-activity silica gel may be used.

<(IV) Synthesis of Compound represented by General Formula (1) (Compound represented by the general formula (1) where $R_1$ and $R_2$ are a hexyloxymethyl group, an octyloxymethyl group, or a decyloxymethyl group; $R_3$ and $R_4$ are a hydrogen atom; $R_5$ and $R_6$ are a sulfur atom; and *a, b,* and *c* are 1)>

A compound represented by the general formula (1) where $R_1$ and $R_2$ are a hexyloxymethyl group, an octyloxymethyl group, or a decyloxymethyl group; $R_3$ and $R_4$ are a hydrogen atom; $R_5$ and $R_6$ are a sulfur atom; and *a, b,* and *c* are 1 may be synthesized as shown in reaction formula (S) as follows:

Chem. 26

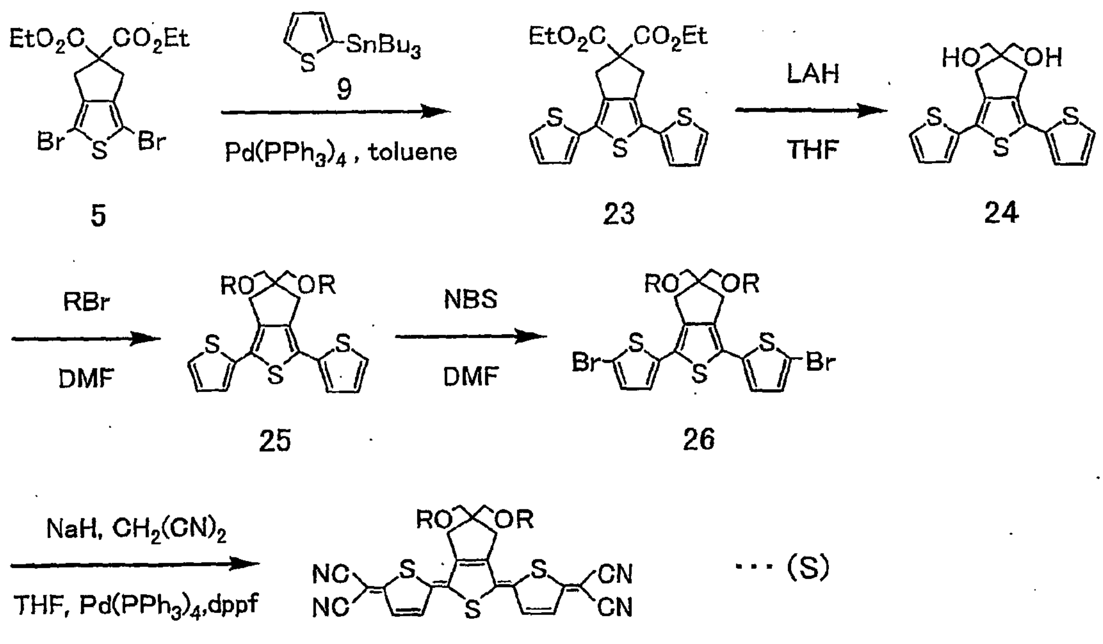


5 23 24

25 26

27 ··· (S)

As shown in reaction formula (S), a compound 24 serves as a common intermediate. This enables synthesis of a compound 27, which is the compound represented by the general formula (1). The compound 27 contains a compound 27a which is an n-hexyl derivative, a compound 27b which is an n-octyl derivative, and a compound 27c which is an n-decyl derivative.

Chem. 27

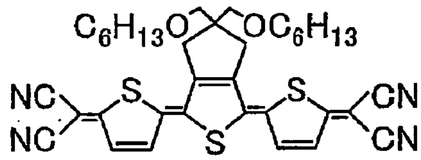

27a

Chem. 28

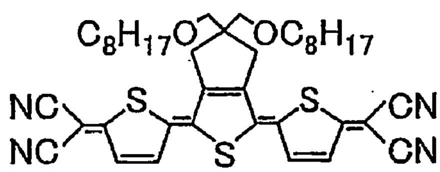

27b

Chem. 29

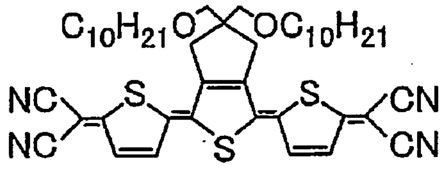

27c

<(V) Synthesis of Compound represented by General Formula (1) (Compound represented by the general formula (1) where $R_1$ and $R_2$ are a hexyloxymethyl group; $R_3$ and $R_4$ are a hydrogen atom; $R_5$ and $R_6$ are a selenium atom; and *a*, *b*, and care 1)>

The compound 27a, which is the n-hexyl derivative, attains an excellent FET feature, as later described. In order to further improve this FET feature, synthesis of a compound in which unsubstituted thiophene rings on both ends was replaced with selenophene rings is carried out. In a same method, synthesis of the n-octyl derivative [(VI) later described] is also carried out.

A compound represented by the general formula (1) where $R_1$ and $R_2$ are a hexyloxymethyl group; $R_3$ and $R_4$ are a hydrogen atom; $R_5$ and $R_6$ are a selenium atom; and *a*, *b*, and *c* are 1 may be synthesized as shown in reaction formula (T) as follows:

Chem. 30

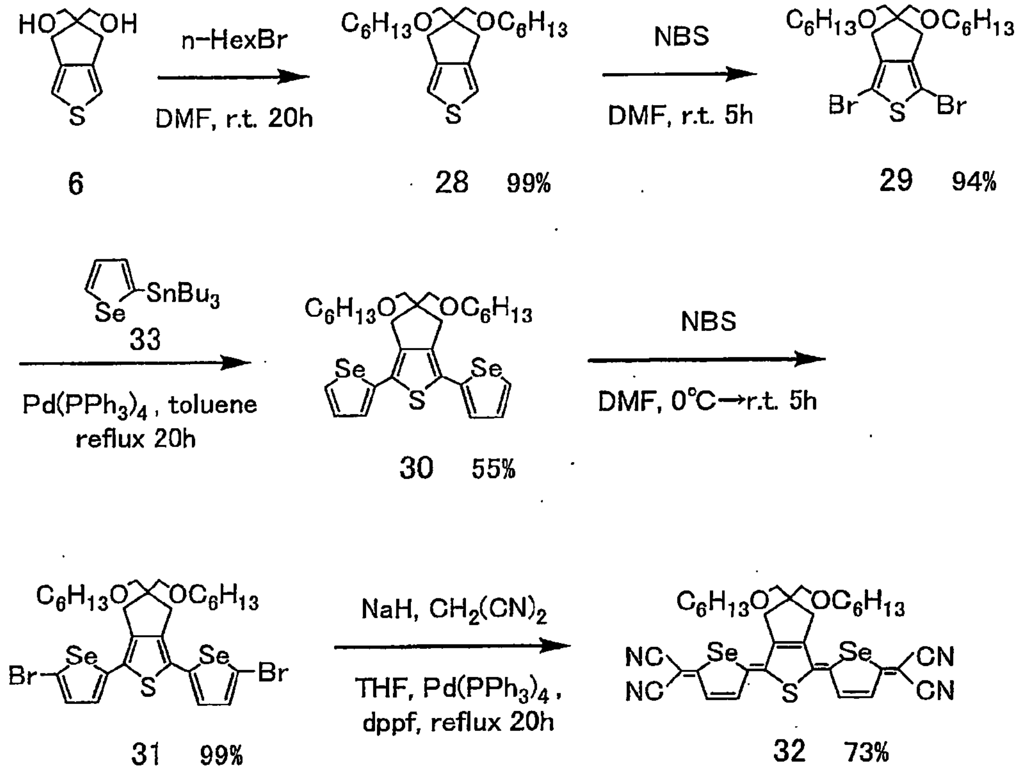


··· (T)

A compound 32 which is the compound represented by the general formula (1) is synthesized as shown in reaction formula (T).

Chem. 31

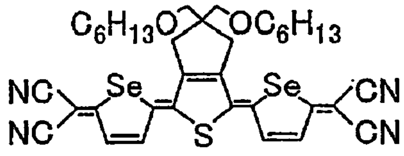

32

<(VI) Synthesis of Compound represented by General Formula (1) (Compound represented by the general formula (1) where $R_1$ and $R_2$ are a octyloxymethyl group; $R_3$ and $R_4$ are a hydrogen atom; $R_5$ and $R_6$ are a selenium atom; and *a*, *b*, and *c* are 1)>

A compound represented by the general formula (1) where $R_1$ and $R_2$ are a octyloxymethyl group; $R_3$ and $R_4$ are a hydrogen atom; $R_5$ and $R_6$ are a selenium atom; and *a*, *b*, and *c* are 1 is represented by reaction formula (U) as follows:

Chem. 32

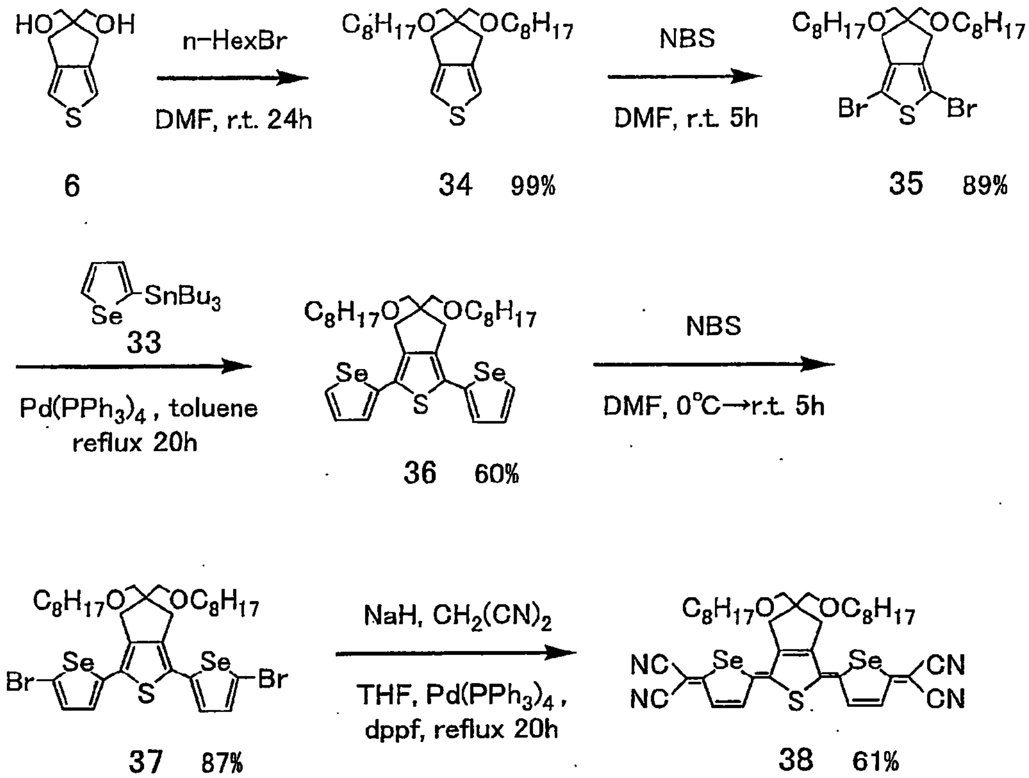


··· (U)

A compound 38 which is the compound represented by the general formula (1) is synthesized as shown in reaction formula (U).

Chem. 33

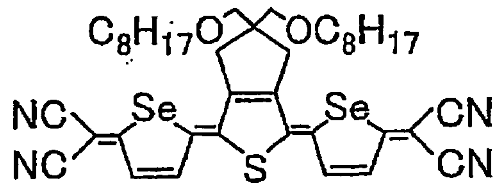

38

<(VII) Synthesis of Organic Semiconductor Material containing Compound represented by General Formula (1)>

The compound 13, 18, 27a, 27b, 27c, 32, or 38 is served as an organic semiconductor material, or an organic semiconductor material is synthesized by containing another material in a degree in which a feature of the compound 13, 18, 27a, 27b, 27c, 32, or 38 is not inhibited. It is not particularly limited in how another material is contained, however one method is, for example, to synthesize the organic semiconductor material by heating and stirring the compound 13, 18, 27a, 27b, 27c, 32, or 38 and another material.

Examples of a conventionally known material encompass dopants such as: bases such as ammonia, organic amine and the like; inorganic salts such as potassium iodide, sodium iodode and the like; and metal atoms such as sodium, potassium and the like.

<Organic Semiconductor Device>

An organic semiconductor device of the present invention contains the organic semiconductor material. It is not particularly limited in how the semiconductor device is manufactured, and various conventionally known manufacturing methods are usable. Among the usable manufacturing methods, a vacuum evaporation method or a welding process may be used as a method for positioning the organic semiconductor material on a substrate. The welding method here denotes a method in which the organic semiconductor material is dissolved in various organic solvents, and a semiconductor device is produced by method of spin coating, inkjet, or the like.

Thus, in order to use the welding method, it is required to dissolve the organic semiconductor material into various organic solvents.

The compound represented by the general formula (1) has a cyclopentane ring-fused thiophene. Therefore, it is possible to prevent a decrease in solubility that occurs along with conjugation expansion of a quinoid structure, which makes it easy to dissolve in the various organic solvents. Further, it is more preferable for the $R_1$ and $R_2$ in the general formula (1) to be easily-dissolvable functional groups, since the cyclopentane ring-fused thiophene becomes even more easily dissolvable in the various organic solvents.

The organic semiconductor, more specifically, includes a semiconductor layer containing the organic semiconductor material positioned on the substrate, and at least two electrodes which are in contact with the semiconductor layer.

Examples of the organic semiconductor device encompass, for example, a transistor, a diode, a condenser, a thin film photoelectric transducer, a dye-sensitized solar cell, a thin film transistor (TFT), and a light emitting device such as an organic carrier transporting layer and/or a light emitting layer. Note that the transistor may be a unipolar transistor (field effect transistor) or a bipolar transistor. The unipolar transistor (field effect transistor) is a transistor having one type of carrier, and the bipolar transistor is a transistor having two types of carriers.

The organic semiconductor material of the present invention introduces a thiophene or a selenophene which are not cyclopentane ring-fused on ends of the cyclopentane ring-fused thiophene. This uniforms a film which is inhomogeneous due to a bulky functional group of the cyclopentane ring-fused thiophene. As a result, it is possible to prevent a decrease in the FET (field effect transistor) feature. Therefore, the organic semiconductor material of the present invention is suitably used by the field effect transistor. The following description describes a mode of the field effect transistor and its manufacturing method.

Provided that the organic semiconductor material of the present invention is used, the arrangement and the manufacturing method of the field effect transistor may be, for example, the ones disclosed in Japanese Patent Application Publication, Tokukai, No. 2006-28055 or the ones disclosed in Japanese Patent Application Publication, Tokukai, No. 2006-114701.

The field effect transistor may be, for example, a field effect transistor including a source electrode, a drain electrode, and a gate electrode. The source electrode and the drain electrode are in contact with a semiconductor layer

provided on a substrate. The gate electrode is provided between the source electrode and the drain electrode, and is on the semiconductor layer via a gate insulating layer.

With this arrangement, by changing a voltage applied to the gate electrode while a predetermined voltage is applied to the drain electrode, it is possible to control current flowing between the source electrode and the drain electrode. The following deals with further detailed description of the field effect transistor having this arrangement.

The source electrode, the drain electrode, and the gate electrode can be made of any material that is electrically conductive. Examples of the material encompass: metals such as platinum, gold, silver, nickel, chrome, copper, iron, tin, aluminum, titanium, and magnesium; alloys at least including any of these metals; an electrically conductive oxide such as a tin oxide, an indium oxide, and tin (ITO); and a semiconductor such as silicon and germanium. Especially, it is preferable that the material is the platinum, the gold, the silver, the copper, the aluminum, or the ITO.

Further, each of the materials of the source electrode, the drain electrode, and the gate electrode may preferably be a publicly-known electrically conductive polymer improved in its electrical conductivity by doping or the like. Examples of the electrically conductive polymer encompass: electrically conductive polyaniline; electrically conductive polypyrrole; electrically conductive polythiophene; and a complex of polyethylenedioxythiophene and polystyrene sulfonate. Of these, it is preferable to use the one which allows the electrodes to have a low electric resistance on their surfaces in contact with the semiconductor layer.

Each of the electrodes may be provided, for example, such that (i) an electrically conductive thin film is made of the above-mentioned material by means of the vacuum vapor deposition, the sputtering, or the like and then (ii) the electrically conductive thin film thus made is subjected to a publicly-known photolithographic method or a lift-off method. Further, instead of this, each of the electrodes may also be provided such that metal foil (e.g., aluminum foil, copper foil) is subjected to etching using a resist prepared by a thermal transfer method, the inkjet method, or the like.

Furthermore, each of the electrodes may be provided such that patterning is carried out directly by means of the inkjet method using (i) a solution or a dispersion liquid of an electrically conductive polymer or (ii) a dispersion liquid of electrically conductive fine particles. Moreover, instead of this, each of the electrodes may be provided such that a film formed by use of application is subjected to lithography, laser ablation, or the like. Furthermore, each of the electrodes may be provided such that patterning is carried out by means of a printing method such as letterpress printing, intaglio printing, offset printing, and screen printing each of which uses (i) ink including the electrically conductive polymer or the electrically conductive particles, (ii) the electrically conductive paste, or the like.

A thickness of each of the electrodes is not particularly limited, but may be selected as needed in accordance with the type or the arrangement of the field effect transistor to be made. For example, in the case of the foregoing field effect transistor, the electrode thereof may preferably be arranged so as to have a thickness of 0.01 $\mu$m or more but 10 $\mu$m or less, and more preferably a thickness of 0.02 $\mu$m or more but 0.1 $\mu$m or less. The thickness of 0.01 $\mu$m or more is necessary to allow the electrode to function as an electrode. Further, the thickness of 0.1 $\mu$m or less is necessary to allow the electrode to maintain its smoothness. Note that the present invention is not limited to this, because a preferable thickness of an electrode varies depending on various factors.

A material of the gate insulating layer may be selected from various kinds of compounds having an insulation property. Examples of the material encompass an inorganic compound, an inorganic oxidized compound, and an organic compound. The inorganic compound may be, for example, silicon nitride, aluminum nitride, or the like. The inorganic oxidized compound may be, for example, a silicon oxide, an aluminum oxide, a tantalum oxide, a titanium oxide, or a tin oxide. The organic compound may be, for example, (i) a polymer such as polyimide, polyamide, polyester, and epoxy resin, (ii) a copolymer produced by a combination of selected ones of these polymers, or (iii) the like.

The gate insulating layer may be provided by means of the vacuum vapor deposition, CVD, or wet processing such as the sputtering, the printing, and the inkjet method. Especially, it is preferable that the wet processing is used in the case where the gate insulating layer is provided with use of the organic compound. The insulating layer may preferably be arranged so as to have a thickness of 50 nm or more but 3 $\mu$m or less, and more preferably a thickness of 100 nm or more but 1 $\mu$m or less.

The organic semiconductor material of the present invention is excellent in mobility. Therefore, as described above, the organic semiconductor material of the present invention may preferably be used as a material of the semiconductor layer included in the field effect transistor. The semiconductor layer including the organic semiconductor material of the present invention may be provided on the substrate by means of the vacuum vapor deposition. Further, instead of this, the semiconductor layer including the organic semiconductor material of the present invention may easily be provided on the substrate by means of the welding process, because the organic semiconductor material includes the cyclopentane ring-fused tiophene.

By using the welding process, it is possible to manufacture a field effect transistor having a light weight, flexibility, and a large area at a low cost and in a simple and easy manner.

The manufacturing method of the field effect transistor including the organic semiconductor material of the present invention is not particularly limited. Provided that the organic semiconductor material of the present invention is used, the method may be a publicly-known manufacturing method of a field effect transistor.

For example, firstly, a gate insulating layer made of the silicon oxide is provided on a substrate made of glass by means of the vacuum vapor deposition. Then, a semiconductor layer including the organic semiconductor material is provided on the gate insulating layer by means of the welding process. Subsequently, a source electrode, a drain electrode, and a gate electrode each of which is made of gold are provided on the substrate by means of the vacuum vapor deposition. This is one example of how to manufacture the field effect transistor.

Provided that the organic semiconductor material of the present invention is used, an arrangement and a manufacturing method of the bipolar transistor may be, for example, the publicly-known ones disclosed in Japanese Patent Application Publication, Tokukaihei, No. 10-214044. Specifically, a pixel of a bipolar transistor according to this publication includes a transparent insulating substrate, an anode, a hole transporting layer, a light-emitting layer, an electron transporting layer, a p-type organic semiconductor layer, an n-type organic semiconductor layer, a base electrode, and a cathode. Further, (a) the electron transporting layer made of an n-type organic semiconductor and (b) the p-type organic semiconductor layer form a pn conjunction, while (b) the p-type organic semiconductor layer and (c) the n-type organic semiconductor layer also form a pn conjunction.

Provided that the organic semiconductor material of the present invention is used, an arrangement and a manufacturing method of the diode may be, for example, the publicly-known ones disclosed in Japanese Patent Application Publication, Tokukai, No. 2002-289878. Specifically, a diode according to this publication includes a hole transporting layer and an electron transporting layer. The hole transporting layer is provided on an anode side, and is made of an organic compound capable of transporting a hole. The electron transporting layer is provided on a cathode side, and is made of an organic compound capable of transporting an electron. Further, the hole transporting layer and the electron transporting layer are stacked. Furthermore, the diode exhibits a non-linear current/voltage characteristic while voltage is applied between the hole transporting layer and the electron transporting layer, the hole transporting layer and the electron transporting layer being in contact with each other.

Provided that the organic semiconductor material of the present invention is used, an arrangement and a manufacturing method of the condenser may be, for example, the publicly-known ones disclosed in Japanese Patent Application Publication, Tokukai, No. 2005-150705. Specifically, a condenser according to this publication includes (i) an electric conductor as one electrode (an anode) and (ii) a semiconductor layer as another electrode (a cathode). The electric conductor has a fine pore whose surface is provided with a dielectric layer. The semiconductor layer is formed on the electric conductor by means of an electricity application method carried out in an electrolyte solution. In the manufacturing method of the condenser, before passing a current, a precursor of a semiconductor layer goes into the fine pore, so that the precursor of the semiconductor layer in the fine pore has a higher concentration than that of the precursor of the semiconductor layer in the electrolyte solution.

Provided that the organic semiconductor material of the present invention is used, an arrangement and a manufacturing method of the thin-film photoelectric converting element may be, for example, the publicly-known ones disclosed in Japanese Patent Application Publication, Tokukai, No. 2006-060104. Specifically, a thin-film photoelectric converting element according to this publication includes: a first base substance; a first electrode; a second electrode; a photoelectric converting layer; and a second substrate substance (a transparent protecting film). The first electrode is formed on the first base substance. The second electrode is provided on a virtual surface substantially parallel to the first base substance, and be adjacent to the first electrode with a gap between the first electrode and the second electrode. The photoelectric converting layer is provided at least between the first electrode and the second electrode. The second substrate substance (the transparent protecting film) is provided so that (i) the second substrate substance faces the first substrate substance and (ii) the first electrode, the second electrode, and the photoelectric converting layer are sandwiched between the first substrate substance and the second substrate substance.

Provided that the organic semiconductor material of the present invention is used, an arrangement and a manufacturing method of the dye-sensitized solar cell may be, for example, the publicly-known ones disclosed in Japanese Patent Application Publication, Tokukai, No. 2004-047752. Specifically, a dye-sensitized solar cell according to this publication includes a light-transmitting member whose sides are shaped in a substantially right-angled triangle. In the light-transmitting member, one of its sides intersecting at a right angle is a side from which a solar beam enters, and the other of the sides is a side to which a solar cell is attached lengthwise. Further, a slanted surface of the light-transmitting member serves as a reflecting surface for reflecting the solar beam entering thereto.

The organic semiconductor device of the present invention may preferably be the thin-film transistor (TFT). The thin-film transistor has a thin thickness. This reduces a size and a weight of the organic semiconductor device. A manufacturing method of the thin-film transistor is not particularly limited. However, it is preferable that the thin-film transistor is made by means of the welding process in which (i) the organic semiconductor material is dissolved in an organic solvent of various kinds, (ii) the organic semiconductor material thus dissolved is applied by a method such as an applying method, a spin-coating method, or an inkjet method, and thus (iii) the thin-film transistor is formed. The welding process allows the substrate to be made of paper, plastic, or the like. This reduces the weight of the organic semiconductor device, and provides the organic semiconductor device with flexibility. The organic semiconductor material of the present invention may easily be provided on the substrate even in the case where the welding process is used.

Therefore, the organic semiconductor material is very effective to give the thin-film transistor a lighter weight, a greater flexibility, and the like.

A thickness of the thin-film transistor may be selected as needed in accordance with the arrangement and the size of the device. For example, the thickness is generally 20 nm or more but 1000 nm or less. However, the present invention is not limited to this. It is preferable that the "thin film" is made so that its thickness is reduced as much as possible. A thin film which is preferably used in an organic semiconductor may have a thickness of 1 nm or more but 1 μm or less, preferably a thickness of 5 nm or more but 500 nm or less, and more preferably a thickness of 10 nm or more but 500 nm or less.

It is preferable that the organic semiconductor device of the present invention is the light-emitting device including the organic carrier transporting layer and/or the light-emitting layer. For example, the organic semiconductor device may be: the light-emitting device including the organic carrier transporting layer alone; the light-emitting device including the light-emitting layer alone; or the light-emitting device including the organic carrier transporting layer and the light-emitting layer.

The organic carrier transporting layer provides a transporting layer, in which a carrier freely moves. This makes it easy to form an electrode and the like in manufacturing of a semiconductor device. The light-emitting layer provides an area where (i) holes injected from the anode side and (ii) electrons injected from the cathode side are recombined when voltage is applied. The organic carrier transporting layer and the light-emitting layer respectively provide a transporting layer in which a carrier freely moves, and a light-emitting layer to which (i) holes injected from the anode side and (ii) electrons injected from the cathode side are transported when voltage is applied. This gives the organic semiconductor device an ability of emitting light.

The organic carrier transporting layer is a layer for transporting a carrier to the light-emitting layer. There are two types of the organic carrier transporting layers. One is a hole transporting layer, and the other is an electron transporting layer. By the hole transporting layer, holes injected from the anode are transported to the light-emitting layer. By the electron transporting layer, electrons injected from the cathode are transported to the light-emitting layer.

The light-emitting layer is a semiconductor layer including a light-emitting material. Further, the light-emitting layer is capable of injecting a hole from the anode side and injecting an electron from the cathode side when voltage is applied. Thereby, the light-emitting layer is capable of providing an area where the hole and the electron are recombined. The light-emitting material may be below-described various kinds of low-molecular-weight light-emitting materials or various kinds of high-molecular-weight light-emitting materials. The light-emitting material may be at least one of them.

The low-molecular-weight light-emitting material may be a publicly-known compound, for example, (i) a benzene compound such as distyrylbenzene (DSB) and diaminodistyrylbenzene (DADSB), a naphthalene compound such as naphthalene and Nile red, or (ii) a phenanthrene compound such as phenanthrene.

The high-molecular-weight light-emitting material may be a publicly-known compound, for example, (i) a polyacetylene compound such as trans-polyacetylene, cis-polyacetylene, poly(di-phenylacetylene) (PDPA), and poly(alkyl phenylacetylene) (PAPA), or (ii) poly(p-phenylene vinylene) (PPV).

A light-emitting device with the organic carrier transporting layer including the light-emitting material can be produced without the light-emitting layer.

That is, in a case where the hole transporting layer includes the light-emitting material, a light-emitting device to be provided is the one including the hole-transporting light-emitting layer and the electron transporting layer. In a case where the electron transporting layer includes the light-emitting material, a light-emitting device to be provided is the one including the electron-transporting light-emitting layer and the hole transporting layer. In these cases, (i) an area in the vicinity of an interface between the hole-transporting light-emitting layer and the electron transporting layer and (ii) an area in the vicinity of an interface between the electron-transporting light-emitting layer and the hole transporting layer serve as the light-emitting layers, respectively.

Specific examples of the light-emitting device including the organic carrier transporting layer and/or the light-emitting layer according to the present invention encompass an organic EL element. Provided that the organic semiconductor material of the present invention is used, an arrangement and a manufacturing method of the organic EL element may be, for example, the publicly-known ones disclosed in Japanese Patent Application Publication, Tokukai, No. 2006-199909. Specifically, the organic EL element may include: a substrate; an anode layer provided on the substrate; a semiconductor layer which includes the organic semiconductor material of the present invention and is provided on the anode; a cathode layer provided on the semiconductor layer; and a protecting layer provided so as to cover each of the layers.

The semiconductor layer includes an organic carrier transporting layer and/or a light-emitting layer. The organic carrier transporting layer includes two layers, that is, a hole transporting layer and an electron transporting layer. In a case where the semiconductor layer includes the organic carrier transporting layer and the light-emitting layer, the semiconductor layer is formed so that the hole transporting layer, the light-emitting layer, and the electron transporting layer are provided on the anode in this order. By the hole transporting layer, the holes injected from the anode are transported to the light-emitting layer. By the electron transporting layer, electrons injected from the cathode are transported to the light-emitting layer.

With this arrangement, when it is started to pass a current (i.e., to apply a voltage) between the anode and the cathode, holes move in the hole transporting layer, and electrons move in the electron transporting layer. Then, the holes and the electrons are recombined in the light-emitting layer. Subsequently, in the light-emitting layer, energy is discharged due to the recombination, and the energy thus discharged generates an exciton. Then, when the exciton returns to its ground state, the exciton discharges energy (fluorescence, phosphorescence), so that light is emitted.

Further, the semiconductor layer may be the one which does not include the light-emitting layer but includes the organic carrier transporting layer. With this arrangement, the organic carrier transporting layer may include the hole-transporting light-emitting layer and the electron-transporting light-emitting layer. In this case, (i) an area in the vicinity of an interface between the hole-transporting light-emitting layer and the electron transporting layer and (ii) an area in the vicinity of an interface between the electron-transporting light-emitting layer and the hole transporting layer function as the light-emitting layers, respectively. In the case of using the hole-transporting light-emitting layer, holes injected from the anode to the hole-transporting light-emitting layer are confined to the electron transporting layer. In the case of using the electron-transporting light-emitting layer, electrons injected from the cathode to the electron-transporting light-emitting layer are confined to the electron-transporting light-emitting layer. These improve recombination efficiency of the holes and electrons.

Furthermore, the semiconductor layer may be the one which does not include the organic carrier transporting layer but includes the light-emitting layer. With this arrangement, it is possible to use the light-emitting layer including the organic semiconductor material of the present invention and the foregoing light-emitting material.

The substrate is a supporting body for supporting the organic EL element. The above-mentioned layers are provided on the substrate. Material of the substrate may be the one which transmits light and exhibits a good optical property.

Examples of the material encompass: various kinds of resin materials such as polyethylene terephthalate, polyethylene naphthalate, polypropylene, cycloolefin polymer, polyamide, polyether sulfone, polymethyl methacrylate, polycarbonate, and polyarylate; and various kinds of glass materials. The material may be at least one of them.

The substrate is not particularly limited in terms of thickness. However, it is preferable that the substrate has a thickness of 0.1 mm or more but 30 mm or less, and more preferably has a thickness of 0.1 mm or more but 10 mm or less.

The anode is an electrode from which holes are injected to the hole transporting layer. The anode is substantially transparent (i.e., clear and colorless, clear and colored, or translucent) so that light emitted from the light-emitting layer is visually recognized. Examples of a material of the anode encompass: an oxide such as an ITO (Indium Tin Oxide), $SnO_2$, $SnO_2$ containing Sb, and ZnO containing Al; Au; Pt; Ag; Cu; and an alloy of these. At least one of these may be used as the material.

A thickness of the anode is not particularly limited. However, it is preferable that the anode has a thickness of 10 nm or more but 200 nm or less, and more preferably has a thickness of 50 nm or more but 150 nm or less. An excessively thin anode would not sufficiently function as an anode. On the other hand, an excessively thick anode would cause a significant decrease in its light transmission to an impractical level, depending on which material the anode is made from.

The cathode is an electrode from which electrons are injected to the electron transporting layer. Examples of a material of the cathode encompass Li, Mg, Ca, Sr, La, Ce, Er, Eu, Sc, Y, Yb, Ag, Cu, Al, Cs, Rb, and an alloy of these. At least one of these may be used as the material.

It is preferable that the cathode has a thickness of 1 nm or more but 1 $\mu$m or less, and more preferably has a thickness of 100 nm or more but 400 nm or less. An excessively thin cathode would not sufficiently function as a cathode. An excessively thick cathode would cause a decrease in light-emitting efficiency of the organic EL element. The organic semiconductor material of the present invention may be used for the hole transporting layer, the light-emitting layer, and the electron transporting layer each of which is included in the semiconductor layer. That is, the light-emitting device including the organic carrier transporting layer and/or the light-emitting layer encompass (i) the one including the organic semiconductor material of the present invention for one of the layers included in the semiconductor layer, (ii) the one including the organic semiconductor material of the present invention for one or more of the layers included in the semiconductor layer, and (iii) the one including the organic semiconductor material of the present invention for all of the layers included in the semiconductor layer.

The organic semiconductor material of the present invention may be provided to any of the layers included in the semiconductor layer by means of the vacuum vapor deposition. However, it is preferable that the organic semiconductor material of the present invention may be provided to any of the layers included in the semiconductor layer by means of the foregoing welding process. By using the welding process, it is possible to manufacture an organic EL element having a light weight, flexibility, and a large area at a low cost and in a simple and quick manner.

Further, a layer for a purpose may be provided between any of the layers included in the organic EL element. For example, a hole injecting layer may be provided between the hole transporting layer and the anode. The hole injecting layer improves injection efficiency of holes injected from the anode. Further, an electron injecting layer may be provided

between the electron transporting layer and the cathode. The electron injecting layer improves injection efficiency of electrons injected from the cathode. In the case where the hole injecting layer and the electron injecting layer are provided to the organic EL element, it is possible to use the organic semiconductor material of the present invention as materials of the hole injecting layer and the electron injecting layer.

It is preferable that each of the layers included in the organic EL element are covered with a protecting layer. The protecting layer seals each of the layers included in the organic EL element so that the layers airtightly. This allows the layers to be blocked from oxygen and water. Providing the protecting layer gives an effect such as: an improvement in reliability of the organic EL element; prevention of a change in properties of the organic EL element; and prevention of deterioration of the organic EL element.

Examples of a material of the protecting layer encompass Al, Au, Cr, Nb, Ta, Ti, an alloy of them, a silicon oxide, and various kinds of resin materials. In a case where the protecting layer is made of an electrically conductive material, it is preferable that insulating layers are provided as needed between the protecting layer and the layers included in the organic EL element, respectively. This is for avoiding a short circuit between the protecting layer and the layers.

The manufacturing method of the semiconductor device of the present invention is carried out such that the organic semiconductor material is annealed. This improves mobility of an electron in an n-type material to which the welding process is applicable.

The annealing is a process in which (i) a temperature of a material is increased to a melting point (or a decomposition point) or less and then (ii) the material is gradually and slowly cooled down. The annealing may be carried out by means of a conventionally and publicly known method. For example, the annealing may preferably be carried out as follows: The organic semiconductor material is processed at 80°C or more but 200°C or less for one hour with use of a batch type anneal oven which uses gas, petroleum, or electricity as a heat source.

The present invention is not limited to the description of the embodiments above, but may be altered by a skilled person within the scope of the claims. An embodiment based on a proper combination of technical means disclosed in different embodiments is encompassed in the technical scope of the present invention.

Examples

Described below are examples of synthesis of a compound represented by the general formula (1).

(I) Synthesis of monomer unit (cyclopentane ring-fused thiophene)

Methyl chloroacetate was reacted with sodium sulfide nonahydrate as shown in a reaction formula (D), so that a compound 1 was synthesized with a yield of 47%. Then, the compound 1 was subjected to Hinsberg dehydrative cyclization using 2,3-butanedione, so that a compound 2 was synthesized with a yield of 50%.

Chem. 34

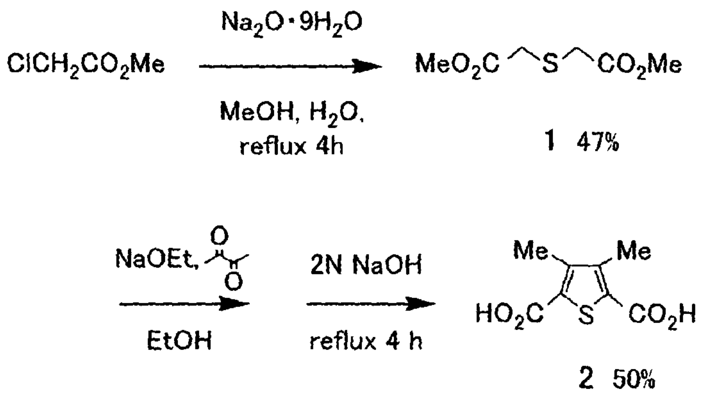


··· (D)

The compound 2 was then subjected to decarbonization and bromination as shown in a reaction formula (E), so that a compound 3 was synthesized with a yield of 84%. Then, the compound 3 was subjected to radical bromination using benzoyl peroxide as an initiator, so that a compound 4 was synthesized with a yield of 80%. Each reaction described above quantitatively proceeded.

Chem. 35

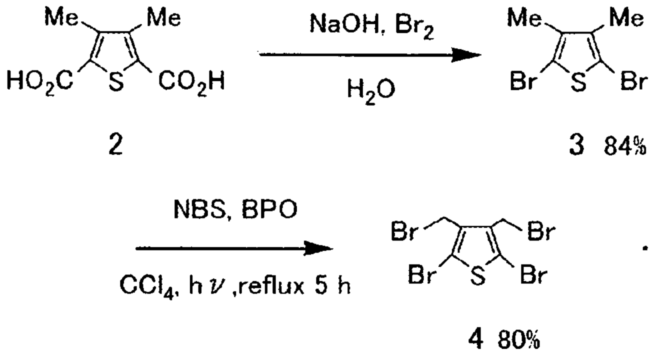


··· (E)

Then, the compound 4 causes an intramolecular cyclization therein with malonic diester as shown in a reaction formula (F), so that a compound 5 was synthesized.

Chem. 36

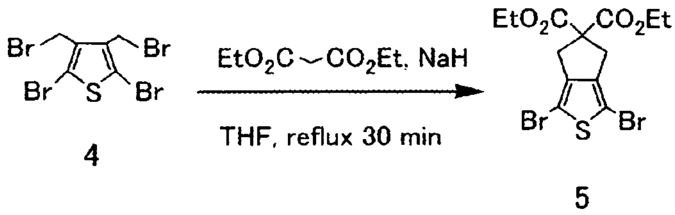


··· (F)

A reaction shown in the reaction formula (F) was carried out under conditions shown in Table 1.

Table 1

| Condition | Amount of compound 4/ Solvent and amount thereof/ Concentration of compound 4 | Temp. | Amount of diethyl malonate / Solvent and amount thereof/ Concentration of diethyl malonate | Yield (%) |
|---|---|---|---|---|
| 1 | 10g/ toluene-DMF 96ml/ 0.24M | 60°C | 3.66ml/ toluene 75ml/ 0.32M | 22 |
| 2 | 20g/ toluene-DMF 128ml/ 0.37M | 70°C | 7.30ml/ toluene 100ml/ 0.48M | 32 |
| 3 | 10g/ THF 60ml/ 0.38M | 60°C | 3.66ml/ THF 60ml/ 0.40M | 63 |
| 4 | 5g/ THF 200ml/ 0.06M | 65°C (under reflux) | 1.83ml/ THF 20ml/ 0.60M | 73 |
| 5 | 10g/ THF 200ml/ 0.13M | 65°C (under reflux) | 3.66ml/ THF 20ml/ 1.20M | 67 |
| 6 | 20g/ THF 200ml/ 0.23M | 65°C (under reflux) | 7.30ml/ THF 20ml/ 2.40M | 64 |
| * Dropping of diethyl malonate was carried out for 3 hours (constant). | | | | |

In a condition 1 shown in Table 1, 96ml of toluene-DMF was added to 10g of the compound 4 so that a solution containing 0.24mol/l (hereinafter referred to as "M") of the compound 4 was obtained; and 75ml of toluene was added to 3.66ml of diethyl malonate so that a solution containing 0.32M of diethyl malonate was obtained. Then, these solutions were reacted at 60°C, so that the compound 5 was synthesized with a yield of 22% by the intramolecular cyclization. The condition 1 resulted in a low yield of the compound 5 due to Claisen condensation between diethyl malonates.

In a condition 2 shown in Table 1, 128ml of toluene-DMF was added to 20g of the compound 4 so that a solution containing 0.37M of the compound 4 was obtained; and 100ml of toluene was added to 7.30ml of diethyl malonate so that a solution containing 0.48M of diethyl malonate was obtained. Then, these solutions were reacted at 70°C, so that the compound 5 was synthesized with a yield of 32% by the intramolecular cyclization.

In a condition 3 shown in Table 1, 60ml of THF was added to 10g of the compound 4 so that a solution containing

0.38M of the compound 4 was obtained; and 60ml of THF was added to 3.66ml of diethyl malonate so that a solution containing 0.4M of diethyl malonate was obtained. Then, these solutions were reacted, so that the compound 5 was synthesized with a yield of 63% by the intramolecular cyclization. By comparing the condition 1 with the condition 3, it was found that the yield of the compound 5 increased by approximately 40% by changing the solvent from toluene-DMF to THF.

In a condition 4 shown in Table 1, 200ml of THF was added to 5g of the compound 4 so that a solution containing 0.06M of the compound 4 was obtained; and 20ml of THF was added to 1.83ml of diethyl malonate so that a solution containing 0.6M of diethyl malonate was obtained. Then, these solutions were reacted at 65°C (under reflux), so that the compound 5 was synthesized with a yield of 73% by the intramolecular cyclization. By comparing the condition 3 with the condition 4, it was found that the yield of the compound 5 slightly increased by changing the reaction temperature from 60°C to 65°C (under reflux).

In a condition 5 shown in Table 1, 200ml of THF was added to 10g of the compound 4 so that a solution containing 0.13M of the compound 4 was obtained; and 20ml of THF was added to 3.66ml of diethyl malonate so that a solution containing 1.2M of diethyl malonate was obtained. Then, these solutions were reacted at 65°C (under reflux), so that the compound 5 was synthesized with a yield of 67% by the intramolecular cyclization.

In a condition 6 shown in Table 1, 200ml of THF was added to 20g of the compound 4 so that a solution containing 0.23M of the compound 4 was obtained; and 20ml of THF was added to 7.30ml of diethyl malonate so that a solution containing 2.4M of diethyl malonate was obtained. Then, these solutions were reacted at 65°C (under reflux), so that the compound 5 was synthesized with a yield of 64% by the intramolecular cyclization. By comparing the condition 4 with the conditions 5 and 6, it was found that the yield of the compound 5 slightly decreased by increasing a concentration of diethyl malonate. An increase in the concentration of diethyl malonate causes a large amount of diethyl malonate to exist in a reaction system. As a result, the Claisen condensation between diethyl malonates occurs. This causes a decrease of the yield of the compound 5.

In the reaction shown in the reaction formula (F), it is possible to selectively produce the compound 5 by using a dilute solution of diethyl malonate and by dropping the solution over a long drop time. In view of this, a temperature, a solvent, and a concentration of the solution were changed, respectively. As a result, it was found that the use of different solvents most affected the yield of the compound 5 whereas changes in the temperature and in the concentration of the solution had a small effect on the yield. It is considered that, since the reaction is a polar reaction, an anion generated when sodium hydride takes $\alpha$-hydrogen out of diethyl malonate is stabilized in case where THF is used as the solvent.

Next, ester groups of the compound 5 were reduced by lithium aluminum hydride as shown in a reaction formula (G), so that a compound 6 was synthesized with a yield of 74%. Further, the compound 6 was subjected to Williamson etherification using n-butylbromide, so that a compound 7 was quantitatively synthesized with a yield of 97%.

Chem. 37

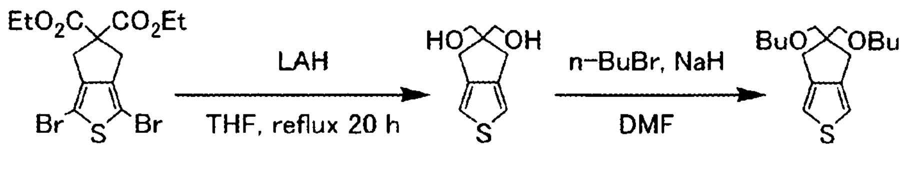


··· (G)

5 6 74% 7 97%

(II) Synthesis of compound represented by the general formula (1), where each of $R_1$ and $R_2$ is a butoxymethyl group; each of $R_3$ and $R_4$ is a hydrogen atom; each of $R_5$ and $R_6$ is a sulfur atom; and each of a, b, and c is a number of 1.

The compound 7 was brominated with N-bromosuccinimide as shown in a reaction formula (H), so that a compound 8 was synthesized with a yield of 92%.

Chem. 38

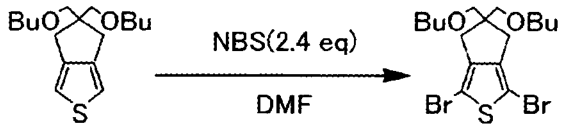

··· (H)

7 8 92%

Then, the compound 8 was reacted with n-butyllithium and modified with tin with the use of tributyltin chloride as shown in a reaction formula (I), so that a compound 9 was synthesized with a yield of 87%.

Chem. 39

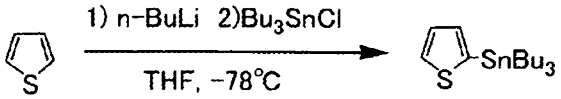

··· (I)

9 87%

Then, the compound 8 and the compound 9 was coupled by Stille coupling as shown in a reaction formula (J), so that a compound 10 was synthesized with a yield of 88%. At this point, since tin cannot be easily separated from the compound 10, the compound 10 was purified by recrystallization with the use of ethanol.

Chem. 40

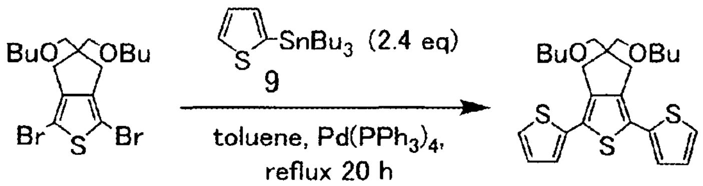

··· (J)

8 10 88%

The compound 10 was then brominated with N-bromosuccinimide as shown in a reaction formula (K), so that a compound 11 was synthesized with a yield of 86%. When this reaction was carried out at a room temperature, a tribromo compound and a tetrabromo compound, each of which has a terminal thiophene having β-position substituted by Br, were produced. The compound 11 was obtained with a high yield when the reaction was carried out in an ice bath.

Chem. 41

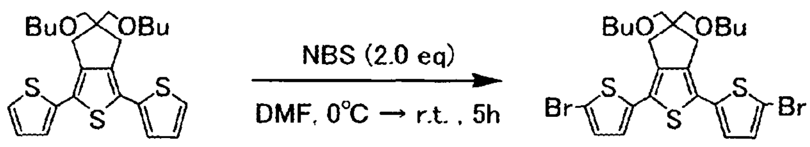

··· (K)

10 11 86%

Then, a dicyanomethyl group was introduced into the compound 11 as shown in a reaction formula (L), so that a compound 13 was synthesized with a yield of 72%.

Chem. 42

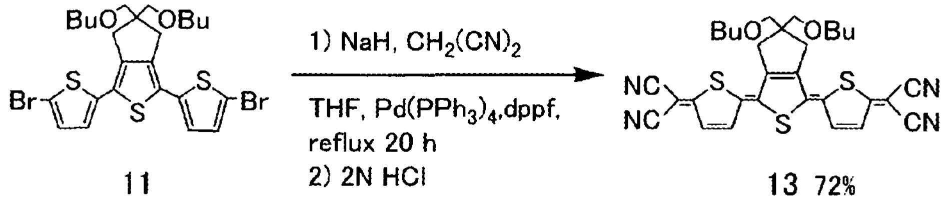


11 13 72% ··· (L)

The compound 10 was iodized with iodosuccinimide as shown in a reaction formula (M), so that a compound 12 was synthesized with a yield of 88%.

Chem. 43

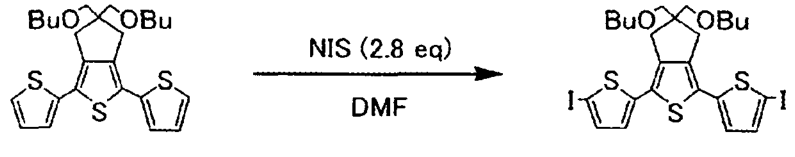


10 12 88% ··· (M)

Then, a dicyanomethyl group was introduced into the compound 12 as shown in a reaction formula (N), so that the compound 13 was synthesized with a yield of 38%.

Chem. 44

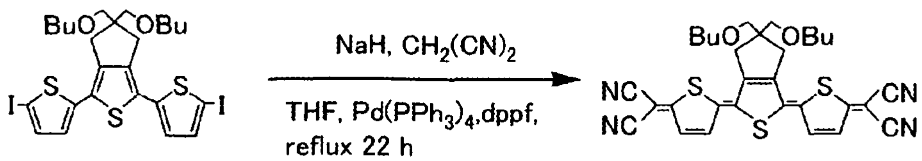


12 13 38% ··· (N)

Producing the compound 13 from a bromo compound as shown in the reaction formula (L) resulted in a moderately high yield. In contrast, producing the compound 13 from an iodo compound as shown in the reaction formula (N) resulted in a low yield. It is considered that this is because a free iodine hinders proceeding of oxidation when the oxidation is carried out by using 2N-HCl. In view of this, impurities were removed by column chromatography, and then 2N-HCl was added again. The solution thus obtained was stirred over night, while monitoring the reaction by a TLC plate. By this, the compound 13 was purified.

Although it is possible to produce the compound 13 from both of the bromo compound and the iodo compound, use of the iodo compound causes a low yield and requires a large amount of time for reflux and stirring. Therefore, it was found that use of the bromo compound is preferable for production of the compound 13.

The compound 13 was purified by column chromatography and recrystallized with an acetone-acetonitrile mixed solvent. A low-active silica gel was used in the column chromatography.

Fig. 1 shows an MS chart and IR chart of the compound 13 after recrystallization. The MS chart shows molecular ion peaks of the compound 13. The IR chart indicates that the compound 13 contains a nitrile group.

Fig. 2 shows a $^1$H-NMR chart of the compound 13 after recrystallization. As shown in the $^1$H-NMR chart, respective peaks of hydrogen of a cyclopentane ring and a thiophene ring became complicated. Since a by-product or the like was not detected in the MS chart and the IR chart, it was found that geometric isomers of the compound 13 exist.

Fig. 3 illustrates each structure of the geometric isomers. As shown in Fig. 3, a geometric isomer in which each

thiophene at both ends has an orientation opposite to a thiophene in the center is referred to as "trans-trans isomer"; a geometric isomer in which the thiophenes at both ends have orientations same as and opposite to the thiophene in the center, respectively, is referred to as "trans-cis isomer"; and a geometric isomer in which each of the thiophenes at both ends has the same orientation as the thiophene in the center is referred to as "cis-cis isomer". By homonuclear 2D-NOE-NMR (NOESY), it was found that the trans-trans isomer, the trans-cis isomer, and the cis-cis isomer exist at a ratio of 2:3:1.

Since all types of the geometric isomers have a degree of crystallization similar to each others, it is difficult to separate the geometric isomers by recrystallization. However, a compound 18 (shown below) produced by introducing a methyl group to a β-position of the compound 13 can be separately obtained as a single component because rotation of thiophenes at its ends can be controlled due to an interaction between the methyl group and methylene hydrogen of a cyclopentane ring.

(III) Synthesis of compound represented by the general formula (1), where each of $R_1$ and $R_2$ is a butoxymethyl group; each of $R_3$ and $R_4$ is a methyl group; each of $R_5$ and $R_6$ is a sulfur atom; and each of a, b, and c is a number of 1.

As shown in a reaction formula (O), in a route 1, 3-methylthiophene was reacted with n-butyllithium and then modified with tin with the use of tributyltin chloride, so that a compound 15 was synthesized with a yield of 53%. In the route 1, the compound 15 having a substituent at a 5-position was preferentially obtained.

In a route 2, 3-methylthiophene was brominated with N-bromosuccinimide, so that a compound 14 was synthesized with a yield of 61%. The compound 14 was reacted with n-butyllithium and then tributyltin chloride was slowly added thereto in drops so that the compound 14 was modified with tin. As a result, the compound 15 was synthesized with a yield of 74%. In the route 2, the compound 15 having the substituent at a 2-position was preferentially obtained. A small amount of the compound 15 having the substituent at the 5-potision was also obtained.

It is considered that occurrence of the compound 15 having the substituent at the 5-position is attributed to that a reaction solution of the compound 15 was increased in temperature to a room temperature after n-butyllithium was added at -78°C and then tributyltin chloride was added at -78°C. Therefore, by keeping the temperature of the reaction solution at -78°C without increasing to a room temperature, it is considered possible to control the occurrence of the compound 15 having the substituent at the 5-position. It is difficult to separately purify the compound 15 having the substituent at the 2-position and the compound 15 having the substituent at the 5-position. Therefore, a route 3 shown in a reaction formula (P) is preferable because less by-product is produced in the route 3.

Chem. 45

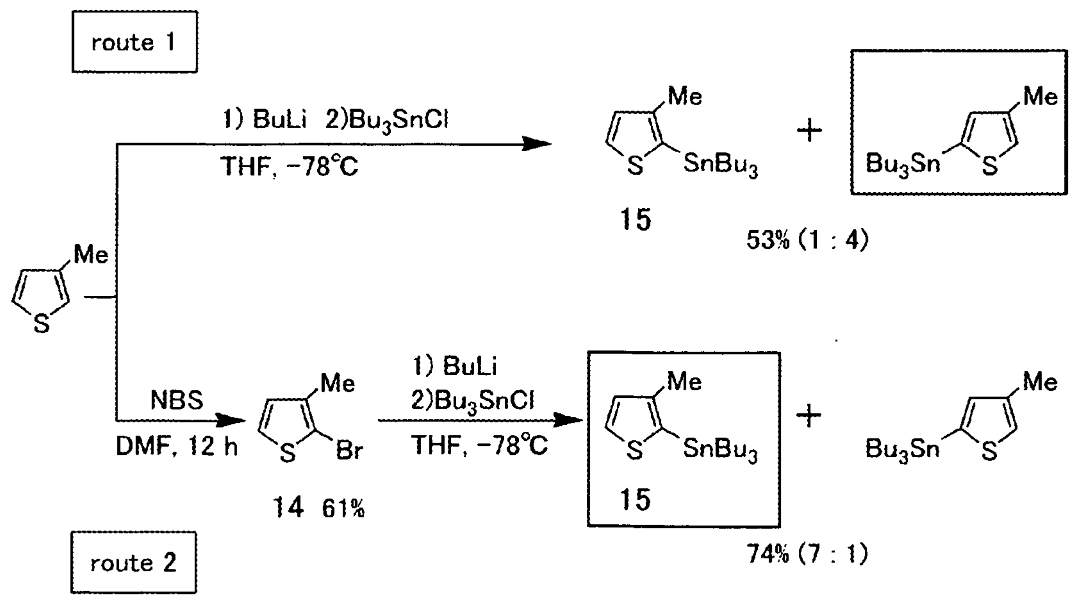


··· (O)

In the route 3 shown in the reaction formula (P), a compound 16 was synthesized under the following two conditions: (i) Grignard reagent containing excess magnesium was added in drops to the compound 8 dissolved in ether and reacted with the compound 8 in the presence of $Ni(dppp)Cl_2$ serving as a catalyst; and (ii) Grignard reagent containing excess magnesium was added in drops to the compound 8 dissolved in THF and reacted with the compound 8 in the presence of $Pd(dppp)Cl_2$ serving as a catalyst. As a result of reactions in the conditions (i) and (ii), the compound 16 was synthesized with a yield of 12% and 57%, respectively.

Chem. 46

route 3

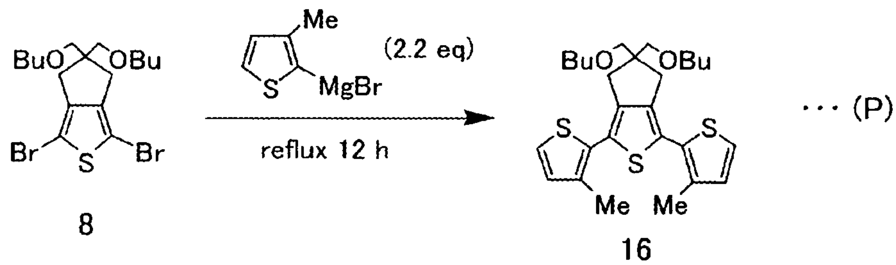


8

16

The condition (i) resulted in a low yield of the compound 16 because ether used as a reaction solvent was evaporated and decreased to less than half amount during a reflux operation. In contrast, the condition (ii) resulted in a moderately high yield of the compound 16 because THF was used as the reaction solvent.

As shown in a reaction formula (Q), the compound 16 was then brominated with N-bromosuccinimide, so that a compound 17 was synthesized with a yield of 85%.

Chem. 47

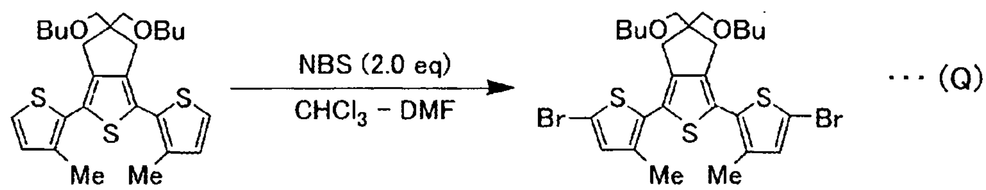


16

17 85%

As shown in a reaction formula (R), a dicyanomethyl group was then introduced into the compound 17, so that a compound 18 was synthesized with a yield of 63%.

Chem. 48

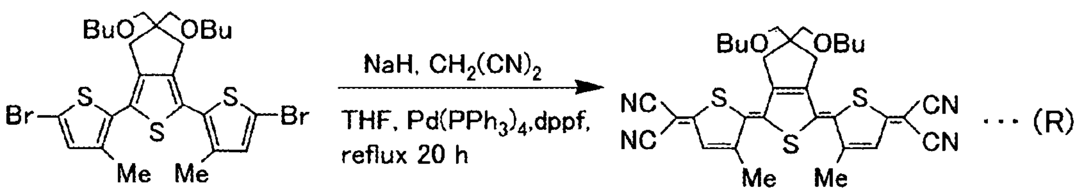


17

18 63%

The compound 18 was purified by column chromatography and recrystallized with an acetone-acetonitrile mixed solvent. A low-active silica gel was used in the column chromatography.

Fig. 4 shows an MS chart and IR chart of the compound 18 after recrystallization. The MS chart shows molecular ion peaks of the compound 18. The IR chart indicates that the compound 18 contains a nitrile group.

Fig. 5 shows a $^1$H-NMR chart of the compound 18 after recrystallization. As shown in the $^1$H-NMR chart, respective peaks of hydrogen of an aromatic part and a cyclopentane ring did not become complicated. Since a by-product or the like, or a geometric isomer of the compound 18 was not detected in the MS chart and the IR chart, it was found that the compound 18 was singularly obtained.

(IV) Physical property of compounds 13 and 18 according to the present invention and of a compound (hereinafter referred to as "compound 30") represented by the general formula (13) according to Non-Patent Literature 3

Chem. 49

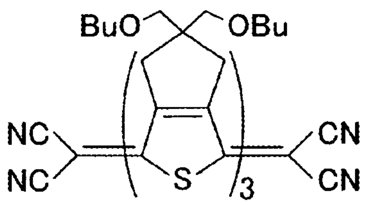

··· (13)

30

(Analysis of electronic absorption spectrum)

Fig. 6 is a view showing a relation between electronic absorption spectrum and absorption wavelength in regard to the compounds 13, 18, and 30. It was found that the compounds 13, 18, and 30 have a similar maximum absorption wavelength $\lambda$max in each of the electronic absorption spectrum. Further, the comparison of the compound 13 with the compound 18 showed that the maximum absorption wavelength was not largely affected by an introduction of a methyl group. However, in the electronic absorption spectrum of the compound 13, there is a peak of absorption at approximately 426nm wavelength. It is considered this is because the compound 13 is a mixed compound of geometric isomers and therefore have the peak since a trans-cis isomer and a cis-cis isomer are produced by forbidden transition. On the other hand, it is considered that each of the electronic absorption spectrums of the compounds 18 and 30 does not have the peak of absorption at approximately 426nm because each of the compounds 18 and 30 includes only a trans-trans isomer.

(Analysis of CV)

Fig. 7 is a view showing each HOMO-LUMO energy of the compounds 13, 18, and 30. The compounds 13, 18, and 30 were subjected to a CV (cyclic voltammetry) analysis using a benzonitrile solvent. Each HOMO-LUMO energy gap of the compounds 13, 18, and 30 was found by a half-wave potential of its oxidation-reduction potential. As a result, the HOMO-LUMO energy gaps of the compound 13, 18, and 30 were 1.35eV, 1.45eV, and 1.46eV, respectively, so that it was found that the compounds 13, 18, and 30 did not differ in HOMO-LUMO energy gap. Further, it was found that the HOMO-LUMO energy gap did not affected by the introduction of a methyl group. It is considered that the compounds 13 and 18, each of which had a lower LUMO level than the compound 30, are more likely to be exposed to electronic injection. Further, it is considered from reduction potentials of the compounds 13, 18, and 30 that the compounds 13, 18, and 30 become more highly applicable to an n-type FET in this order, that is, in the order corresponding to easiness of reduction.

It should be noted that, in a neutral molecule, HOMO means a highest-energy molecular orbital occupied with electrons, and LUMO means a lowest-energy molecular orbital not containing electrons.

(Evaluation of FET characteristic)

For evaluation of FET characteristics of the compounds 13, 18, and 30, an FET element was produced by the following method. First, a $SiO_2$ substrate was cut so as to have a size of approximately 1 × 1cm, and then a back surface (underside surface) of the $SiO_2$ substrate was treated with hydrofluoric acid so as to eliminate silica oxidized thereon by exposure to air. Thereafter, the $SiO_2$ substrate was coated with Au by vaccum vapor deposition, so that a gate electrode was produced. Next, an organic thin film was formed on a surface of the $SiO_2$ substrate by a spin-coating method (method in which the compounds are dropped on a spinning $SiO_2$ substrate via a Pasteur pipette) using a 0.4wt% chloroform solution of each of the compounds (forming conditions of the organic thin film: 2000rpm, 30sec). The $SiO_2$ substrate was a $SiO_2$ substrate that was not subjected to a surface treatment (hereinafter referred to as "untreated $SiO_2$ substrate"), or a $SiO_2$ substrate whose surface was treated with octyl trichlorosilane (hereinafter referred to as "OTS-treated $SiO_2$ substrate"). Then, the organic thin film was coated with Au by vacuum vapor deposition using a shadow mask, so that a source-drain electrode was produced. The FET element in the present example was produced so as to have a channel length of 50$\mu$m and a channel width of 1.5mm. The FET element thus produced has a top-contact structure, and Fig. 8 illustrates an arrangement of the FET element.

Performance of an FET element depends on an amount of electric current passing therethrough when an electric potential is applied between a source and a drain while an electric current is applied to a gate. Measuring the electric current makes it possible to determine a mobility of the FET, which is one of FET characteristics. The mobility can be calculated with an equation (a) representing an electrical characteristic of a carrier that is occurred in an organic semiconductor layer when a gate electric field is applied to $SiO_2$ serving as an insulator.

$$Id = Z\mu Ci\ (Vg - Vt)^2 / 2L \cdots (a)$$

In this equation, Id is a saturated source-drain electric current, Z is a channel width, Ci is a capacitance of an insulator, Vg is a gate electric potential, Vt is a threshold electric potential, L is a channel length, and $\mu$ is a mobility ($cm^2/Vs$) to be determined. Ci is determined according to a dielectric constant of a $SiO_2$ insulating film to be used. Z and L are determined according to an element structure of the FET element. Id and Vg are determined when an electric current in the FET element is measured. Vt can be calculated from Id and Vg. It is possible to calculate the mobility determined in response to a gate electric potential by substituting each value in the equation (a).

FET characteristics of the compounds 13, 18, and 30 were evaluated by using an analysis described above. First, the compound 13 was analyzed. Fig. 9 shows conditions of the spin-coating method and results of the analysis of the FET characteristic. In the analysis, an FET element was produced with an untreated $SiO_2$ substrate and an OTS-treated $SiO_2$ substrate. However, results of the analysis did not largely differ between these substrates. Evaluations of both p-type FET characteristic and n-type FET characteristic were carried out.

In Fig. 9, graphs in an upper column show a p-type FET characteristic measured by applying a negative gate voltage, and graphs in a lower column show an n-type FET characteristic measured by applying a positive gate voltage.

The compound 13 did not exhibit an FET behavior in the analysis of the p-type FET characteristic (Vd-Id, Vg-Id). In contrast, the compound 13 exhibited the FET behavior in the analysis of the n-type FET characteristic (Vd-Id, Vg-Id) although an amount of electric current was very small. Calculation results according to the equation (a) was $\mu$=5.3$\times 10^{-6}$ ($cm^2/Vs$), on/off ratio=9, Vth=7V.

Next, the compound 18 was analyzed in the same way as the compound 13. Fig. 10 shows conditions of the spin-coating method and results of the analysis of the FET characteristic.

As shown in Fig. 10, the compound 18 did not exhibit the FET behavior in the analysis of the p-type or n-type FET characteristic (Vd-Id, Vg-Id). The compound 18 has a higher degree of crystallization than the compound 13 due to introduction of a methyl group and therefore has a lower film-forming ability. This inhibits formation of a continuous semiconductor channel. As a result, it becomes difficult for electric current to pass through. This is why the compound 18 did not exhibit the FET behavior. In the analysis, an FET element was produced with an untreated $SiO_2$ substrate and an OTS-treated $SiO_2$ substrate. The FET behavior was not shown with both of these substrates.

It is considered that, although the compound 18 had a LUMO energy high enough to exhibit the n-type FET behavior for an easy charge injection, the compound 18 did not exhibit the FET behavior because a film cannot be uniformly formed on the substrate.

Next, the compound 30 was analyzed in the same way as the compounds 13 and 18. Fig. 11 shows conditions of the spin-coating method and results of the analysis of the FET characteristic.

As shown in Fig. 11, the compound 30 slightly exhibited the n-type FET behavior. The compound 30 had a higher film-forming ability than the compound 18 and formed a uniform film. In the analysis, an FET element was produced with an untreated $SiO_2$ substrate and an OTS-treated $SiO_2$ substrate. However, the FET behavior exhibited by the compound 30 did not differ between these substrates. Calculation results according to the equation (a) was $\mu$=2.87$\times 10^{-7}$ ($cm^2/Vs$), on/off ratio=111, Vth=-166V.

For evaluation of FET characteristics of the compounds 13, 18, and 30 after annealing, an OTFT (organic thin film transistor) element was produced by the following method. First, an interleave electrode having a channel length of 10$\mu$m and a channel width of 20mm was formed by electron lithography on an n-doped Si/$SiO_2$ substrate. The interleave electrode was coated with 2nm of Cr and 50nm of Au by vapor deposition, so that a gold electrode was produced. Next, an organic thin film was formed on the gold electrode by the spin-coating method using a 0.4wt% chloroform solution of each of the compounds. Then, the gold electrode having a channel length of 10$\mu$m and a channel width of 2cm was analyzed in the FET characteristics.

Fig. 12 illustrates a bottom-contact type OTFT element described above. Fig. 13 shows results of the analysis of n-type FET characteristic (Vd-Id, Vg-Id) in regard to the compound 13 after annealing for 1 hour at 80°C. As shown in Fig. 13, the compound 13 exhibited the FET characteristic. The calculation according to the equation (a) showed that $\mu$=1.28$\times 10^{-3}$ ($cm^2/Vs$). The compound 30 was analyzed in the same way as the compound 13. In this case, the calculation according to the equation (a) showed that $\mu$=3.2$\times 10^{-4}$ ($cm^2/Vs$).

Table 2 shows FET characteristics (mobility, on/off ratio, and threshold voltage) under different conditions of

annealing.

Table 2

| Annealing temperature [°C] | Mobility [$cm^2/Vs$] | On/off ratio | Threshold voltage [V] |
|---|---|---|---|
| Without annealing | $2.9\times10^{-5}$ | 330 | -7.6 |
| 60 | $2.0\times10^{-4}$ | 8500 | 9.1 |
| 80 | $1.3\times10^{-3}$ | 3800 | 13.8 |
| 100 | $1.6\times10^{-3}$ | 1400 | 16.6 |
| 120 | $2.2\times10^{-3}$ | 1100 | 13.1 |
| 140 | $2.5\times10^{-3}$ | 1900 | 11.6 |
| 160 | $2.5\times10^{-3}$ | 4100 | 12.3 |
| 180 | $1.3\times10^{-3}$ | 6800 | 3.34 |
| 200 | $2.8\times10^{-3}$ | $1.3\times10^{-4}$ | -10.57 |

As shown in Table 2, it is considered that annealing improves mobility.

For the purpose of evaluating the FET characteristics (mobility and on-off ratio) generated by annealing (heat treatment), FET characteristics without annealing and FET characteristics after annealing at 50°C, 100°C, 150°C, and 200°C were analyzed, respectively. Results of the analysis were described below.

Table 3 shows results of the analysis of the FET characteristics in regard to the compound 13 (butyl derivative) represented by the general formula (1), synthesized as shown in a reaction formula (B).

Table 3

| Annealing temperature [°C] | Untreated | | OTS-treated | |
|---|---|---|---|---|
| | Mobility [$cm^2/Vs$] (Average) | On/off ratio (Maximum) | Mobility [$cm^2/Vs$] (Average) | On/off ratio (Maximum) |
| Without annealing | No movement | - | $6.19\times10^{-5}$ | $10^3$ |
| 50 | No movement | - | $7.57\times10^{-5}$ | $10^3$ |
| 100 | $2.11\times10^{-5}$ | $10^2$ | $4.62\times10^{-3}$ | $10^3$ |
| 150 | $6.22\times10^{-5}$ | $10^4$ | $1.37\times10^{-2}$ | $10^4$ |

Table 4 shows results of the analysis of the FET characteristics in regard to a compound 27a (hexyl derivative) represented by the general formula (1), synthesized as shown in a reaction formula (S).

Table 4

| Annealing temperature [°C] | Untreated | | OTS-treated | |
|---|---|---|---|---|
| | Mobility [$cm^2/Vs$] (Average) | On/off ratio (Maximum) | Mobility [$cm^2/Vs$] (Average) | On/off ratio (Maximum) |
| Without annealing | $1.80\times10^{-4}$ | $10^2$ | $1.40\times10^{-4}$ | $10^3$ |
| 50 | $2.04\times10^{-4}$ | $10^2$ | $1.70\times10^{-4}$ | $10^3$ |
| 100 | $3.33\times10^{-2}$ | $10^2$ | $2.50\times10^{-2}$ | $10^4$ |
| 150 | $5.55\times10^{-2}$ | $10^3$ | $1.60\times10^{-1}$ | $10^5$ |

In view of the results shown in Tables 3 and 4, in which the untreated substrate was inferior in reproducibility and performance, only an OTS-treated substrate was used in analysis shown in Table 5 and its subsequent tables.

Table 5 shows results of the analysis of the FET characteristics in regard to a compound 27b (octyl derivative) represented by the general formula (1), synthesized as shown in the reaction formula (S).

Table 5

| Annealing temperature [°C] | OTS-treated | |
|---|---|---|
| | Mobility [$cm^2$/Vs] (Average) | On/off ratio (Maximum) |
| Without annealing | No movement | - |
| 50 | $6.17 \times 10^{-4}$ | $10^2$ |
| 100 | $7.25 \times 10^{-2}$ | $10^4$ |
| 150 | $9.68 \times 10^{-2}$ | $10^4$ |
| 200 | $6.19 \times 10^{-2}$ | $10^3$ |
| 250 | No movement | - |

Table 6 shows results of the analysis of the FET characteristics in regard to a compound 27c (decyl derivative) represented by the general formula (1), synthesized as shown in the reaction formula (S).

Table 6

| Annealing temperature [°C] | OTS-treated | |
|---|---|---|
| | Mobility [$cm^2$/Vs] (Average) | On/off ratio (Maximum) |
| Without annealing | No movement | - |
| 50 | No movement | - |
| 100 | $1.30 \times 10^{-2}$ | $10^4$ |
| 150 | $6.91 \times 10^{-3}$ | $10^4$ |
| 200 | $5.13 \times 10^{-3}$ | $10^3$ |
| 250 | No movement | - |

Table 7 shows results of the analysis of the FET characteristics in regard to a compound 32 (selenophene substituted hexyl derivative) represented by the general formula (1), synthesized as shown in a reaction formula (T).

Table 7

| Annealing temperature [°C] | OTS-treated | |
|---|---|---|
| | Mobility [$cm^2$/Vs] (Average) | On/off ratio (Maximum) |
| Without annealing | No movement | - |
| 50 | No movement | - |
| 100 | $1.72 \times 10^{-3}$ | $10^2$ |
| 150 | $3.62 \times 10^{-3}$ | $10^4$ |
| 200 | $1.77 \times 10^{-4}$ | $10^3$ |
| 250 | No movement | - |

Table 8 shows results of the analysis of the FET characteristics in regard to a compound 38 (selenophene substituted octyl derivative) represented by the general formula (1), synthesized as shown in a reaction formula (U).

Table 8

| Annealing temperature [°C] | OTS-treated | |
|---|---|---|
| | Mobility [$cm^2$/Vs] (Average) | On/off ratio (Maximum) |
| Without annealing | No movement | - |

(continued)

| Annealing temperature [°C] | OTS-treated | |
|---|---|---|
| | Mobility [$cm^2/Vs$] (Average) | On/off ratio (Maximum) |
| 50 | $1.31 \times 10^{-3}$ | $10^3$ |
| 100 | $1.17 \times 10^{-3}$ | $10^2$ |
| 150 | $4.64 \times 10^3$ | $10^4$ |
| 200 | $1.93 \times 10^{-3}$ | $10^3$ |
| 250 | No movement | - |

(Analysis of conductivity)

Conductivity of the compounds 13 and 18 were measured by a two-terminal method using a pellet and compared to conductivity of the compound 30 and a compound (hereinafter referred to as "compound 20") represented by the general formula (14) according to Non-Patent Literature 1.

Chem. 50

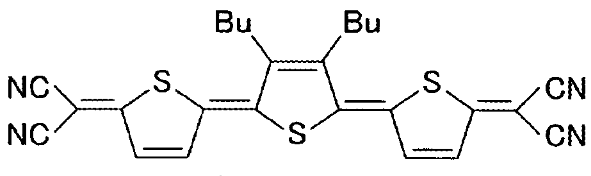

··· (14)

20

The compound 20 is a trimer containing unsubstituted thiophenes, thereby being largely polarized. This contributes to an increase in interaction between molecules. Therefore, it is considered that the compound 20 has a high conductivity. The conductivity of the compound 20 was $1.7 \times 10^{-6}$ (S/cm). In contrast, it is considered that the compound 30 has a low conductivity because the compound 30 contains butoxymethyl groups, which are large in size, and therefore cause a decrease in interaction between molecules. The conductivity of the compound 30 was not more than $10^{-11}$ (S/cm). Conductivity of the compounds 13 and 18 was not more than $10^{-11}$ (S/cm) like the compound 30.

(V) Conclusion

In the present invention, a hybrid compound was synthesized by combining unsubstituted thiophene or selenophene, which has a high conductivity, to a compound 7, which is a monomer unit (cyclopentane ring-fused thiophene) having a high solubility. It was determined by $^1$H-NMR that a compound 13 includes geometric isomers. For the purpose of controlling an occurrence of the geometric isomers, a compound 18 was produced with a methyl group introduced therein. These compounds were analyzed in electronic absorption spectrum, a CV oxidation-reduction potential, FET characteristics, and conductivity.

According to the analysis of electronic absorption spectrum, it was found that having a quinoid structure causes an absorption region to expand to a near-infrared region. The compound 13 did not largely differ in absorption range from the compound 18, which contains the methyl group. However, the compound 13 exhibited a characteristic absorption at approximately 420nm. It was considered that this absorption was attributed to the geometric isomers.

According to the CV measurement, it was found that both of the compounds 13 and 18 have an amphoteric redox property and are low in LUMO level so as to be easily subjected to a charge injection. This indicates that the compounds 13 and 18 can exhibit n-type FET characteristics.

In the evaluation of FET characteristics, the compound 13 exhibited the n-type FET characteristics. According to the FET characteristics of the compound 13, it was found that the compound 13 had a higher mobility than a compound 30. The compound 18 had a lower mobility than the compound 13 and did not exhibit the FET characteristics to the same extent as the compound 13. It is considered that this is because crystallization on a substrate prevented uniform formation of a film.

Why the compound 18 could not uniformly form the film is hypothesized to be due to the following reason: The compound 18 has a higher degree of crystallization than the compound 13 due to introduction of a methyl group and therefore has a lower film-forming ability. This prevents formation of a continuous semiconductor channel. As a result, it becomes difficult for electric current to pass through. In view of this, the compound 18 also can be used as an organic semiconductor material of the present invention, only provided that the compound 18 can uniformly form the film.
As described above, since the compound 13 has a similar solubility to the compound 30 and exhibits the n-type FET characteristics, the compound 13 is usefully applicable to an n-type FET material that can be welded.
The compound 18 has a similar solubility to the compound 30 and therefore is applicable to welding. In addition, the compound 18 has a LUMO energy high enough to exhibit the n-type FET characteristics so that a charge injection easily occurs. In view of this, it is considered that the compound 18 also exhibits the n-type FET characteristics like the compound 13, provided that the film is uniformly formed on the substrate. Therefore, the compound 18 can be usefully applicable to the n-type FET material that can be welded.

(VI) Concrete methods for synthesis of compounds 1 through 18 and 23 through 38

Compounds 1 through 18 shown in reaction formulas (A) through (R) and compounds 23 through 38 shown in reaction formulas (S) through (U) were synthesized by the following operations. In the operations, solvents distilled in anhydrous state were used in reactions or measurements under an inactive gas atmosphere; and first-class or special-class commercially available solvents were used in other reactions or operations. Reagents were purified by distillation in anhydrous state or the like, if necessary. If not, first-class or special-class commercially produced reagents were used. Purification by column chromatography was carried out by using Daiso gel IR-60 (silica gel, active) and MERCK Art 1097 Aluminiumoxide 90 (alumina, active). TLC was carried out by using Silicagel 60F254 (MERCK). A rotary evaporator was used in distilling the solvents away. Analytical instruments and measuring instruments used in the operations were described below.
Liquid chromatography (hereinafter referred to as "L.C.") was carried out by using Japanese Analytical Industry LC-08, LC-9204. Melting point determination (hereinafter referred to as "m.p.") was carried out by using Yanagimoto Micro Melting Point Apparatus MP-3S (uncorrected). Nuclear magnetic resonance spectroscopy (hereinafter referred to as "$^1$H-NMR") was carried out by using HITACHI RS-1200 nuclear magnetic resonance apparatus (60MHz, $\sigma$ value, ppm, internal standard TMS) and JEOL LAMBDA-NMR (395.75MHz, $\sigma$ value, ppm, internal standard TMS). Mass spectroscopy (hereinafter referred to as "MS") was carried out by using MALDI-MS KRATOS ANALYYTICAL KOMPACT MALDI and SHIMADZU GCMS-QP5050 mass spectrometer. Elemental analysis (hereinafter referred to as "Anal.") was carried out by using Parkin Elmer 2400CHN elemental analyzer (request analysis). Ultraviolet-visible absorption spectroscopy (hereinafter referred to as "U.V.") was carried out by using SHIMADZU spectrophotometer UV-3100. Electrochemical measurement by cyclic voltammogram method (hereinafter referred to as "CV") was carried out by using Hokuto Denko HA-301 potentiostat and Hokuto Denko HB-104 function generator.

(Compound 1)

An aqueous solution (700ml) of sodium sulfide nonahydrate (400g, 1.67mol) was added in drops to a methanol (800ml) solution of methyl chloroacetate (300ml, 3.42mol) over 3 hours, and then refluxed for 4 hours. After a reaction was completed, the resulting solution was cooled down to a room temperature, and methanol was removed therefrom. The resulting solution was subjected to extraction using methylene chloride (200mlx3) and then dried with anhydrous magnesium sulfate. After removing the solvent, the obtained yellow oily substance was distilled under a reduced pressure (95°C or more and 106°C or less, 7mmHg), so that a compound 1 (138g, 0.79mol, 47%) was obtained in the form of a transparent and colorless oily substance.
A result of analysis with regard to the compound 1 is described below.
$^1$H-NMR (60MHz, $CDCl_3$): $\delta$3.37 (s, 4H), 3.72 (s, 6H)

(Compound 2)

Under a nitrogen atmosphere, natrium (40g, 1.74mol) was dissolved in ethanol (500ml) in a water bath. Then, an ethanol (200ml) solution of 2,3-butanedione (50ml, 0.57mol) and the compound 1 (83ml, 0.57mol) was added thereto in drops over 2 hours. After the resulting solution was stirred at a room temperature for 4 hours, 2N aqueous sodium hydroxide (200ml) was added to the solution, and then refluxed for 4 hours. After a reaction in the resulting solution was completed, ethanol was removed from the solution. Then, 5N concentrated hydrochloric acid was added to the solution until the solution was acidified. As a result, a brown solid substance was precipitated therein. The brown solid substance was isolated by filtration, washed with water (100m1×3), and then dried with a heater. As a result, a roughly-purified compound 2 (56.5g, 0.28mol, 50%) was obtained in the form of a brown solid substance.

The compound 2 was used in synthesizing a compound 3 without further purifying.

(Compound 3)

Sodium hydroxide (11.5g, 0.29mol) was dissolved in water (300ml), and the compound 2 (25g, 0.12mol) was added thereto. The resulting solution was kept at 65°C within a range from pH7 to pH8 while bromine (46.8g, 15ml, 0.29mol) was added in drops to the solution over 3 hours, and then stirred at 65°C for 1hour. After a reaction in the solution was completed, the solution was cooled down to a room temperature. Then, 2N hydrochloric acid was added to the solution until the solution was acidified, so that a solid substance was obtained. The solid substance was isolated by filtration using celite, and washed with methylene chloride (200m1×3). Next, a filtrate was subjected to extraction using methylene chloride (150ml×3), thereafter washed with saturated saline (100ml), and dried with anhydrous magnesium sulfate. After removing the solvent, the obtained yellow oily substance was subjected to column chromatography (silica gel, hexane, batch: methylene chloride), so that a component of Rf 0.7 was isolated. In this way, a compound 3 (27.6g, 0.10mol, 82%) was obtained in the form of a faintly-yellow oily substance.

A result of analysis with regard to the compound 3 is described below.
$^1$H-NMR (60MHz, $CDCl_3$): δ2.15 (s, 6H)

(Compound 4)

Under a nitrogen atmosphere, N-bromosuccinimide (26g, 0.15mol) and benzoyl peroxide (1.79g, 7.39mmol) were added to a carbon tetrachloride (130ml) solution of the compound 3 (20g, 74mmol). The reaction mixture thus obtained was reacted under reflux for 5 hours while being irradiated with a halogen lamp. After this reaction was completed, the reaction mixture was cooled down to a room temperature. The obtained solid substance was isolated by filtration using celite, and washed with carbon tetrachloride (100ml×3). After removing the solvent, the obtained yellow solid substance was subjected to column chromatography (silica gel, hexane, batch: methylene chloride), so that a component of Rf 0.2 was isolated. The component dissolved in hexane was recrystallized. As a result, a compound 4 (27.6g, 87%) was obtained in the form of a colorless plate crystal.

A result of analysis with regard to the compound 4 is described below.
$^1$H-NMR (60MHz, $CDCl_3$): δ4.55 (s, 4H)

(Compound 5)

Under a nitrogen atmosphere, a THF (200ml) solution of the compound 4 (5g, 11.7mmol) and sodium hydride (60%, in oil, 1.05g, 26.3mmol) was refluxed, and a THF (20ml) solution of diethyl malonate (1.83ml, 12.1mmol) was added thereto in drops over 3 hours. Then, the resulting solution was stirred for 30 minutes. After a reaction in the solution was completed, water was added to the solution on ice. The obtained solid substance was isolated by filtration using celite, and washed with methylene chloride (100ml×3). A filtrate was subjected to extraction using methylene chloride (100ml×3) and then dried with anhydrous magnesium sulfate. After removing the solvent, the obtained yellow solid substance was subjected to column chromatography (silica gel, hexane: methylene chloride = 1:1, batch: methylene chloride), so that a component of Rf 0.5 was isolated. The component dissolved in hexane was recrystallized. As a result, a compound 5(3.65g, 8.57mmol, 73%) was obtained in the form of a colorless plate crystal.

A result of analysis with regard to the compound 5 is described below.
$^1$H-NMR (60MHz, $CDCl_3$): δ1.25 (t, J=7Hz, 6H), 3.24 (s, 4H), 4.20 (q, J=7Hz, 4H)

(Compound 6)

Under a nitrogen atmosphere, on ice, a THF (23ml) solution of the compound 5 (5g, 11.7mmol) was added in drops to a THF (100ml) solution of lithium aluminum hydride (1.79g, 47mmol) over 1 hour, and then refluxed for 20 hours. After a reaction in the resulting solution was completed, ethyl acetate (28ml), water (4ml), and 1N hydrochloric acid (4ml) were added in this order to the solution on ice. The obtained solid substance was isolated by filtration using celite, and washed with acetone (100ml×3), and thereafter the solvent was removed from the solid substance. Then, the obtained white solid substance was subjected to column chromatography (silica gel, ethyl acetate batch: acetone), so that a compound of Rf 0.5 was isolated. The compound dissolved in a mixed solution of ethyl acetate: chloroform = 1:4 was recrystallized. As a result, a compound 6 (3.65g, 8.57mmol, 73%) was obtained in the form of a colorless needle-like crystal.

A result of analysis with regard to the compound 6 is described below.
$^1$H-NMR (400MHz, $CDCl_3$): δ2.28 (t, J=5.12Hz, 2H), 2.62 (s, 4H), 3.77 (d, J=4.88Hz, 4H), 6.79 (s, 2H)

(Compound 7)

Under a nitrogen atmosphere, sodium hydride (60%, in oil, 2.2g, 55.0mmol) was added to a DMF (30ml) solution of the compound 6 (2.5g, 13.6mmol), and thereafter stirred at a room temperature for 30 minutes. Then, n-butyl bromide (4.95g, 3.9ml, 32.8mmol) was added in drops thereto on ice over 1 hour, and thereafter stirred at a room temperature for 24 hours. After a reaction in the resulting solution was completed, water was added to the solution, and then the solution was subjected to extraction using hexane (150ml×3). The obtained yellow oily substance was subjected to column chromatography (silica gel, methylene chloride), so that a component of Rf 0.8 was isolated. In this way, a compound 7 (3.92g, 13mmol, 97%) was obtained in the form of a colorless oily substance.

A result of analysis with regard to the compound 7 is described below.

$^1$H-NMR (400MHz, $CDCl_3$): δ0.91 (t, J=7.56Hz, 6H), 1.37 (sext, J=7.32Hz, 4H), 1.54 (quint, J=6.36Hz, 4H), 2.59 (s, 4H), 3.37 (s, 4H), 3.41 (t, J=6.60Hz, 4H), 6.73(s, 2H)

(Compound 8)

N-bromosuccinimide (1.58g, 8.88mmol) was added to a DMF (50ml) solution of the compound 7 (1.0g, 3.38mmol), and then stirred at a room temperature for 4 hours. After a reaction in the resulting solution was completed, a saturated sodium hydrogen carbonate aqueous solution (20ml) was added to the solution, and then stirred for 30 minutes. The resulting solution was subjected to extraction using hexane (50ml×3), thereafter washed with saturated saline (100ml), and dried with anhydrous magnesium sulfate. After removing the solvent, the obtained orange-colored oily substance was subjected to column chromatography (silica gel, methylene chloride), so that a component of Rf 0.9 was isolated. In this way, a compound 8 (1.40g, 3.08mmol, 91%) was obtained in the form of a faintly-yellow oily substance.

A result of analysis with regard to the compound 8 is described below.

$^1$H-NMR (400MHz, $CDCl_3$): δ0.91 (t, J=7.32Hz, 6H), 1.25 (sext, J=7.56Hz, 4H), 1.53 (quint, J=6.40Hz, 4H), 2.54 (s, 4H), 3.35 (s, 4H), 3.41 (t, J=6.56Hz, 4H)

(Compound 9)

Under a nitrogen atmosphere, a THF (20ml) solution of thiophene (0.94ml, 11.9mmol) was cooled down to -78°C, and then 1.6M n-BuLi (7.70ml, 12.0mmol) was slowly added thereto in drops. The resulting solution was stirred for 1 hour while being increased in temperature to a room temperature. The solution was cooled again down to -78°C. Then, tributyltin chloride (3.30ml, 12.1mmol) was added thereto and thereafter stirred for 2 hours. Water (80ml) was added to the resulting solution. Then, the resulting solution was subjected to extraction using methylene chloride (100ml×3), and dried with anhydrous magnesium sulfate. After removing the solvent, the resultant was subjected to column chromatography (alumina, hexane) and then purified by liquid chromatography (JAIGEL, 1H/2H, chloroform), so that a compound 9 (2.00g, 5.36mmol, 45%) was obtained in the form of a yellow oily substance.

A result of analysis with regard to the compound 9 is described below.

$^1$H-NMR (400MHz, $CDCl_3$): δ0.89 (t, J=7.32Hz, 9H), 1.10 (t, J=8.32Hz, 6H), 1.33 (sext, J=7.32Hz, 6H), 1.53-1.61 (m, 6H), 7.20 (dd, J=0.50, 3.16Hz, 1H), 7.26 (dd, J=3.16, 4.40Hz, 1H), 7.66 (dd, J=0.50, 4.40Hz, 1H)

(Compound 10)

Under a nitrogen atmosphere, argon gas was injected for 30 minutes to an anhydrous toluene solution (60ml) of the compound 8 (150mg, 0.33mmol) and the compound 9 (295mg, 0.79mmol), so that the solution was deaerated. Then, in a reaction container shielded from light, $Pd(PPh_3)_4$ (91 mg, 0.79μmol) was added to the solution and then refluxed for 13 hours. After a reaction was completed, the resulting solution was cooled down to a room temperature and filtered with celite. Then, the solvent was removed from the obtained solution. After that, the obtained red solid substance was subjected to column chromatography (silica gel, chloroform), so that a component of Rf 0.9 was isolated. The component was recrystallized with ethanol. As a result, a compound 10 (133mg, 0.29mmol, 88%) was obtained in the form of a faintly-yellow solid substance.

Results of analysis with regard to the compound 10 are described below.

Melting point: 101-102°C;

$^1$H-NMR (400MHz, $CDCl_3$): δ0.91 (t, J=7.60Hz, 6H), 1.36 (sext, J=7.30Hz, 4H), 1.53 (quint, J=6.30Hz, 4H), 2.75(s, 4H), 3.43 (t, J=6.6Hz, 4H), 3.44 (s, 4H), 7.02 (dd, J=3.68, 5.16Hz, 2H), 7.10 (dd, J=0.96, 3.68Hz, 2H), 7.21 (dd, J=0.96, 5.16Hz, 2H);

MS (MALDI-TOF) m/z = 459.91 ($M^+$) (calcd. = 460.16);

Anal.Calcd for $C_{25}H_{32}O_2S_3$ :C, 65.17;H, 7.00%;

Found: C, 65.01; H, 6.94%

(Compound 11)

N-bromosuccinimide (235mg, 1.04mmol) was added to a chloroform (20ml) solution of the compound 10 (200mg, 0.43mmol) on ice and then stirred at a room temperature for 5 hours. After a reaction in the resulting solution was completed, a saturated sodium hydrogen carbonate aqueous solution (10ml) was added to the solution and thereafter stirred for 30 minutes. Then, the solution was subjected to extraction using chloroform (50mlx3), thereafter washed with saturated saline (100ml), and dried with anhydrous magnesium sulfate. After removing the solvent, the obtained yellow oily substance was subjected to column chromatography (silica gel, chloroform), so that a component of Rf 0.9 was isolated. The component was recrystallized with ethanol. As a result, a compound 11 (133mg, 0.15mmol, 71%) was obtained in the form of a yellow solid substance.

Results of analysis with regard to the compound 11 are described below.
$^1$H-NMR (400MHz, $CDCl_3$): δ0.91 (t, J=7.60Hz, 6H), 1.36 (sext, J=7.30Hz, 4H), 1.53 (quint, J=6.30Hz, 4H), 2.69 (s, 4H), 3.41-3.44 (m, 8H), 6.82 (d, J=4.16Hz, 2H), 6.95 (d, J=3.92Hz, 2H);
MS (DI) m/z = 615 ($M^+$) (calcd. =615)

(Compound 12)

N-iodosuccinimide (77mg, 0.43mmol) was added to a chloroform (10ml) solution of the compound 10 (100mg, 0.22mmol) and then stirred at a room temperature for 5 hours. After a reaction in the resulting solution was completed, a saturated sodium hydrogen carbonate aqueous solution (10ml) was added to the solution and thereafter stirred for 30 minutes. Then, the solution was subjected to extraction using chloroform (50mlx3), thereafter washed with saturated saline (100ml), and dried with anhydrous magnesium sulfate. After removing the solvent, the obtained yellow oily substance was subjected to column chromatography (silica gel, hexane: methylene chloride = 1:1), so that a component of Rf 0.8 was isolated. The component was recrystallized with ethanol. As a result, a compound 12 (273mg, 0.38mmol, 88%) was obtained in the form of a yellow solid substance.

Results of analysis with regard to the compound 12 are described below.
Melting point: 85-87°C;
$^1$H-NMR (400MHz, $CDCl_3$): δ0.89 (t, J=7.30Hz, 6H), 1.36 (sext, J=7.60Hz, 4H), 1.53 (quint, J=5.20Hz, 4H), 2.69 (s, 4H), 3.41-3.43 (m, 8H), 6.75 (d, J=3.92Hz, 2H), 7.15 (d, J=3.92Hz, 2H)

(Compound 13)

Under a nitrogen atmosphere, sodium hydride (60%, in oil, 112mg, 4.67mmol) and malononitrile (152mg, 2.30mmol) were added to a THF (45ml) solution and then stirred at a room temperature for 30 minutes. To the resulting solution, in a reaction container shielded from light, $Pd(PPh_3)_4$ (26mg, 23μmol) and dppf (26mg, 47μmol) were added, and then a THF (15ml) solution of the compound 11 (143mg, 0.23mmol) was added thereto in drops over 20 minutes and thereafter refluxed for 20 hours. After a reaction in the solution was completed, a 2N hydrochloric acid aqueous solution was added thereto and then stirred for 30 minutes. Then, the solution was subjected to extraction using chloroform (50ml×3), thereafter washed with saturated saline (100ml), and dried with anhydrous magnesium sulfate. After removing a solvent, the obtained yellow oily substance was subjected to column chromatography (10% low-active silica gel, chloroform), so that a component of Rf 0.6 was isolated. The compound was recrystallized with acetone-acetonitrile. As a result, a compound 13 (271mg, 0.44mmol, 72%) was obtained in the form of a green solid substance.

Another method for synthesizing the compound 13 is described below. Under a nitrogen atmosphere, sodium hydride (60%, in oil, 112mg, 4.67mmol) and malononitrile (152mg, 2.30mmol) were added to a THF (45ml) solution and then stirred at a room temperature for 30 minutes. To the resulting solution, in a reaction container shielded from light, $Pd(PPh_3)_4$ (26mg, 23μmol) and dppf (26mg, 47μmol) were added, and then a THF (15ml) solution of the compound 12 (280mg, 0.39mmol) was added thereto in drops over 20 minutes and thereafter refluxed for 22 hours. After a reaction in the solution was completed, a 2N hydrochloric acid aqueous solution was added thereto and then stirred for 2 hours. Then, the solution was subjected to extraction using chloroform (50mlx3), thereafter washed with saturated saline, and dried with anhydrous magnesium sulfate. After removing the solvent, the obtained yellow oily substance was subjected to column chromatography (10% low-active silica gel, chloroform), so that a component of Rf 0.6 was isolated. After removing the solvent, the component was subjected to $^1$H-NMR and IR. As a result, the component was determined as the compound 13.

Still another method for synthesizing the compound 13 was described below. Under a nitrogen atmosphere, sodium hydride (60%, in oil, 112mg, 4.67mmol) and malononitrile (152mg, 2.30mmol) were added to a THF (45ml) solution and then stirred at a room temperature for 30 minutes. To the resulting solution, in a reaction container shielded from light, $Pd(PPh_3)_4$ (26mg, 23μmol) and dppf (26mg, 47μmol) were added, and then a THF (15ml) solution of the compound 11 (143mg, 0.23mmol) was added thereto in drops over 20 minutes and thereafter refluxed for 20 hours.

After a reaction in the solution was completed, saturated bromine water (10ml) was added thereto and then stirred for 30 minutes. Then, the solution was subjected to extraction using chloroform (50mlx3), thereafter washed with saturated saline (100ml), and dried with anhydrous magnesium sulfate. After removing the solvent, the obtained substance was subjected to $^1$H-NMR and IR. As a result, the substance was determined not to correspond to the compound 13.

Results of analysis with regard to the compound 13 are described below.

Melting point: degraded at 300°C;

$^1$H-NMR (400MHz, $CDCl_3$): $\delta$0.91 (t, J=7.56Hz, 6H), 1.36 (sext, J=7.56Hz, 4H), 1.53 (quint, J=5.20Hz, 4H), 2.83-2.92 (m, 4H), 3.42-3.48 (m, 4H), 7.22-7.24 (dd, J=2.96, 5.64Hz, 1H), 7.32-7.36 (dd, J=4.64, 5.88Hz, 1H), 7.40-7.42 (dd, J=2.68, 5.64Hz, 1H), 7.52-7.55 (dd, J=4.64, 5.60Hz, 1H);
MS (DI) m/z =586($M^+$) (calcd. =586); IR (KBr) 2208.8cm$^{-1}$;
Anal. Calcd for $C_{31}H_{30}N_4O_2S_3$: C, 63.45; H, 5.15; N, 9.55%;
Found: C, 63.44; H, 5.25; N, 9.39%

(Compound 14)

A DMF (90ml) solution of N-bromosuccinimide (35.6g, 0.20mol) was slowly added in drops to a DMF (65ml) solution of 3-methylthiophene (19.6g, 0.20mol) in an ice bath over 1 hour and then stirred at a room temperature for 12 hours. After a reaction in the resulting solution was completed, a saturated sodium hydrogen carbonate aqueous solution (100ml) was added thereto and then stirred for 30 minutes. Then, the solution was subjected to extraction using hexane (100ml×3), thereafter washed with water (100ml), and dried with anhydrous magnesium sulfate. After removing the solvent, the obtained transparent oily substance was distilled under a reduced pressure (45-62°C, 15mmHg), so that a compound 14 (21.7g, 122mmol, 61%) was obtained in the form of a transparent and colorless oily substance.

Results of analysis with regard to the compound 14 are described below.
$^1$H-NMR (400MHz, $CDCl_3$): $\delta$2.19 (s, 3H), 6.77 (d, J=5.60Hz, 1H), 7.15 (d, J=5.64, 1H);
MS (DI) m/z =176($M^+$) (calcd. =176)

(Compound 15)

Under a nitrogen atmosphere, a THF (20ml) solution of 3-methylthiophene (379mg, 3.87mmol) was cooled down to -78°C, and 1.6M n-BuLi (2.50ml, 4.08mmol) was slowly added thereto in drops. The resulting solution was increased in temperature to a room temperature, stirred for 1 hour, and cooled again down to -78°C. Tributyltine chloride (1.10ml, 4.08mmol) was added thereto and then stirred for 2 hours. Then, water (80ml) was added to the resulting solution. The solution was subjected to extraction using methylene chloride, and dried with anhydrous magnesium sulfate. After removing the solvent, the obtained substance was subjected to column chromatography (alumina, hexane), so that a component of Rf 0.6 was isolated. After removing a solvent, the component was subjected to $^1$H-NMR. As a result, the component was determined to be a mixture (800mg, 2.06mmol) constituted by a compound 15 and 5-Tributhylstannyl-3-methylthiophene at a ratio of 1:4.

Another method for synthesizing the compound 15 is described below. Under a nitrogen atmosphere, a THF (20ml) solution of the compound 14 (685mg, 3.87mmol) was cooled down to -78°C, and 1.6M n-BuLi (2.40ml, 3.84mmol) was slowly added thereto in drops. The obtained solution was increased in temperature to a room temperature, stirred for 1 hour, and cooled again down to -78°C. Tributyltine chloride (1.10ml, 4.08mmol) was added thereto and then stirred for 2 hours. Then, water was added to the resulting solution. The solution was subjected to extraction using methylene chloride (100ml×3), and dried with anhydrous magnesium sulfate. After removing the solvent, the obtained substance was subjected to column chromatography (alumina, hexane), so that a component of Rf 0.6 was isolated. After removing a solvent, the component was subjected to $^1$H-NMR. As a result, the component was determined to be a mixture (1.11g, 2.86mmol) constituted by the compound 15 and 5-Tributhylstannyl-3-methylthiophene at a ratio of 7:1.

The compound 15 could not be used in synthesizing a compound 18 because of being obtained as the mixture with 5-Tributhylstannyl-3-methylthiophene.

(Compound 16)

Under a nitrogen atmosphere, magnesium (600mg, 24.7mmol) was put into a reaction container, and the reaction container was dried. Then, a THF (45ml) solution was poured into the reaction container. To the THF solution, 1,2-dibromoethane (0.1ml, 1.16mmol) was added if necessary. The compound 14 (2.31ml, 20.5mmol) was slowly added in drops to the THF solution and then refluxed for 3 hours. As a result, a Grignard reagent was obtained.

Under a nitrogen atmosphere, $Pd(dppf)Cl_2$ was added to a THF (45ml) solution of the compound 8 (1.42g,

3.12mmol) and then stirred. Approximately 30ml of the Grignard reagent was transferred to a dripping funnel so as to be slowly added in drops to the resulting solution. After 12 hours of reflux, a saturated ammonium chloride aqueous solution (100ml) was added to the solution in an ice bath. Then, the solution was filtered with celite, subjected to extraction using chloroform (100mlx3), washed with a saturated sodium hydrogen carbonate aqueous solution (300ml), saturated saline (300ml), and water (300ml), and dried with anhydrous magnesium sulfate. After removing the solvent, the obtained substance was subjected to column chromatography (silica gel, hexane: methylene chloride = 2:1), so that a component of Rf 0.2 was isolated. The component was recrystallized with ethanol. As a result, a compound 16 (860mg, 1.76mmol, 57%) was obtained in the form of a yellow needle-like crystal.

Results of analysis with regard to the compound 16 are described below.
Melting point: 63-64°C;
$^1$H-NMR (400MHz, $CDCl_3$): δ0.89 (t, J=7.36Hz, 6H), 1.36 (sext, J=7.56Hz, 4H), 1.53 (quint, J=6.60Hz, 4H), 2.39 (s, 6H), 2.73 (s, 4H), 3.40-3.43 (m, 8H), 6.86 (d, J=5.36Hz, 2H), 7.16 (d, J=5.24Hz, 2H);
MS (DI) m/z =488($M^+$) (calcd. =488);
Anal. Calcd for $C_{27}H_{36}O_2S_3$: C, 66.35; H, 7.42%;
Found: C, 66.36; H, 7.41%

(Compound 17)

A DMF (20ml) solution of N-bromosuccinimide (541mg, 3.04mmol) was slowly added in drops to a chloroform (30ml) solution of the compound 16 (743mg, 1.52mmol), and then stirred at a room temperature for 5 hours. After a reaction in the resulting solution was completed, a saturated sodium hydrogen carbonate aqueous solution (10ml) was added thereto and then stirred for 30 minutes. The resulting solution was subjected to extraction using chloroform (50mlx3), thereafter washed with saturated saline (100ml), and dried with anhydrous magnesium sulfate. After removing a solvent, the obtained yellow oily matter was subjected to column chromatography (silica gel, hexane: methylene chloride = 2:1), so that a component of Rf 0.6 was isolated. The component was recrystallized with ethanol. As a result, a compound 17 (837mg, 1.29mmol, 85%) was obtained in the form of a yellow oily substance.

Results of analysis with regard to the compound 17 are described below.
$^1$H-NMR (400MHz, $CDCl_3$): δ0.90 (t, J=7.36Hz, 6H), 1.36 (sext, J=7.56Hz, 4H), 1.53 (quint, J=6.60Hz, 4H), 2.30 (s, 6H), 2.68 (s, 4H), 3.39-3.42 (m, 8H), 6.81 (s, 2H);
MS (DI) m/z =646($M^+$) (calcd. =646)

(Compound 18)

Under a nitrogen atmosphere, sodium hydride (60%, in oil, 112mg, 4.67mmol) and malononitrile (154mg, 2.34mmol) were added to a THF (45ml) solution and then stirred at a room temperature for 30 minutes. To the resulting solution, in a reaction container shielded from light, $Pd(PPh_3)_4$ (27mg, 23.4μmol) and dppf (27mg, 46.8μmol) were added, and then a THF (15ml) solution of the compound 17 (151mg, 0.23mmol) was added thereto in drops over 20 minutes and thereafter refluxed for 20 hours. After a reaction in the solution was completed, a 2N hydrochloric acid aqueous solution was added thereto, and then stirred for 30 minutes. The resulting solution was subjected to extraction using chloroform (50ml×3), thereafter washed with saturated saline (100ml), and dried with anhydrous magnesium sulfate. After removing the solvent, the obtained yellow oily substance was subjected to column chromatography (10% low-active silica gel, chloroform), so that a component of Rf 0.6 was isolated. The component was recrystallized with acetone- acetonitrile. As a result, a compound 18 (90mg, 0.146mmol, 63%) was obtained in the form of a green solid substance.

Results of analysis with regard to the compound 18 are described below.
Melting point: degraded at 300°C;
$^1$H-NMR (400MHz, $CDCl_3$): δ0.91 (t, J=7.32Hz, 6H), 1.35 (sext, J=7.56Hz, 4H), 1.53 (quint, J=6.60Hz, 4H), 2.56 (d, J=0.72Hz, 6H), 2.96 (s, 4H), 3.43-3.48 (m, 8H), 7.15 (d, J=0.96Hz, 2H); MS (MALDI-TOF) m/z =613.81($M^+$) (calcd. =614.18);
IR (KBr) 2208.8$cm^{-1}$;
Anal. Calcd for $C_{33}H_{34}N_4O_2S_3$: C, 64.46; H, 5.57; N, 9.11%; Found: C, 64.36; H, 5.59; N, 9.04%

(Compound 23)

Under a nitrogen atmosphere, argon gas was injected for 30 minutes to an anhydrous toluene solution (30ml) of the compound 5 (500mg, 1.17mmol) and the compound 9 (962mg, 2.58mmol), so that the solution was deaerated. In a reaction container shielded from light, $Pd(PPh_3)_4$ (14mg, 12.1μmol) was added to the solution and then refluxed for 20 hours. After a reaction was completed, the resulting solution was cooled down to a room temperature and filtered

with celite. Then, a solvent was removed from the obtained solution. Then, the obtained yellow solid substance was subjected to column chromatography (silica gel, hexane: methylene chloride = 2:1), so that a component of Rf 0.3 was isolated. The component was recrystallized with a mixed solvent of hexane-methylene chloride. As a result, a compound 23 (422mg, 0.98mmol, 83%) was obtained in the form of a faintly-yellow plate-like solid substance.

Results of analysis with regard to the compound 23 are described below.
Melting point: 112-113°C;
$^1$H-NMR (60MHz, $CDCl_3$): δ1.26 (t, J=7.20Hz, 6H), 3.50 (s, 4H), 4.23 (q, J=7.20Hz, 4H), 7.02 (dd, J=3.60, 5.20Hz, 2H), 7.10 (dd, J=1.60, 3.60Hz, 2H), 7.24 (dd, J=1.60, 5.20Hz, 2H); $^{13}$C-NMR (100MHz, $CDCl_3$): δ13.9, 35.8, 61.9, 65.5, 123.6, 124.5, 125.3, 127.7, 136.4, 140.5, 170.9:
MS (DI) m/z =432($M^+$) (calcd. =432);
Anal. Calcd for $C_{21}H_{20}O_4S_3$: C, 58.31; H, 4.66%;
Found: C, 58.25; H, 4.72%

(Compound 24)

Under a nitrogen atmosphere, on ice, a THF (25ml) solution of the compound 23(400mg, 0.93mmol) was added in drops to a THF (25ml) solution of lithium aluminum hydride (140mg, 3.70mmol), and then refluxed for 20 hours. After a reaction in the resulting solution was completed, ethyl acetate (28ml), water (4ml), and 1N hydrochloric acid (4ml) was added in this order to the solution on ice. The obtained solid substance was isolated by filtration using celite and then washed with acetone (100ml×3), and the solvent was removed from the substance. As a result, a roughly-purified compound 24 was obtained in the form of an ocher solid substance.

The compound 24 was used in synthesizing compounds 25a, 25b, and 25c without further purifying.

(Compound 25a)

Under a nitrogen atmosphere, sodium hydride (60%, in oil, 41mg, 1.72mmol) was added to a DMF (20ml) solution of the compound 24 (100mg, 0.29mmol), and then stirred for 30 minutes at a room temperature. To the resulting solution on ice, n-hexyl bromide (117mg, 0.10ml, 6.25mmol) was slowly added in drops over 1 hour, and then stirred for 24 hours at a room temperature. Water was added to the solution, so as to terminate the reaction in the solution. Then, the solution was subjected to extraction using hexane (150ml×3), and the solvent was removed from the obtained solution. Then, the obtained yellow oily substance was subjected to column chromatography (silica gel, hexane: methylene chloride = 2:1), so that a component of Rf 0.4 was isolated. The component was recrystallized with ethanol. As a result, a compound 25a (103mg, 0.20mmol, 70%) was obtained in the form of a faintly-yellow needle-like solid substance.

Results of analysis with regard to the compound 25a are described below.
Melting point: 65-66°C;
$^1$H-NMR (400MHz, $CDCl_3$): δ0.85-0.89 (t, J=6.84Hz, 6H), 1.25-1.52 (m, 12H), 1.53-1.58 (m, 4H), 2.75 (s, 4H), 3.41-3.44 (m, 8H), 7.00-7.03 (dd, J=3.64, 5.12Hz, 2H), 7.09-7.11 (dd, J=1.00, 3.64Hz, 2H), 7.21-7.22 (dd, J=1.00, 5.12Hz, 2H); $^{13}$C-NMR (100MHz, CDCl3): δ14.0, 22.6, 25.8, 29.5, 31.6, 34.4, 55.2, 71.5, 73.8, 123.2, 124.1, 125.1, 127.6, 137.2, 143.8; MS (DI) m/z =516($M^+$) (calcd. =516);
Anal. Calcd for $C_{29}H_{40}O_2S_3$: C, 67.39; H, 7.80%;
Found: C, 67.31; H, 7.63%

(Compound 26a)

In an ice bath, N-bromosuccinimide (201mg, 1.13mmol) was added to a chloroform (30ml) solution of the compound 25 (293mg, 0.57mmol) and then stirred for 5 hours at a room temperature. After the reaction in the resulting solution was completed, a saturated sodium hydrogen carbonate aqueous solution (10ml) was added to the solution and then stirred for 30 minutes. Then, the solution was subjected to extraction using chloroform (50ml×3), thereafter washed with saturated saline (100ml), and dried with anhydrous magnesium sulfate. After removing the solvent, the obtained yellow oily substance was subjected to column chromatography (silica gel, hexane: methylene chloride = 2:1), so that a component of Rf 0.5 was isolated. The component was recrystallized with a mixed solvent of methylene chloride-acetonitrile. As a result, a compound 26a (380mg, 0.56mmol, 99%) was obtained in the form of a yellow needle-like solid substance.

Results of analysis with regard to the compound 26a are described below.
$^1$H-NMR (400MHz, $CDCl_3$): δ0.88 (t, J=6.84Hz, 6H), 1.26-1.28 (m, 12H), 1.53-1.56 (m, 4H), 2.69 (s, 4H), 3.40-3.43 (m, 8H), 6.82 (d, J=3.92, 2H), 6.95 (d, J=3.88Hz, 2H);
$^{13}$C-NMR (100MHz, $CDCl_3$): δ14.0, 22.6, 25.8, 29.4, 31.6, 34.2, 55.4, 71.4, 73.7, 110.9, 123.2, 124.5, 130.4, 138.5, 144.2; MS (DI) m/z =674($M^{+2}$) (calcd. =674);

Anal. Calcd for $C_{29}H_{38}Br_2O_2S_3$: C, 51.63; H, 5.68%;
Found: C, 51.60; H, 5.54%

(Compound 27a)

Under a nitrogen atmosphere, sodium hydride (60%, in oil, 71mg, 2.96mmol) and malononitrile (98mg, 1.48mmol) were added to a THF (20ml) solution and then stirred for 30 minutes at a room temperature. To the resulting solution, in a reaction container shielded from light, $Pd(PPh_3)_4$ (17mg, 14.7μmol) and dppf (17mg, 30.7μmol) were added, and then a THF (30ml) solution of the compound 26a (100mg, 0.15mmol) was added thereto in drops over 20 minutes and thereafter refluxed for 20 hours. After a reaction in the resulting solution was completed, a 2N hydrochloric acid aqueous solution was added thereto and stirred for 30 minutes. Then, the solution was subjected to extraction using chloroform (50mlx3), thereafter washed with saturated saline, and dried with anhydrous magnesium sulfate. After removing a solvent, the obtained yellow oily substance was subjected to column chromatography (10% low-active silica gel, chloroform), so that a component of Rf 0.5 was isolated. The component was recrystallized with acetone-acetonitrile. As a result, a compound 27a (60mg, 0.98mmol, 63%) was obtained in the form of a green solid substance.

Results of analysis with regard to the compound 27a are described below.
Melting point: degraded at 300°C;
$^1$H-NMR (400MHz, $CDCl_3$): δ0.87 (t, J=6.84Hz, 6H), 1.25-1.30 (m, 12H), 1.53-1.56 (m, 4H), 2.83-2.92 (m, 4H), 3.42-3.46 (m, 8H), 7.21-7.26 (m, 2H), 7.33-7.36 (dd, J=5.60, 8.28Hz, 2H), 7.41-7.43 (dd, J=2.92, 5.60Hz, 2H), 7.52-7.56 (dd, J=5.60, 8.28Hz, 2H);
MS (DI) m/z=642($M^+$) (calcd. =642); IR (KBr) 2206.8cm$^{-1}$; Anal. Calcd for $C_{35}H_{38}N_4O_2S_3$: C, 65.39; H, 5.96; N, 8.71%;
Found: C, 65.44; H, 5.94; N, 8.62%

(Compound 25b)

Under a nitrogen atmosphere, sodium hydride (60%, in oil, 179mg, 7.47mmol) was added to a DMF (50ml) solution of the compound 24 (260mg, 0.75mmol) and then stirred for 30 minutes at a room temperature. To the resulting solution on ice, n-octyl bromide (474mg, 0.43ml, 2.46mmol) was slowly added in drops over 1 hour, and then stirred for 24 hours at a room temperature. Water was added to the solution, so as to terminate the reaction in the solution. Then, the solution was subjected to extraction using chloroform (150ml×3), and the solvent was removed from the obtained solution. Then, the obtained yellow oily substance was subjected to column chromatography (silica gel, hexane: methylene chloride = 2:1), so that a compound of Rf 0.5 was isolated. The compound was recrystallized with a mixed solvent of ethanol and methylene chloride. As a result, a compound 25b (340mg, 0.59mmol, 80%) was obtained in the form of a faintly-yellow needle-like crystal.

Results of analysis with regard to the compound 25b are described below.
Melting point: 83-84°C;
$^1$H-NMR (400MHz, $CDCl_3$): δ0.87 (t, J=6.84Hz, 6H), 1.25-1.57 (m, 4H), 2.75 (s, 4H), 3.41-3.43 (m, 8H), 7.02 (dd, J=3.68, 5.16Hz, 2H), 7.10 (dd, J=0.96, 3.64Hz, 2H), 7.21 (dd, J=1.00, 5.12Hz, 2H);
$^{13}$C-NMR (100MHz, $CDCl_3$): δ14.1, 22.6, 26.2, 29.3, 29.4, 29.5, 31.8, 34.4, 55.2, 71.5, 73.8, 123.2, 124.1, 125.1, 127.6, 137.2, 143.8;
MS (DI) m/z =572($M^+$) (calcd. =572);
Anal. Calcd for $C_{33}H_{48}O_2S_3$: C, 69.18; H, 8.44%;
Found: C, 69.32; H, 8.38%

(Compound 26b)

N-bromosuccinimide (194mg, 1.09mmol) was added to a chloroform (50ml) solution of the compound 25b (312mg, 0.55mmol) in an ice bath and then stirred for 5 hours at a room temperature. After a reaction was completed in the resulting solution, a saturated sodium hydrogen carbonate aqueous solution (10ml) was added thereto and then stirred for 30 minutes. Then, the solution was subjected to extraction using chloroform (50mlx3), thereafter washed with saturated saline (100ml), and dried with anhydrous magnesium sulfate. After removing a solvent, the obtained yellow oily substance was subjected to column chromatography (silica gel, hexane: methylene chloride = 2:1), so that a compound of Rf 0.4 was isolated. In this way, a compound 26b (378mg, 0.52mmol, 99%) was obtained in the form of a faintly-yellow needle-like crystal.

Results of analysis with regard to the compound 26b are described below.
$^1$H-NMR (400MHz, $CDCl_3$): δ0.87 (t, J=6.84Hz, 6H), 1.26-1.31 (m, 20H), 1.53-1.56 (m, 4H), 2.69 (s, 4H), 3.40-3.43 (m, 8H), 6.83 (d, J=3.88, 2H), 6.96 (d, J=3.92Hz, 2H);
$^{13}$C-NMR (100MHz, $CDCl_3$): δ14.1, 22.6, 26.2, 29.3, 29.4, 29.5, 31.8, 34.2, 55.4, 71.5, 73.7, 110.9, 123.1, 124.5, 130.4,

138.5, 144.2;
MS (DI) m/z =730($M^{+2}$) (calcd. =730)

(Compound 27b)

Under a nitrogen atmosphere, sodium hydride (60%, in oil, 248mg, 10.3mmol) and malononitrile (341mg, 5.18mmol) were added to a THF (20ml) solution and then stirred for 30 minutes at a room temperature. To the resulting solution, in a reaction container shielded from light, $Pd(PPh_3)_4$ (35mg, 30.3μmol) and dppf (35mg, 63.2μmol) were added, and then a THF (30ml) solution of the compound 26b (378mg, 0.52mmol) was added thereto in drops over 20 minutes and thereafter refluxed for 20 hours. After the reaction was completed in the resulting solution, a 2N hydrochloric acid aqueous solution was added thereto and then stirred for 30 minutes. Then, the solution was subjected to extraction using chloroform (50mlx3), thereafter washed with saturated saline (100ml), and dried with anhydrous magnesium sulfate. After removing the solvent, the obtained yellow oily substance was subjected to column chromatography (10% low-active silica gel, chloroform), so that a component of Rf 0.4 was isolated. The component was recrystallized with acetone-acetonitrile. As a result, a compound 27b (267mg, 0.38mmol, 73%) was obtained in the form of a green solid substance.

Results of analysis with regard to the compound 27b are described below.
Melting point: degraded at 300°C;
$^1$H-NMR (400MHz, $CDCl_3$): δ0.87 (t, J=6.84Hz, 6H), 1.24-1.30 (m, 20H), 1.54-1.57 (m, 4H), 2.83-2.93 (m, 4H), 3.44-3.47 (m, 8H), 7.23-7.26 (m, 2H), 7.33-7.36 (m, 8.28Hz, 2H), 7.41-7.43 (m, 2H), 7.52-7.56 (m, 2H);
MS (DI) m/z =699($M^+$) (calcd. =699); IR (KBr) 2206.57cm$^{-1}$; Anal. Calcd for $C_{39}H_{46}N_4O_2S_3$: C, 67.01; H, 6.63; N, 8.02%; Found: C, 67.16; H, 6.73; N, 7.92%

(Compound 25c)

Under a nitrogen atmosphere, sodium hydride (60%, in oil, 317mg, 13.2mmol) was added to a DMF (50ml) solution of the compound 24 (443mg, 1.32mmol) and then stirred for 30 minutes at a room temperature. To the resulting solution on ice, n-decyl bromide (875mg, 0.82ml, 3.96mmol) was slowly added in drops over 1 hour and then stirred for 24 hours at a room temperature. Water was added to the solution, so as to terminate the reaction in the solution. Then, the solution was subjected to extraction using methylene chloride (150ml×3), and the solvent was removed from the solution. Then, the obtained yellow oily substance was subjected to column chromatography (silica gel, hexane: methyl chloride = 1:1), so that a component of Rf 0.4 was isolated. The component was recrystallized with a mixed solvent of acetonitrile-methylene chloride. As a result, a compound 25c (598mg, 0.95mmol, 72%) was obtained in the form of a faintly-yellow needle-like crystal.

Results of analysis with regard to the compound 25c are described below.
Melting point: 73-74°C;
$^1$H-NMR (400MHz, $CDCl_3$): δ0.87 (T, J=6.84Hz, 6H), 1.25-1.35 (m, 28H), 1.51-1.56 (m, 4H), 2.75 (s, 4H), 3.40-3.45 (m, 8H), 7.01 (dd, J=3.68, 5.12Hz, 2H), 7.10 (dd, J=1.24, 3.68Hz, 2H), 7.21 (dd, J=1.24, 5.12Hz, 2H);
$^{13}$C-NMR (100MHz, $CDCl_3$): δ14.1, 22.6, 26.1, 29.3, 29.4, 29.5, 29.5, 29.6, 31.4, 34.4, 55.2, 71.4, 73.8, 123.2, 124.1, 125.0, 127.6, 137.2, 143.8;
MS (DI) m/z =629($M^+$) (calcd. =629);
Anal. Calcd for $C_{37}H_{56}O_2S_3$: C, 70.65; H, 8.97%;
Foung: C, 70.61; H, 9.16%

(Compound 26c)

N-bromosuccinimide (226mg, 1.27mmol) was added to a chloroform (60ml) solution of the compound 25c (400mg, 0.64mmol) in an ice bath and then stirred for 5 hours at a room temperature. After a reaction in the resulting solution was completed, a saturated sodium hydrogen carbonate aqueous solution (10ml) was added thereto and then stirred for 30 minutes. Then, the solution was subjected to extraction using chloroform (50mlx3), thereafter washed with saturated saline (100ml), and dried with anhydrous magnesium sulfate. After removing the solvent, the obtained yellow oily substance was subjected to column chromatography (silica gel, hexane: methylene chloride = 1:1), so that a component of Rf 0.6 was isolated. In this way, a compound 26c (449mg, 0.57mmol, 90%) was obtained in the form of a faintly-yellow needle-like crystal.

Results of analysis with regard to the compound 26c are described below.
$^1$H-NMR (400MHz, $CDCl_3$): δ0.87 (t, J=6.60Hz, 6H), 1.25-1.35 (m, 28H), 1.53-1.56 (m, 4H), 2.69 (s, 4H), 3.40-3.43 (m, 8H), 6.83 (d, J=3.92Hz, 2H), 6.96 (d, J=3.92Hz, 2H);
$^{13}$C-NMR (100MHz, $CDCl_3$): δ14.1, 22.6, 26.2, 29.3, 29.4, 29.5, 29.6, 29.6, 31.9, 34.2, 55.4, 71.5, 73.7, 110.9, 123.2,

124.6, 130.4, 138.5, 144.2;
MS (DI) m/z =786($M^{+2}$) (calcd. =786);
Anal. Calcd for $C_{37}H_{54}Br_2O_2S_3$: C, 56.48; H, 6.92%;
Found: C, 56.56; H, 6.98%

(Compound 27c)

Under a nitrogen atmosphere, sodium hydride (60%, in oil, 122mg, 5.08mmol) and malononitrile (168mg, 2.54mmol) were added to a THF (20ml) solution and then stirred for 30 minutes at a room temperature. To the resulting solution, in a reaction container shielded from light, $Pd(PPh_3)_4$ (29mg, 25.4μmol) and dppf (29mg, 52.3μmol) were added, and then a THF (30ml) solution of the compound 26c (200mg, 0.25mmol) was added thereto in drops over 20 minutes and thereafter refluxed for 20 hours. After a reaction in the resulting solution was completed, a 2N hydrochloric acid aqueous solution was added thereto and then stirred for 30 minutes. Then, the solution was subjected to extraction using chloroform (50ml×3), thereafter washed with saturated saline, and dried with anhydrous magnesium sulfate. After removing the solvent, the obtained yellow oily substance was subjected to column chromatography (10% low-active silica gel, chloroform), so that a component of Rf 0.3 was isolated. The component was recrystallized with acetone-acetonitrile. As a result, a compound 27c (140mg, 0.19mmol, 73%) was obtained in the form of a green solid substance.

Results of analysis with regard to the compound 27c are described below.
Melting point: degraded at 300°C;
$^1$H-NMR (400MHz, $CDCl_3$): δ0.87 (t, J=5.88Hz, 6H), 1.24-1.30 (m, 28H), 1.53-1.56 (m, 4H), 2.83-2.92 (m, 4H), 3.42-3.46 (m, 8H), 7.22-7.28 (m, 2H), 7.32-7.36 (m, 2H), 7.40-7.43 (m, 2H), 7.52-7.55 (m, 2H);
MS (DI) m/z =754($M^+$) (calcd. =754); IR (KBr) 2206.8$cm^{-1}$

(Compound 28)

Under a nitrogen atmosphere, sodium hydride (60%, in oil, 208mg, 8.68mmol) was added to a DMF (30ml) solution of an alcohol (400mg, 2.17mmol) and then stirred for 30 minutes at a room temperature. To the resulting solution on ice, n-hexyl bromide (1.08g, 0.96ml, 6.51mmol) was added in drops over 1 hour, and then stirred for 20 hours at a room temperature. After the reaction in the resulting solution was completed, water was added thereto. Then, the solution was subjected to extraction using hexane (150ml×3). The obtained yellow oily substance was subjected to column chromatography (silica gel, methylene chloride), so that a component of Rf 0.7 was isolated. In this way, a compound 28 (824mg, 2.01mmol, 93%) was obtained in the form of a colorless oily substance.

Results of analysis with regard to the compound 28 are described below.
$^1$H-NMR (400MHz, $CDCl_3$): δ0.88 (t, J=7.16Hz, 6H), 1.25-1.35 (m, 12H), 1.50-1.57 (m, 4H), 2.59 (s, 4H), 3.37 (s, 4H), 3.40 (t, J=6.60Hz, 4H), 6.73 (s, 2H);
$^{13}$C-NMR (100MHz, $CDCl_3$): δ14.0, 22.6, 25.8, 29.5, 31.6, 33.2.4, 55.3, 71.4, 73.6, 114.6, 147.0;
MS (DI) m/z =352($M^+$) (calcd. =352)

(Compound 29)

N-bromosuccinimide (1.11g, 6.23mmol) was added to a chloroform (30ml)-DMF (10ml) solution of the compound 28 (1.04g, 2.84mmol) and then stirred for 5 hours at a room temperature. After a reaction in the resulting solution was completed, a saturated sodium hydrogen carbonate aqueous solution (30ml) was added thereto and then stirred for 30 minutes. Then, the solution was subjected to extraction using chloroform (50mlx3), thereafter washed with saturated saline, and dried with anhydrous magnesium sulfate. After removing the solvent, the obtained faintly-yellow oily substance was subjected to column chromatography (silica gel, hexane: methylene chloride = 1:1), so that a component of Rf 0.9 was isolated. In this way, a compound 29 (1.36g, 2.67mmol, 94%) was obtained in the form of a faintly-yellow oily substance.

Results of analysis with regard to the compound 29 are described below.
$^1$H-NMR (400MHz, $CDCl_3$): δ0.89 (t, J=6.80Hz, 6H), 1.25-1.31 (m, 12H), 1.54 (quint, J=7.60Hz, 4H), 2.59 (s, 4H), 3.35 (s, 4H), 3.40 (t, J=6.80Hz, 4H);
$^{13}$C-NMR (100MHz, $CDCl_3$): 14.0, 22.6, 25.8, 29.4, 31.6, 34.4, 54.5, 71.4, 73.5, 101.4, 147.3;
MS (DI) m/z =510($M^+$) (calcd. =510)

(Compound 30)

Under a nitrogen atmosphere, argon gas was injected for 30 minutes to an anhydrous toluene (60ml) solution of the compound 29 (1.0g, 1.97mmol) and a compound 33 (1.83g, 4.33mmol), so that the solution was deaerated. In a

reaction container shielded from light, $Pd(PPh_3)_4$ (228mg, 0.19mmol) was added to the solution and then refluxed for 20 hours. After the reaction was completed, the resulting solution was cooled down to a room temperature and filtered with celite. Then, a solvent was removed from the obtained solution. Then, the obtained orange-colored solid substance was subjected to column chromatography (silica gel, hexane: methylene chloride = 2:1), so that a component of Rf 0.5 was isolated. The component was recrystallized with a mixed solvent of ethanol-hexane. As a result, a compound 30 (661mg, 1.07mmol, 55%) was obtained in the form of a faintly-yellow needle-like crystal.

Results of analysis with regard to the compound 30 are described below.
Melting point: 74-75°C;
$^1$H-NMR (400MHz, $CDCl_3$): δ0.87 (t, J=6.80Hz, 6H), 1.26-1.29 (m, 12H), 1.52-1.56 (m, 4H), 2.71 (s, 4H), 3.41-3.44 (m, 8H), 7.24-7.27 (m, 4H), 7.92 (dd, J=1.48, 5.36Hz, 2H);
$^{13}$C-NMR (100MHz, $CDCl_3$): δ14.0, 22.6, 25.8, 29.4, 31.6, 34.4, 55.2, 71.4, 73.8, 125.1, 127.5, 129.6, 130.0, 141.8, 144.0; MS (DI) m/z =612($M^+$) (calcd. =612);
Anal. Calcd for $C_{29}H_{40}O_2SSe_2$: C, 57.04; H, 6.60%;
Found: C, 57.04; H, 6.63%

(Compound 31)

N-bromosuccinimide (116mg, 0.65mmol) was added to a chloroform (40ml) solution of the compound 30 (200mg, 0.32mmol) in an ice bath and then stirred for 5 hours at a room temperature. After a reaction in the resulting solution was completed, a saturated sodium hydrogen carbonate aqueous solution (10ml) was added thereto and then stirred for 30 minutes. Then, the solution was subjected to extraction using chloroform (50ml×3), thereafter washed with saturated saline (100ml), and dried with anhydrous magnesium sulfate. After removing the solvent, the obtained yellow oily substance was subjected to column chromatography (silica gel, hexane: methylene chloride = 1:1), so that a component of Rf 0.5 was isolated. The component was recrystallized with a mixed solvent of ethanol-hexane. As a result, a compound 31 (230mg, 0.29mmol, 92%) was obtained in the form of a yellow needle-like crystal.

Results of analysis with regard to the compound 31 are described below.
Melting point: 64-65°C;
$^1$H-NMR (400MHz, $CDCl_3$): δ0.88 (t, J=6.84Hz, 6H), 1.26-1.34 (m, 12H), 1.51-1.56 (m, 4H), 2.63 (s, 4H), 3.40-3.43 (m, 8H), 6.94 (d, J=4.12, 2H), 7.16 (d, J=4.12Hz, 2H);
$^{13}$C-NMR (100MHz, $CDCl_3$): δ14.0, 22.6, 25.8, 29.4, 31.6, 34.1, 55.4, 71.5, 73.7, 114.6, 124.8, 127.0, 133.4, 143.4, 144.4; MS (DI) m/z =768($M^+$) (calcd. =768);
Anal. Calcd for $C_{29}H_{38}Br_2O_2SSe_2$: C, 45.33; H, 4.98%;
Found: C, 45.25; H, 5.05%

(Compound 32)

Under a nitrogen atmosphere, sodium hydride (60%, in oil, 81mg, 3.38mmol) and malononitrile (112mg, 1.70mmol) were added to a THF (20ml) solution and then stirred for 30 minutes at a room temperature. To the resulting solution, in a reaction container shielded from light, $Pd(PPh_3)_4$ (19mg, 17.0μmol) and dppf (19mg, 35.2μmol) were added, and then a THF (30ml) solution of the compound 31 (130mg, 0.17mmol) was added thereto in drops over 20 minutes and thereafter refluxed for 20 hours. After a reaction in the resulting solution was completed, a 2N hydrochloric acid aqueous solution was added thereto and then stirred for 30 minutes. Then, the solution was subjected to extraction using chloroform (50mlx3), thereafter washed with saturated saline (100ml), and dried with anhydrous magnesium sulfate. After removing the solvent, the obtained yellow oily substance was subjected to column chromatography (10% low-active silica gel, chloroform), so that a component of Rf 0.5 was isolated. The component was recrystallized with acetone-acetonitrile. As a result, a compound 32 (79mg, 99.4μmol, 63%) was obtained in the form of a green solid substance.

Results of analysis with regard to the component 32 are described below.
Melting point: degraded at 300°C;
$^1$H-NMR (400MHz, $CDCl_3$): δ0.87 (t, J=6.40Hz, 6H), 1.25-1.34 (m, 12H), 1.53-1.57 (m, 4H), 2.75-2.92 (m, 4H), 3.43-3.47 (m, 8H), 7.22-7.26 (m, 2H), 7.36-7.39 (m, 2H), 7.41-7.43 (m, 2H), 7.60-7.63 (m, 2H);
MS (DI) m/z =642($M^+$) (calcd. =642); IR (KBr) 2206.57$cm^{-1}$; Anal. Calcd for $C_{35}H_{38}N_4O_2SSe_2$: C, 57.06; H, 5.20; N, 7.61%; Found: C, 57.06; H, 5.38; N, 7.69%

(Compound 33)

Under a nitrogen atmosphere, selenophene (2.00g, 15.3mmol) was dissolved in an anhydrous THF (15ml) and then cooled down to -78°C. The resultant was added in drops to a n-butyllithium · hexane solution (1.57M, 9.95ml,

15.7mmol) and then increased in temperature to a room temperature. After stirring for 15 minutes, the resulting solution was cooled down to -20°C, and tributyltin chloride (4.84ml, 17.9mmol) was added thereto in drops. After the resulting solution was stirred for 24 hours at a room temperature, water (20ml) was added thereto. The reaction solution thus obtained was subjected to extraction using hexane (30mlx3), and then washed with water (30ml×1). An organic layer obtained by the extraction was dried with anhydrous sodium sulfate, and then the solvent in the organic layer was distilled away under a reduced pressure. The resultant was purified by column chromatography (alumina, hexane, Rf=0.9). As a result, a faintly-yellow liquid was obtained (6.46g, 100%).

A result of analysis with regard to a compound 33 is described below.

$^1$H-NMR (400MHz, $CDCl_3$): δ0.90 (t, J=7.3Hz, 9H), 1.10 (t, J=8.1Hz, 6H), 1.34 (sexi, J=7.3Hz, 6H), 1.57 (quint, J=8.1Hz, 6H), 7.48-7.51 (m, 2H), 8.35 (dd, J=1.0Hz, 4.9Hz, 1H)

(Compound 34)

Under a nitrogen atmosphere, sodium hydride (60%, in oil, 208mg, 8.68mmol) was added to a DMF (30ml) solution of an alcohol (400mg, 2.17mmol) and then stirred for 30 minutes at a room temperature. To the resulting solution on ice, n-octyl bromide (1.08g, 0.96ml, 6.51mmol) was added in drops over 1 hour and then stirred for 24 hours at a room temperature. After the reaction in the resulting solution was completed, water was added thereto. Then, the solution was subjected to extraction using hexane (150ml×3). The obtained yellow oily substance was subjected to column chromatography (silica gel, methylene chloride), so that a component of Rf 0.7 was isolated. In this way, a compound 34 (564mg, 1.38mmol, 99%) was obtained in the form of a colorless oily substance.

Results of analysis with regard to the compound 34 are described below.

$^1$H-NMR (400MHz, $CDCl_3$): δ0.88 (t, J=7.08Hz, 6H), 1.24-1.27 (m, 20H), 1.53 (m, 4H), 2.59 (s, 4H), 3.36 (s, 4H), 3.39 (t, J=6.84Hz, 4H), 6.73 (s, 2H);

$^{13}$C-NMR (100MHz, $CDCl_3$): δ14.0, 22.6, 26.1, 29.3, 29.4, 29.5, 31.8, 33.2, 55.3, 71.4, 73.6, 114.6, 147.0;

MS (DI) m/z =408($M^+$) (calcd. =408)

(Compound 35)

N-bromosuccinimide (780mg, 4.38mmol) was added to a DMF (40ml) solution of the compound 34 (895mg, 2.19mmol) and then stirred for 5 hours at a room temperature. After a reaction in the resulting solution was completed, a saturated sodium hydrogen carbonate aqueous solution (30ml) was added thereto and then stirred for 30 minutes. The resulting solution was subjected to extraction using chloroform (50ml×3), thereafter washed with saturated saline (100ml), and dried with anhydrous magnesium sulfate. After removing the solvent, the obtained faintly-yellow oily substance was subjected to column chromatography (silica gel, hexane: methylene chloride = 3:1), so that a component of Rf 0.3 was isolated. In this way, a compound 35 (1.00g, 1.77mmol, 89%) was obtained in the form of a faintly-yellow oily substance.

A result of analysis with regard to the compound 35 is described below.

$^1$H-NMR (400MHz, $CDCl_3$): δ0.88 (t, J=6.40Hz, 6H), 1.25-1.30 (m, 20H), 1.52-1.57 (m, 4H), 2.54 (s, 4H), 3.34 (s, 4H), 3.40 (t, J=6.80Hz, 4H)

(Compound 36)

Under a nitrogen atmosphere, argon gas was injected for 30 minutes to an anhydrous toluene (50ml) solution of the compound 35 (1.0g, 1.77mmol) and the compound 33 (1.65g, 3.89mmol), so that the solution was deaerated. In a reaction container shielded from light, $Pd(PPh_3)_4$ (204mg, 0.18mmol) was added to the solution and then refluxed for 20 hours. After the reaction was completed, the resulting solution was cooled down to a room temperature and filtered with celite. Then, the solvent was removed from the obtained solution. The obtained orange-colored solid substance was subjected to column chromatography (silica gel, hexane: methylene chloride = 2:1), so that a component of Rf 0.3 was isolated. The component was recrystallized with a mixed solvent of ethanol-hexane. As a result, a compound 36 (520mg, 0.78mmol, 60%) was obtained in the form of a yellow needle-like crystal.

Results of analysis with regard to the compound 36 are described below.

Melting point: 69-70°C;

$^1$H-NMR (400MHz, $CDCl_3$): δ0.87 (t, J=6.84Hz, 6H), 1.26-1.35 (m, 20H), 1.52-1.57 (m, 4H), 2.71 (s, 4H), 3.39-3.43 (m, 8H), 7.23-7.27 (m, 4H), 7.92 (dd, J=1.24, 5.16Hz, 2H);

$^{13}$C-NMR (100MHz, $CDCl_3$): δ14.1, 22.6, 26.1, 29.3, 29.4, 29.5, 31.8, 34.3, 55.2, 71.5, 73.8, 125.1, 127.5, 129.6, 130.0, 141.8, 144.0;

MS (DI) m/z =668($M^+$) (calcd. =668);

Anal. Calcd for $C_{33}H_{48}O_2SSe_2$: C, 59.45; H, 7.26%;

Found: C, 59.33; H, 7.32%

(Compound 37)

N-bromosuccinimide (85mg, 4.82mmol) was added to a chloroform (40ml) solution of the compound 36(161mg, 0.24mmol) in an ice bath and then stirred for 5 hours at a room temperature. After the reaction in the resulting solution was completed, a saturated sodium hydrogen carbonate aqueous solution (10ml) was added thereto and then stirred for 30 minutes. Then, the solution was subjected to extraction using chloroform (50mlx3), thereafter washed with saturated saline (100ml), and dried with anhydrous magnesium sulfate. After removing the solvent, the obtained yellow oily substance was subjected to column chromatography (silica gel, hexane: methylene chloride = 2:1), so that a component of Rf 0.7 was isolated. The component was recrystallized with a mixed solvent of ethanol-hexane. As a result, a compound 37 (172mg, 0.21mmol, 89%) was obtained in the form of a yellow needle-like crystal.

Results of analysis with regard to the compound 37 are described below.
Melting point: 61-62°C;
$^1$H-NMR (400MHz, $CDCl_3$): $\delta$0.87 (t, J=7.08Hz, 6H), 1.26-1.34 (m, 20H), 1.51-1.55 (m, 4H), 2.63 (s, 4H), 3.40-3.43 (m, 8H), 6.94 (d, J=4.16, 2H), 7.16 (d, J=4.16Hz, 2H);
$^{13}$C-NMR (100MHz, $CDCl_3$): $\delta$14.1, 22.6, 26.2, 29.3, 29.4, 29.5, 31.8, 34.1, 55.4, 71.5, 73.7, 114.6, 124.8, 127.0, 133.4, 143.4, 144.4;
MS (MALDI-TOF) m/z =824 ($M^{+2}$) (calcd. =824); $C_{33}H_{46}Br_2O_2SSe_2$: C, 48.07; H, 5.62%;
Found: C, 47.98; H, 5.66%

(Compound 38)

Under a nitrogen atmosphere, sodium hydride (60%, in oil, 58mg, 1.96mmol) and malononitrile (65mg, 0.98mmol) were added to a THF (20ml) solution and then stirred for 30 minutes at a room temperature. To the resulting solution, in a reaction container shielded from light, $Pd(PPh_3)_4$ (1mg, 0.98$\mu$mol) and dppf (1mg, 1.80$\mu$mol) were added, and then a THF (30ml) solution of the compound 37 (81mg, 98.2$\mu$mol) was added thereto in drops over 20 minutes and thereafter refluxed for 20 hours. After the reaction in the resulting solution was completed, a 2N hydrochloric acid aqueous solution was added thereto and then stirred for 30 minutes. Then, the solution was subjected to extraction using chloroform (50ml$\times$3), thereafter washed with saturated saline (100ml), and dried with anhydrous magnesium sulfate. After removing the solvent, the obtained yellow oily substance was subjected to column chromatography (10% low-active silica gel, chloroform), so that a component of Rf 0.5 was isolated. The component was recrystallized with acetone-acetonitrile. As a result, a compound 38 (48mg, 0.06mmol, 61%) was obtained in the form of a green solid substance.

Results of analysis with regard to the compound 38 are described below.
Melting point: degraded at 300°C;
$^1$H-NMR (400MHz, $CDCl_3$): $\delta$0.87 (t, J=5.40Hz, 6H), 1.25-1.30 (m, 20H), 1.53-1.57 (m, 4H), 2.74-2.92 (m, 4H), 3.43-3.46 (m, 8H), 7.22-7.26 (m, 2H), 7.36-7.39 (m, 2H), 7.46-7.50 (m, 2H), 7.60-7.63 (m, 2H);
MS (DI) m/z =794($M^+$) (calcd. =794); IR (KBr) 2206.57$cm^{-1}$

An organic semiconductor material according to the present invention comprises a compound represented by the general formula (1):

Chem. 51

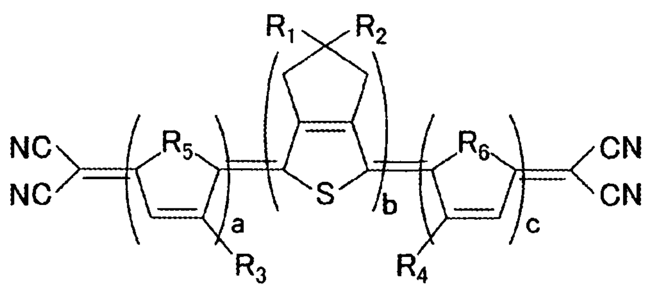

··· (1)

where each of $R_1$ through $R_4$ independently is an atom or functional group selected from the group consisting of (i) a hydrogen atom, (ii) a halogen atom, (iii) an alkyl group in which one or more carbon atoms may be substituted by an oxygen atom and/or a sulfur atom and in which one or more hydrogen atoms may be substituted by a halogen atom,

(iv) an alkyloxy group, (v) an alkylthio group, (vi) an aryl group, and (vii) an alkyl group in which one or more hydrogen atoms are substituted by an aryl group; each of $R_5$ and $R_6$ independently is a sulfur atom or a selenium atom; and a, b, and c are integers.

With this arrangement, in which the compound contains a cyclopentane ring-fused thiophene, it is possible to prevent a decrease in solubility. Further, since a selenophene or a thiophen other than the cyclopentane ring-fused thiophene is introduced to an end of the cyclopentane ring-fused thiophene, it is possible to prevent a decrease in conductivity.

Further, with the arrangement, in which the compound has a polarity due to a cyano group that attracts an electron, it becomes possible to perform an n-type transistor operation. This allows a stable operation of the organic semiconductor material even in an atmosphere.

In the arrangement, the compound includes (i) a cyclopentane ring-fused thiophene, which is soluble, (ii) a selenophene or a thiophene not derived from a condensed cyclopentane, which increases a molecule overlap, and (iii) a cyano group, which has a polarity. Therefore, it is possible to provide an organic semiconductor material that can be subjected to a welding process and allows a stable n-type transistor operation even in an atmosphere.

The embodiments and concrete examples of implementation discussed in the foregoing detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

Industrial Applicability

With the present invention, it is possible to improve an organic semiconductor material in solubility, conductivity, and electron mobility. This makes it possible to provide an organic semiconductor material that can be subjected to a welding process and allows a stable n-type transistor operation even in the atmosphere. Therefore, the present invention can be widely used in various fields such as a transistor, an organic FET device, a diode, a capacitor, a thin-film photoelectric conversion element, a dye-sensitized solar cell, a thin-film transistor (TFT), a light-emitting device including an organic carrier transporting layer and/or light-emitting layer, and an organic EL device.

## Claims

**1.** An organic semiconductor material comprising a compound represented by the general formula (1):

Chem. 1

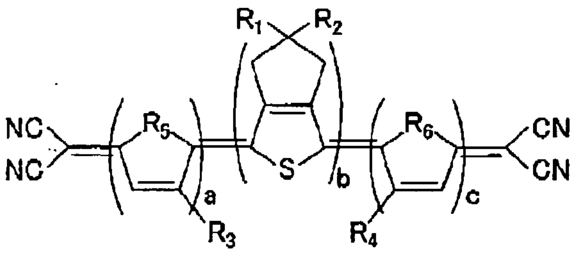

$\cdots$ (1)

where each of $R_1$ through $R_4$ independently is an atom or functional group selected from the group consisting of (i) a hydrogen atom, (ii) a halogen atom, (iii) an alkyl group in which one or more carbon atoms may be substituted by an oxygen atom and/or a sulfur atom and in which one or more hydrogen atoms may be substituted by a halogen atom, (iv) an alkyloxy group, (v) an alkylthio group, (vi) an aryl group, and (vii) an alkyl group in which one or more hydrogen atoms are substituted by an aryl group; each of $R_5$ and $R_6$ independently is a sulfur atom or a selenium atom; and a, b, and c are integers.

**2.** The organic semiconductor material according to claim 1, wherein:
the carbon number of the functional group is 1 or more and 18 or less; and

$$a + b + c \leq 6.$$

**3.** The organic semiconductor material according to claim 1 or 2, wherein:

$R_1$ and $R_2$ are an identical atom or functional group;
$R_3$ and $R_4$ are an identical atom or functional group;
$R_5$ and $R_6$ are an identical atom; and
a = c.

**4.** The organic semiconductor material according to any one of claims 1 through 3, wherein:

in the general formula (1), $R_5$ and $R_6$ are a sulfur atom.

**5.** The organic semiconductor material according to claim 4, wherein, in the general formula (1):

$R_1$ and $R_2$ are a butoxymethyl group;
$R_3$ and $R_4$ are a hydrogen atom; and
$R_5$ and $R_6$ are a sulfur atom.

**6.** The organic semiconductor material according to claim 4, wherein, in the general formula (1):

$R_1$ and $R_2$ are a butoxymethyl group;
$R_3$ and $R_4$ are a methyl group; and
$R_5$ and $R_6$ are a sulfur atom.

**7.** The organic semiconductor material according to any one of claims 1 through 6, being an n-type transistor material.

**8.** An organic semiconductor material being a bipolar transistor material that includes an organic semiconductor material as set forth in claim 7 and a p-type transistor material.

**9.** An organic semiconductor device comprising an organic semiconductor material as set forth in any one of claims 1 through 8.

**10.** The organic semiconductor device according to claim 9, being a light-emitting device that includes an organic carrier transporting layer and/or a light-emitting layer.

**11.** The organic semiconductor device according to claim 9 or 10, being a thin-film transistor that includes an organic semiconductor layer.

**12.** A method for producing an organic semiconductor device, comprising dissolving an organic semiconductor material in an organic solvent and providing the organic solvent containing the organic semiconductor material on a substrate by a method of spin coating or inkjet, the organic semiconductor material being as set forth in any one of claims 1 through 8.

**13.** A mehod for producing an organic semiconductor device
according to claim 12 comprising the step of annealing the organic semiconductor material .

**14.** A method for producing an organic semiconductor material as set forth in any one of claims 1 through 8, comprising:

producing a compound represented by the general formula (4):

Chem. 4

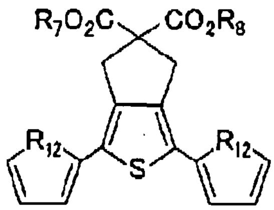

... (4)

where each of $R_7$ and $R_8$ independently is an alkyl group; and $R_{12}$ is a sulfur atom or selenium atom. the compound represented by the general formula (4), being produced by reacting a compound represented by the general formula (2) and a compound represented by the general formula (3):

Chem. 2

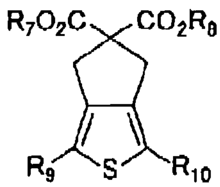

... (2)

where each of $R_7$ and $R_8$ independently is an alkyl group; and each of $R_9$ and $R_{10}$ independently is a halogen atom;

Chem. 3

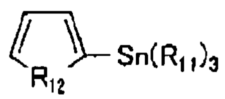

... (3)

where $R_{11}$ is an alkyl group; and $R_{12}$ is a sulfur atom or a selenium atom; and thereafter reducing an ester group of the compound represented by the general formula (4).

**15.** The method according to claim 14, wherein:

in the general formula (2), $R_7$ and $R_8$ are an ethyl group; and $R_9$ and $R_{10}$ are a bromine atom;
in the general formula (3), $R_{11}$ is a butyl group; and $R_{12}$ is a sulfur atom; and
in the general formula (4), $R_7$ and $R_8$ are an ethyl group; and $R_{12}$ is a sulfur atom.

**Patentansprüche**

**1.** Organisches Halbleitermaterial, das eine Verbindung dargestellt durch die allgemeine Formel (1) umfasst:

Chem. 1

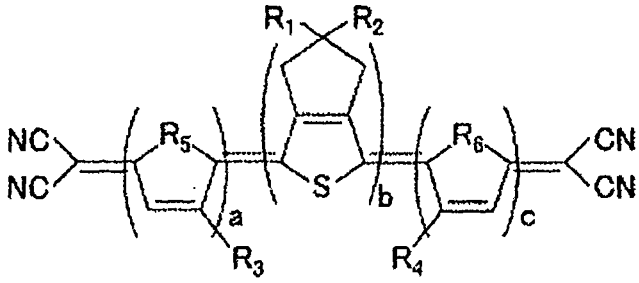

...(1)

worin jedes von $R_1$ bis $R_4$ unabhängig ein Atom oder eine funktionelle Gruppe ist, das/die ausgewählt ist aus der Gruppe bestehend aus (i) einem Wasserstoffatom, (ii) einem Halogenatom, (iii) einer Alkylgruppe, in welcher ein oder mehrere Kohlenstoffatome durch ein Sauerstoff und/oder ein Schwefelatom substituiert sein können, und in welcher ein oder mehrere Wasserstoffatome durch ein Halogenatom substituiert sein können, (iv) eine Alkyloxygruppe, (v) eine Alkylthiogruppe, (vi) eine Arylgruppe und (vii) eine Alkylgruppe, in welcher ein oder mehrere Wasserstoffatome durch eine Arylgruppe substituiert sind; jedes von $R_5$ und $R_6$ unabhängig ein Schwefelatom oder ein Selenatom ist; und a, b und c ganze Zahlen sind.

**2.** Organisches Halbleitermaterial nach Anspruch 1, wobei:

die Kohlenstoffanzahl der funktionellen Gruppe 1 oder mehr und 18 oder weniger ist; und

$$a + b + c \leq 6.$$

**3.** Organisches Halbleitermaterial nach Anspruch 1 oder 2, wobei:

$R_1$ und $R_2$ ein identisches Atom oder eine identische funktionelle Gruppe sind;
$R_3$ und $R_4$ ein identisches Atom oder eine identische funktionelle Gruppe sind;
$R_5$ und $R_6$ ein identisches Atom sind; und
a = c.

**4.** Organisches Halbleitermaterial nach einem der Ansprüche 1 bis 3, wobei:

in der allgemeinen Formel (1) $R_5$ und $R_6$ ein Schwefelatom sind.

**5.** Organisches Halbleitermaterial nach Anspruch 4, wobei in der allgemeinen Formel (1):

$R_1$ und $R_2$ eine Butoxymethylgruppe sind;
$R_3$ und $R_4$ ein Wasserstoffatom sind; und
$R_5$ und $R_6$ ein Schwefelatom sind.

**6.** Organisches Halbleitermaterial nach Anspruch 4, wobei in der allgemeinen Formel (1):

$R_1$ und $R_2$ eine Butoxymethylgruppe sind;
$R_3$ und $R_4$ eine Methylgruppe sind; und
$R_5$ und $R_6$ ein Schwefelatom sind.

**7.** Organisches Halbleitermaterial nach einem der Ansprüche 1 bis 6, welches ein Transistormaterial vom n-Typ ist.

**8.** Organisches Halbleitermaterial, das ein bipolares Transistormaterial ist, das ein organisches Halbleitermaterial nach Anspruch 7 und ein Transistormaterial vom p-Typ enthält.

**9.** Organische Halbleitervorrichtung, die ein organisches Halbleitermaterial nach einem der Ansprüche 1 bis 8 umfasst.

**10.** Organische Halbleitervorrichtung nach Anspruch 9, die eine Licht-emittierende Vorrichtung ist, die eine organische Trägertransportschicht und/oder eine Licht-emittierende Schicht enthält.

**11.** Organische Halbleitervorrichtung nach Anspruch 9 oder 10, die ein Dünnschichttransistor ist, der eine organische Halbleiterschicht enthält.

**12.** Verfahren für die Herstellung einer organischen Halbleitervorrichtung, das das Auflösen eines organischen Halbleitermaterials in einem organischen Lösungsmittel und das Bereitstellen des organischen Lösungsmittels, das das organische Halbleitermaterial enthält, auf einem Substrat durch ein Schleuderbeschichtungsverfahren oder ein Tintenstrahldruckverfahren umfasst, wobei das organische Halbleitermaterial wie in einem der Ansprüche 1 bis 8 festgelegt ist.

**13.** Verfahren für die Herstellung einer organischen Halbleitervorrichtung nach Anspruch 12, das den Schritt des Temperns des organischen Halbleitermaterials umfasst.

**14.** Verfahren für die Herstellung eines organischen Halbleitermaterials nach einem der Ansprüche 1 bis 8, welches umfasst:

Herstellen einer Verbindung dargestellt durch die allgemeine Formel (4):

Chem. 4

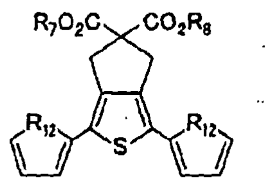

... (4)

worin jedes von $R_7$ und $R_8$ unabhängig eine Alkylgruppe ist; und $R_{12}$ ein Schwefelatom oder ein Selenatom ist; die Verbindung dargestellt durch die allgemeine Formel (4) wird durch
Reagieren einer Verbindung dargestellt durch die allgemeine Formel (2) und einer Verbindung dargestellt durch die allgemeine Formel (3) hergestellt:

Chem. 2

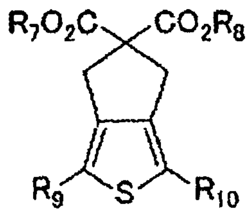

... (2)

worin jedes von $R_7$ und $R_8$ unabhängig eine Alkylgruppe ist; und jedes von $R_9$ und $R_{10}$ unabhängig ein Halogenatom ist;

Chem. 3

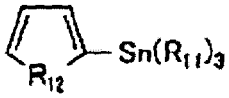

...(3)

worin $R_{11}$ eine Alkylgruppe ist und $R_{12}$ ein Schwefelatom oder ein Selenatom ist; und danach Reduzieren einer Estergruppe der Verbindung dargestellt durch die allgemeine Formel (4).

**15.** Verfahren nach Anspruch 14, wobei:

in der allgemeinen Formel (2) $R_7$ und $R_8$ eine Ethylgruppe sind und $R_9$ und $R_{10}$ ein Bromatom sind; in der allgemeinen Formel (3) $R_{11}$ eine Butylgruppe ist und $R_{12}$ ein Schwefelatom ist; und in der allgemeinen Formel (4) $R_7$, und $R_8$ eine Ethylgruppe sind und $R_{12}$ ein Schwefelatom ist.

## Revendications

**1.** Un matériau semiconducteur organique comprenant un composé répondant à la structure générale (1) :

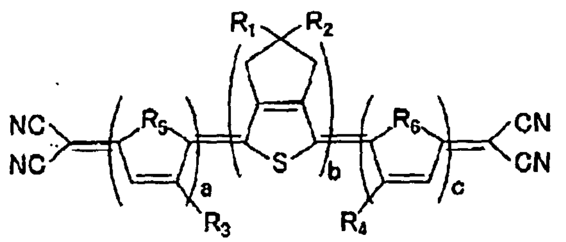

**(1)**

où chacun des radicaux $R_1$ à $R_4$ est indépendamment un atome ou un groupement fonctionnel sélectionné parmi le group consistant de (i) un atome d'hydrogène, (ii) un atome d'halogène, (iii) un groupe alkyle dans lequel un ou plusieurs atomes de carbone peuvent être substitués par un atome d'oxygène et/ou un atome de soufre et dans lequel un ou plusieurs atomes d'hydrogène peuvent être substitués par un atome d'halogène, (iv) un groupe alkyloxy, (v) un groupe alkylthio, (vi) un groupe aryle, et (vii) un groupe alkyle dans lequel un ou plusieurs atomes d'hydrogène sont substitués par un groupe aryle ; chacun des radicaux $R_5$ et $R_6$ est indépendamment un atome de soufre ou un atome de sélénium ; et a, b et c sont des nombres entiers.

**2.** Le matériau semiconducteur organique selon la revendication 1, dans lequel :

le nombre d'atomes de carbone du groupement fonctionnel est compris entre 1 et 18, bornes incluses ; et $a+b+c \leq 6$.

**3.** Le matériau semiconducteur organique selon la revendication 1 ou 2, dans lequel :

$R_1$ et $R_2$ sont un atome ou groupement fonctionnel identique ;
$R_3$ et $R_4$ sont un atome ou groupement fonctionnel identique;
$R_5$ et $R_6$ sont un atome identique; et

**4.** Le matériau semiconducteur organique selon l'une quelconque des revendications 1 à 3, dans lequel :

dans la formule générale (1), $R_5$ et $R_6$ sont un atome de soufre.

**5.** Le matériau semiconducteur organique selon la revendication 4, dans lequel, dans la formule générale (1) :

$R_1$ et $R_2$ sont un groupe butoxyméthyle ;

$R_3$ et $R_4$ sont un atome d'hydrogène ; et
$R_5$ et $R_6$ sont un atome de soufre.

6. Le matériau semiconducteur organique selon la revendication 4, dans lequel, dans la formule générale (1) :

$R_1$ et $R_2$ sont un groupe butoxyméthyle ;
$R_3$ et $R_4$ sont un groupe méthyle ; et
$R_5$ et $R_6$ sont un atome de soufre.

7. Le matériau semiconducteur organique selon l'une quelconque des revendications 1 à 6, lequel matériau est un matériau transistor de type N.

8. Un matériau semiconducteur organique étant un matériau transistor bipolaire qui comprend un matériau semiconducteur organique selon l'une quelconque des revendications 1 à 7 et un matériau transistor de type P.

9. Un dispositif semiconducteur organique comprenant un matériau semiconducteur organique selon l'une quelconque des revendications 1 à 8.

10. Le dispositif semiconducteur organique selon la revendication 9, lequel dispositif est un dispositif électroluminescent qui comprend une couche de transport de porteur organique et/ou une couche électroluminescente.

11. Le dispositif semiconducteur organique selon la revendication 9 ou 10, lequel dispositif est un transistor à couches minces qui comprend une couche de semiconducteur organique.

12. Une méthode pour produire un dispositif semiconducteur organique, comprenant la dissolution d'un matériau semiconducteur organique dans un solvant organique et la disposition du solvant organique contenant le matériau semiconducteur organique sur un substrat par un procédé de dépôt à la tournette ou de jet d'encre, le matériau semiconducteur organique étant tel que défini dans l'une quelconque des revendications 1 à 8.

13. Une méthode pour produire un dispositif semiconducteur organique selon la revendication 12, comprenant l'étape de recuit du matériau semiconducteur organique.

14. Une méthode pour produire un matériau semiconducteur organique tel que défini dans l'une quelconque des revendications 1 à 8, comprenant :

la préparation d'un composé répondant à la formule générale (4) :

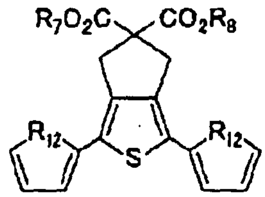

(4)

où chacun des radicaux $R_7$ et $R_8$ est indépendamment un groupe alkyle, et $R_{12}$ est un atome de soufre ou de sélénium,
le composé répondant à la formule générale (4) étant préparé en faisant réagir un composé répondant à la formule générale (2) et un composé répondant à la formule générale (3) :

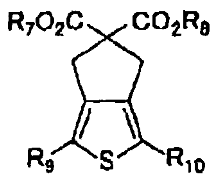

(2)

où chacun des radicaux $R_7$ et $R_8$ est indépendamment un groupe alkyle, et chacun des radicaux $R_9$ et $R_{10}$ est indépendamment un atome d'halogène,

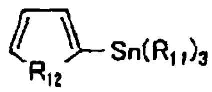

(3)

où $R_{11}$ est un groupe alkyle, et $R_{12}$ est un atome de soufre ou de sélénium ; et
la réduction subséquente d'un groupe ester du composé répondant à la formule générale (4).

**15.** La méthode selon la revendication 14, dans laquelle :

dans la formule générale (2), $R_7$ et $R_8$ sont un groupe éthyle; et $R_9$ et $R_{10}$ sont un atome de brome ;
dans la formule générale (3), $R_{11}$ est un groupe butyle; et $R_{12}$ est un atome de soufre ; et
dans la formule générale (4), $R_7$ et $R_8$ sont un groupe éthyle; et $R_{12}$ est un atome de soufre.

Fig. 1

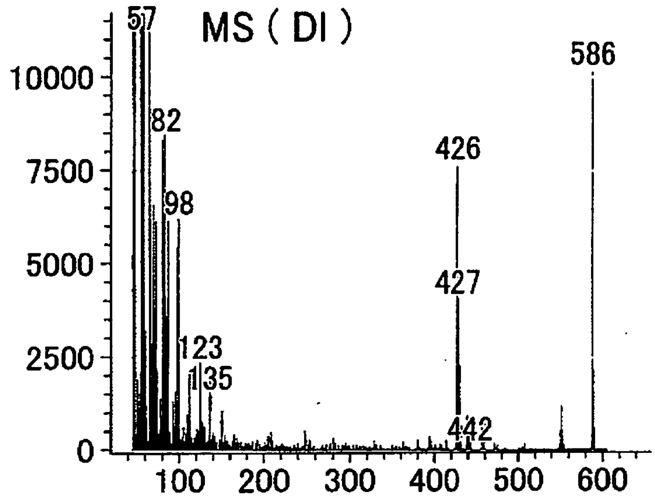
MS ( DI )
57
82
98
123
135
426
427
442
586
10000
7500
5000
2500
0
100
200
300
400
500
600

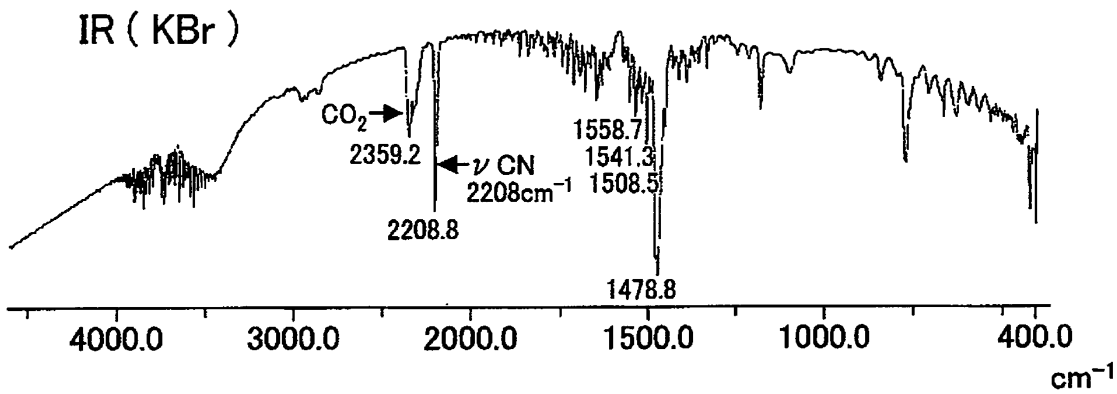
IR ( KBr )
CO2
2359.2
ν CN
2208cm-1
2208.8
1558.7
1541.3
1508.5
1478.8
4000.0
3000.0
2000.0
1500.0
1000.0
400.0
cm-1

Fig. 2

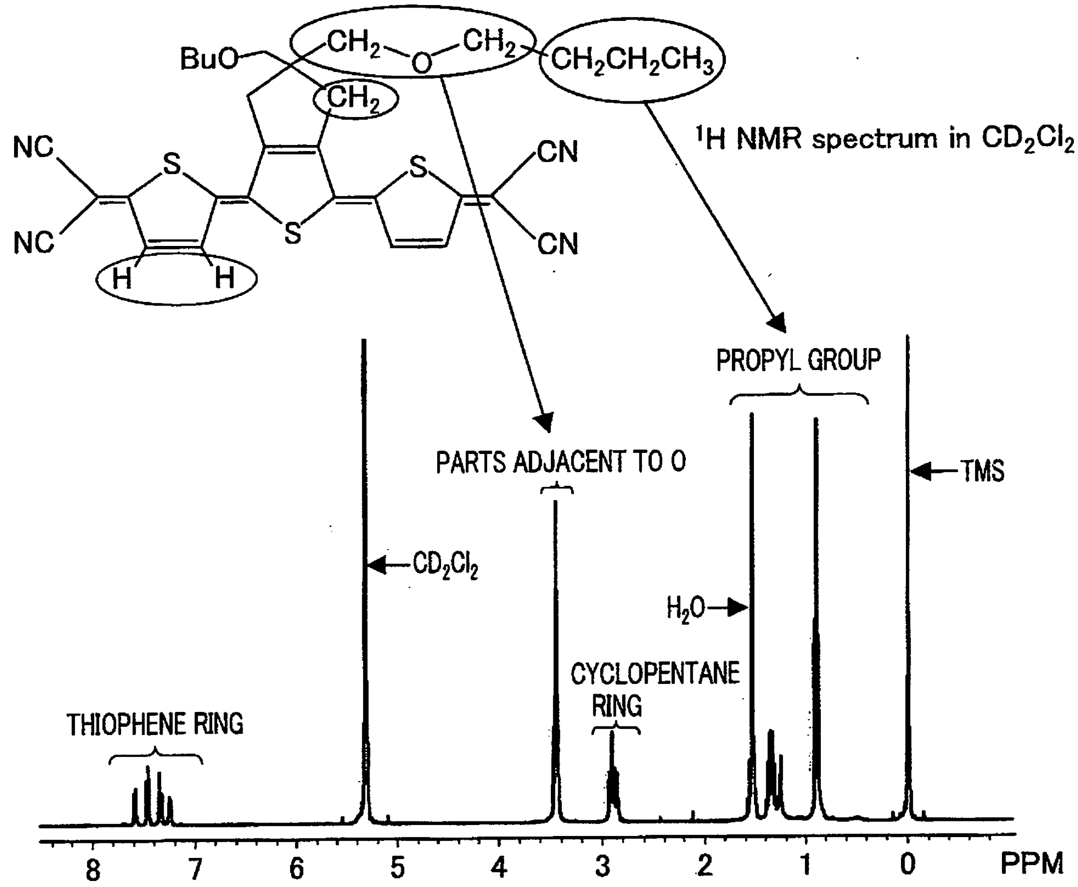

$^1H$ NMR spectrum in $CD_2Cl_2$
PROPYL GROUP
PARTS ADJACENT TO O
TMS
$CD_2Cl_2$
$H_2O$
CYCLOPENTANE RING
THIOPHENE RING
8
7
6
5
4
3
2
1
0
PPM

Fig. 3

trans-trans ISOMER

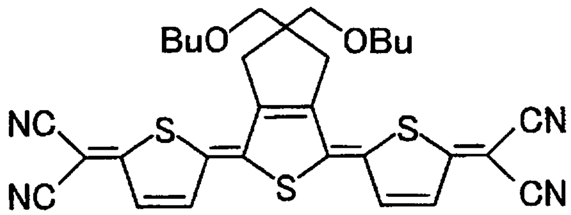

trans-cis ISOMER

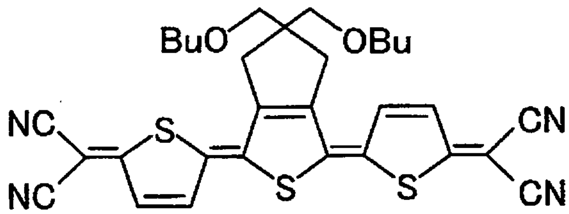

cis-cis ISOMER

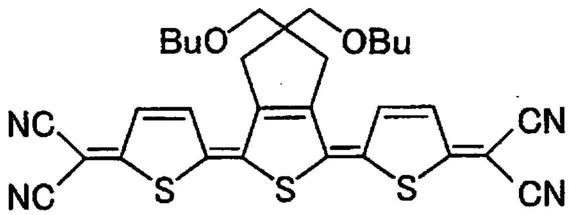

Fig. 4

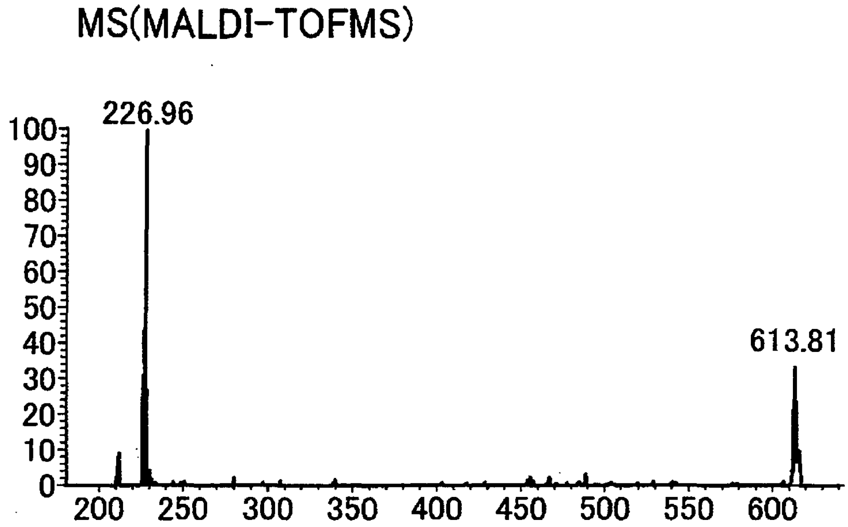
MS(MALDI-TOFMS)
226.96
613.81
100
90
80
70
60
50
40
30
20
10
0
200 250 300 350 400 450 500 550 600

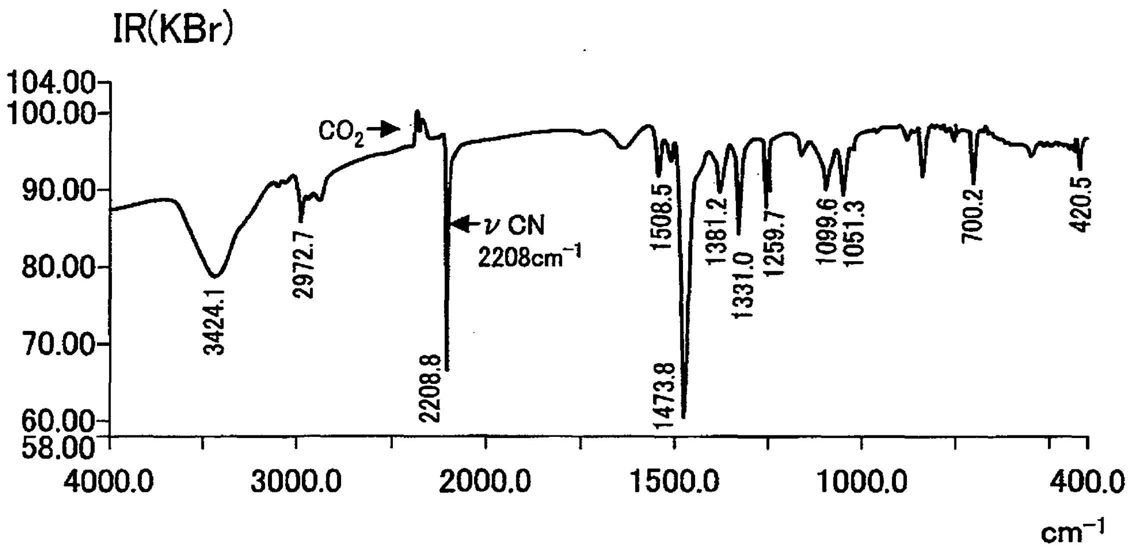
IR(KBr)
104.00
100.00
90.00
80.00
70.00
60.00
58.00
CO2
ν CN
2208cm-1
3424.1
2972.7
2208.8
1508.5
1473.8
1381.2
1331.0
1259.7
1099.6
1051.3
700.2
420.5
4000.0 3000.0 2000.0 1500.0 1000.0 400.0
cm-1

Fig. 5

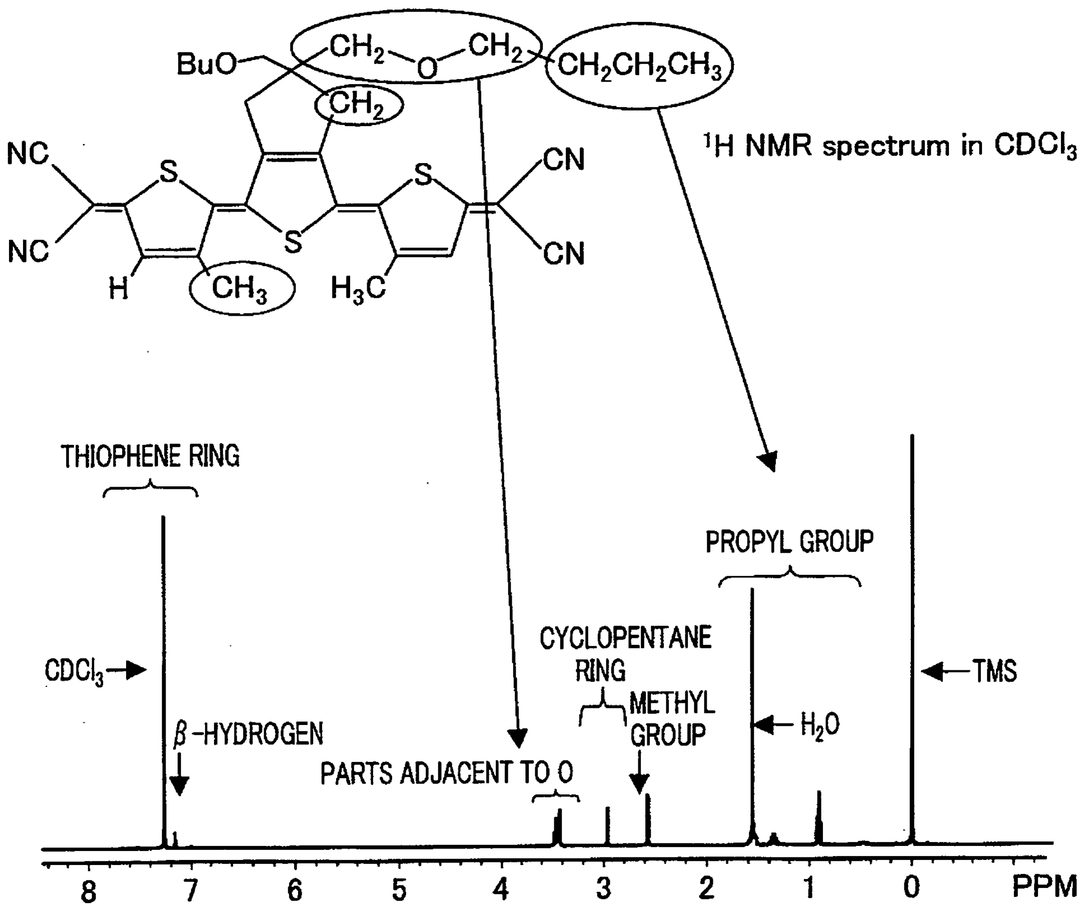

$^1H$ NMR spectrum in $CDCl_3$
THIOPHENE RING
PROPYL GROUP
CYCLOPENTANE RING
METHYL GROUP
$CDCl_3$
TMS
$H_2O$
β-HYDROGEN
PARTS ADJACENT TO O
8 7 6 5 4 3 2 1 0 PPM

Fig. 6

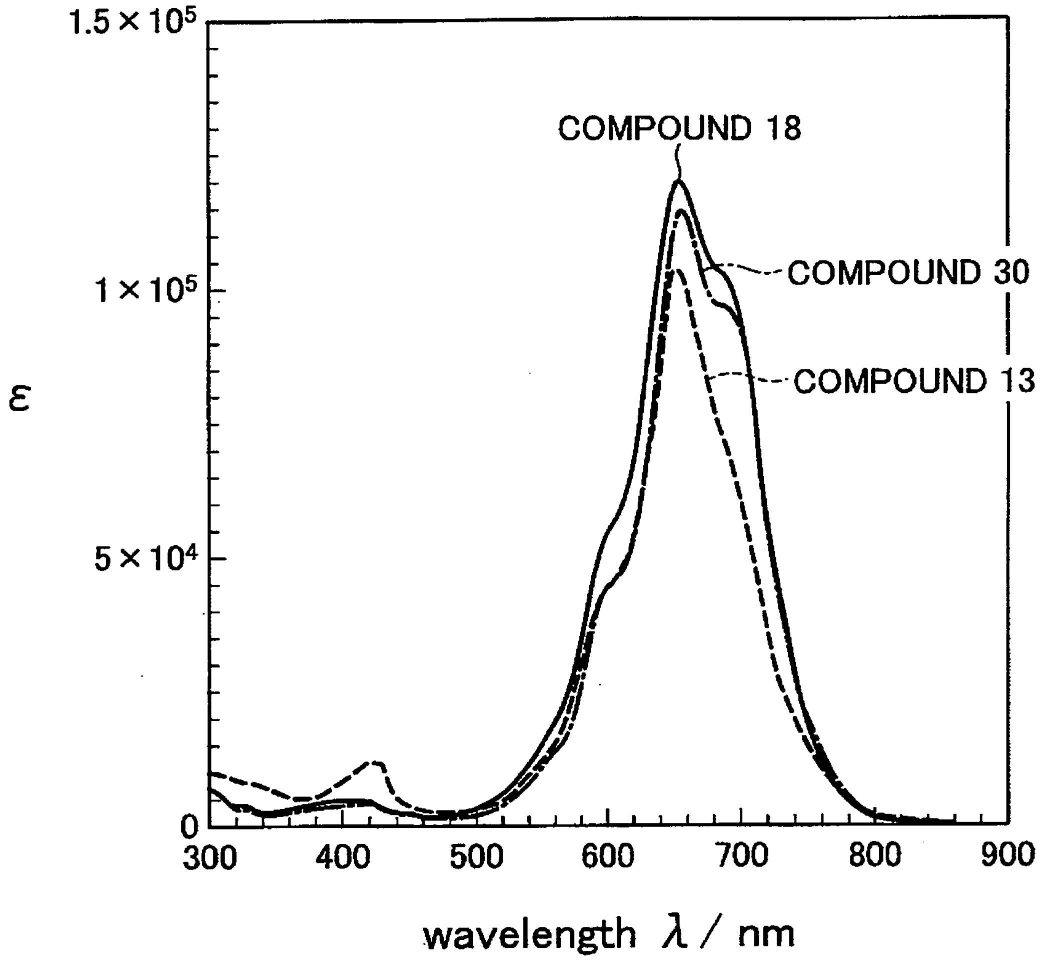
1.5×10⁵
1×10⁵
5×10⁴
0
ε
COMPOUND 18
COMPOUND 30
COMPOUND 13
300
400
500
600
700
800
900
wavelength λ / nm

Fig. 7

COMPOUND 13

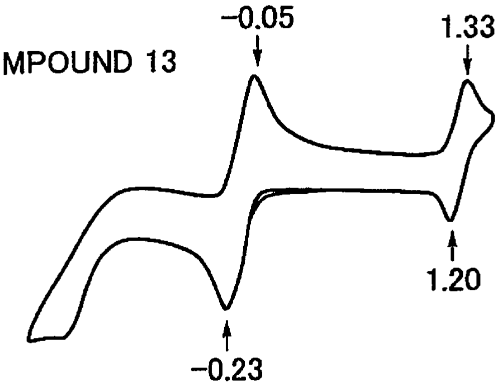


| Compound | $E_{1/2}^{red}/V$ | $E_{1/2}^{ox}/V$ |
|---|---|---|
| COMPOUND 13 | -0.14 | +1.21 |
| COMPOUND 18 | -0.19 | +1.26 |
| COMPOUND 30 | -0.28 | +1.18 |

COMPOUND 18

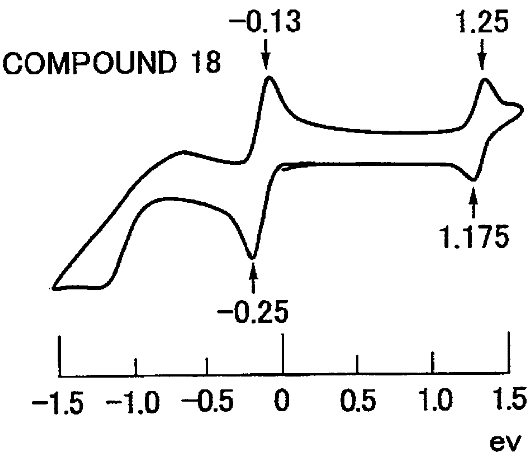


COMPOUND 30

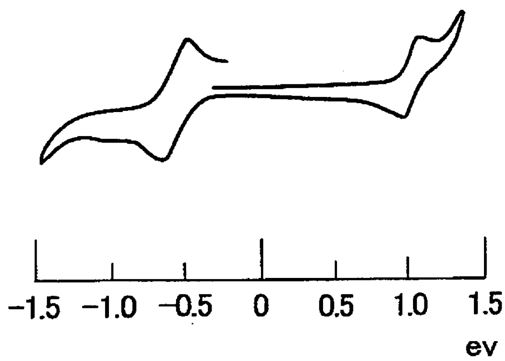

Fig. 8

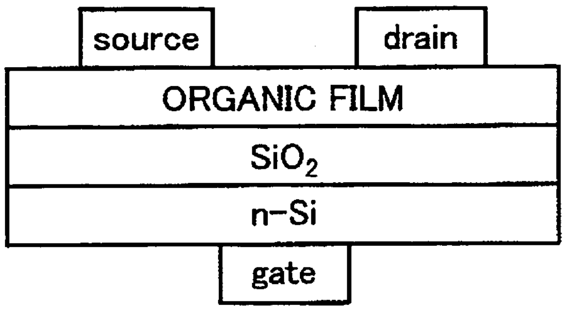


SIDE VIEW

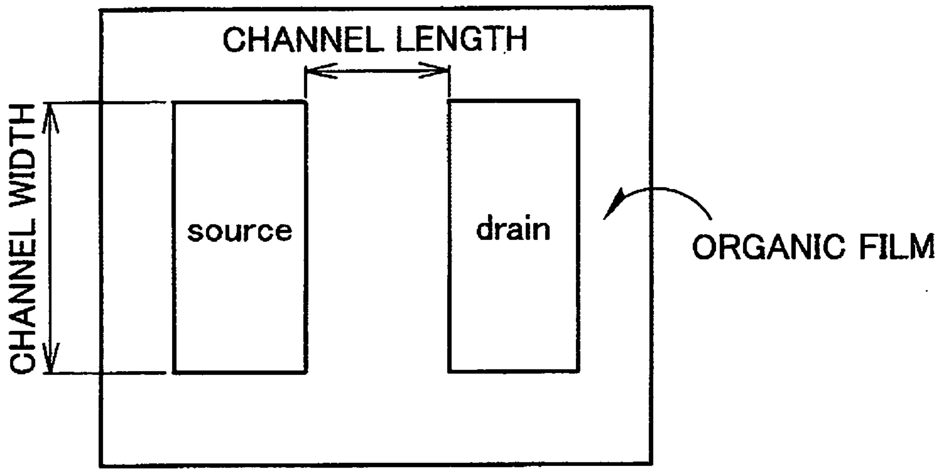


TOP VIEW

Fig. 9

FET CHARACTERISTIC OF COMPOUND 13

SOLUTION CONCENTRATION : 0.4 wt. %
SOLVENT : PURIFIED CHLOROFORM
SPIN-COATING CONDITION : 2000 rpm , 30 sec

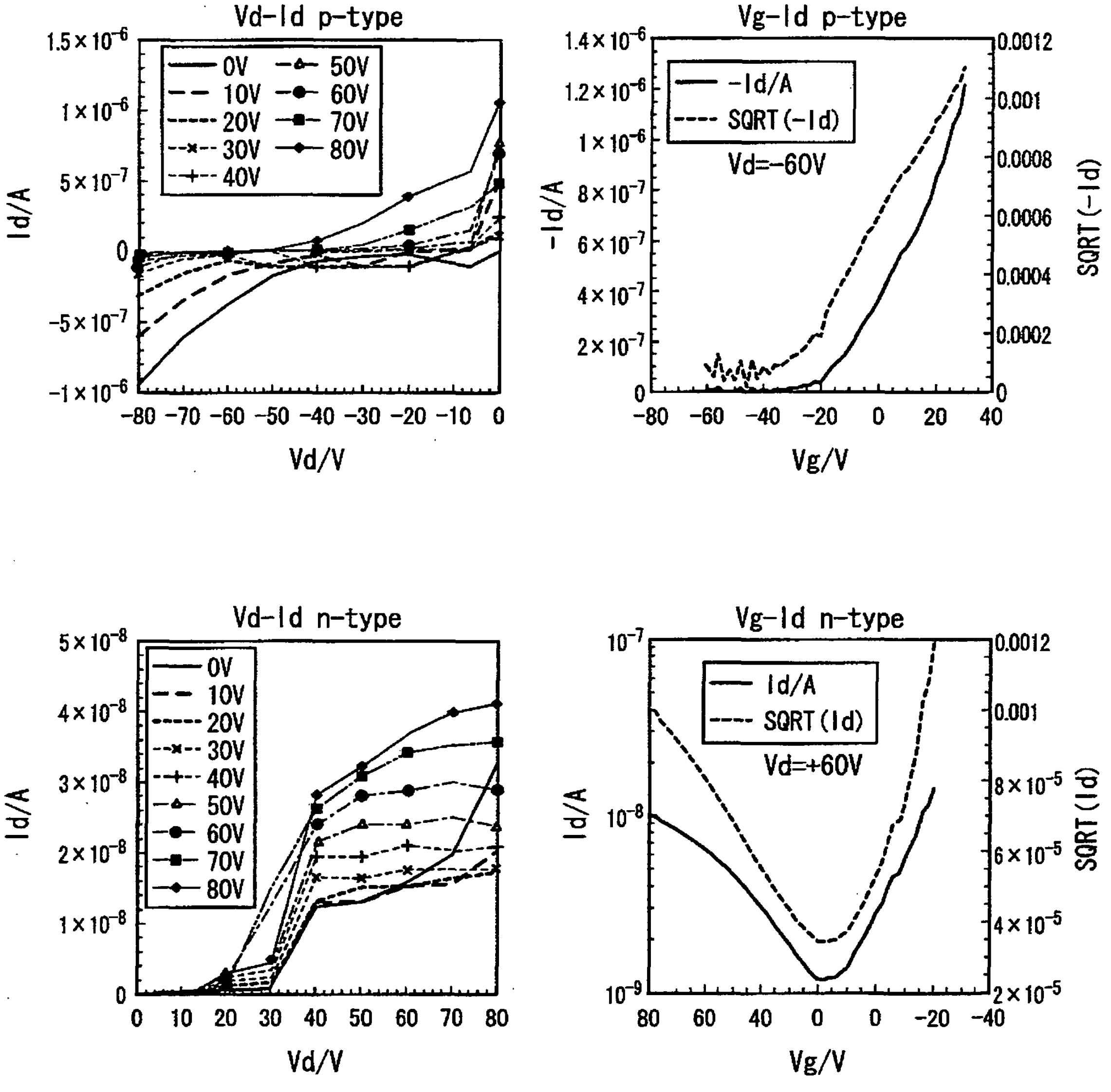

Fig. 10

FET CHARACTERISTIC OF COMPOUND 18

SOLUTION CONCENTRATION : 0.4 wt. %
SOLVENT : PURIFIED CHLOROFORM
SPIN-COATING CONDITION : 2000 rpm , 30 sec

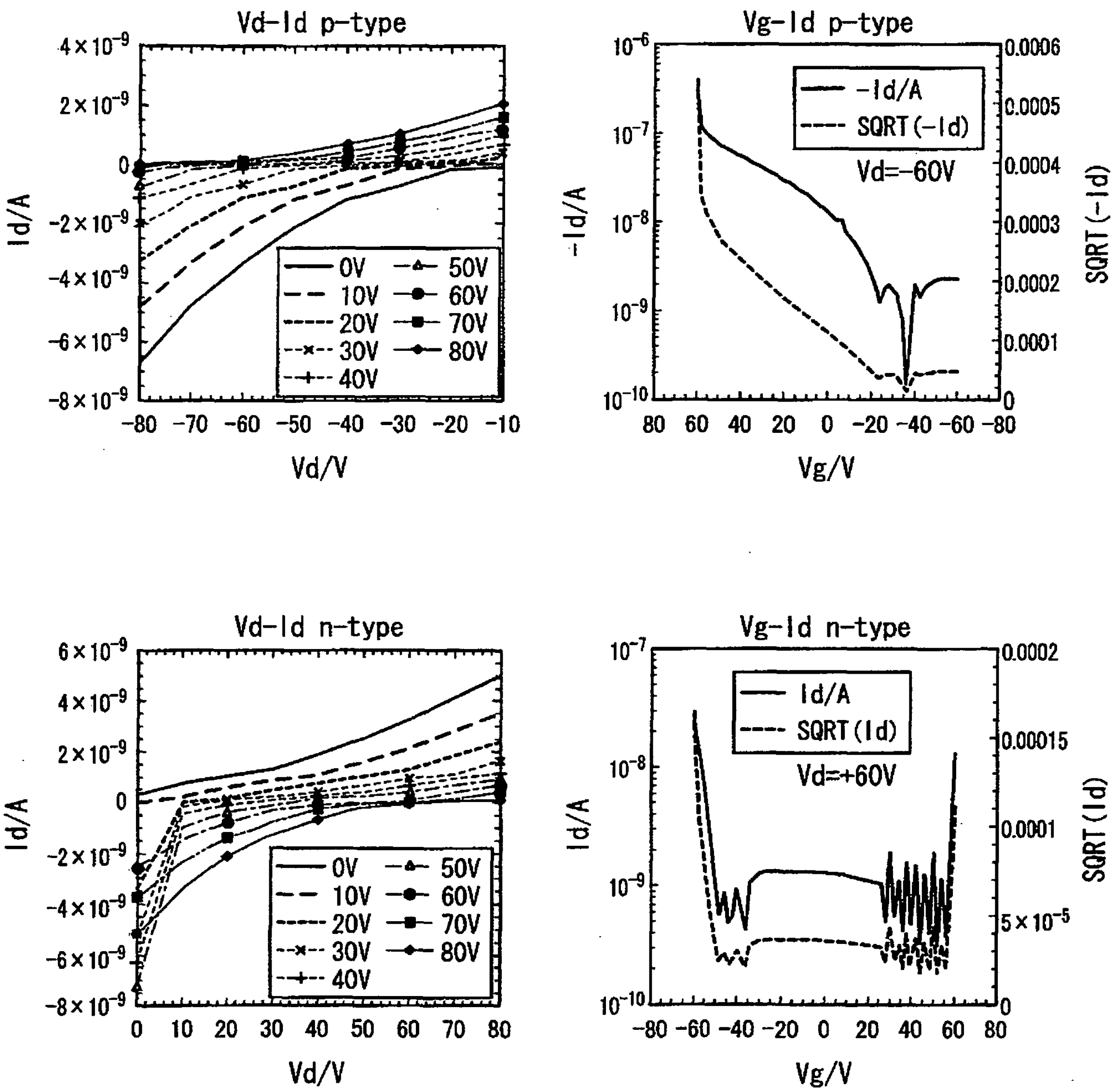

Fig. 1 1

FET CHARACTERISTIC OF COMPOUND 30

SOLUTION CONCENTRATION : 0.4 wt. %
SOLVENT : PURIFIED CHLOROFORM
SPIN-COATING CONDITION : 2000 rpm , 30 sec

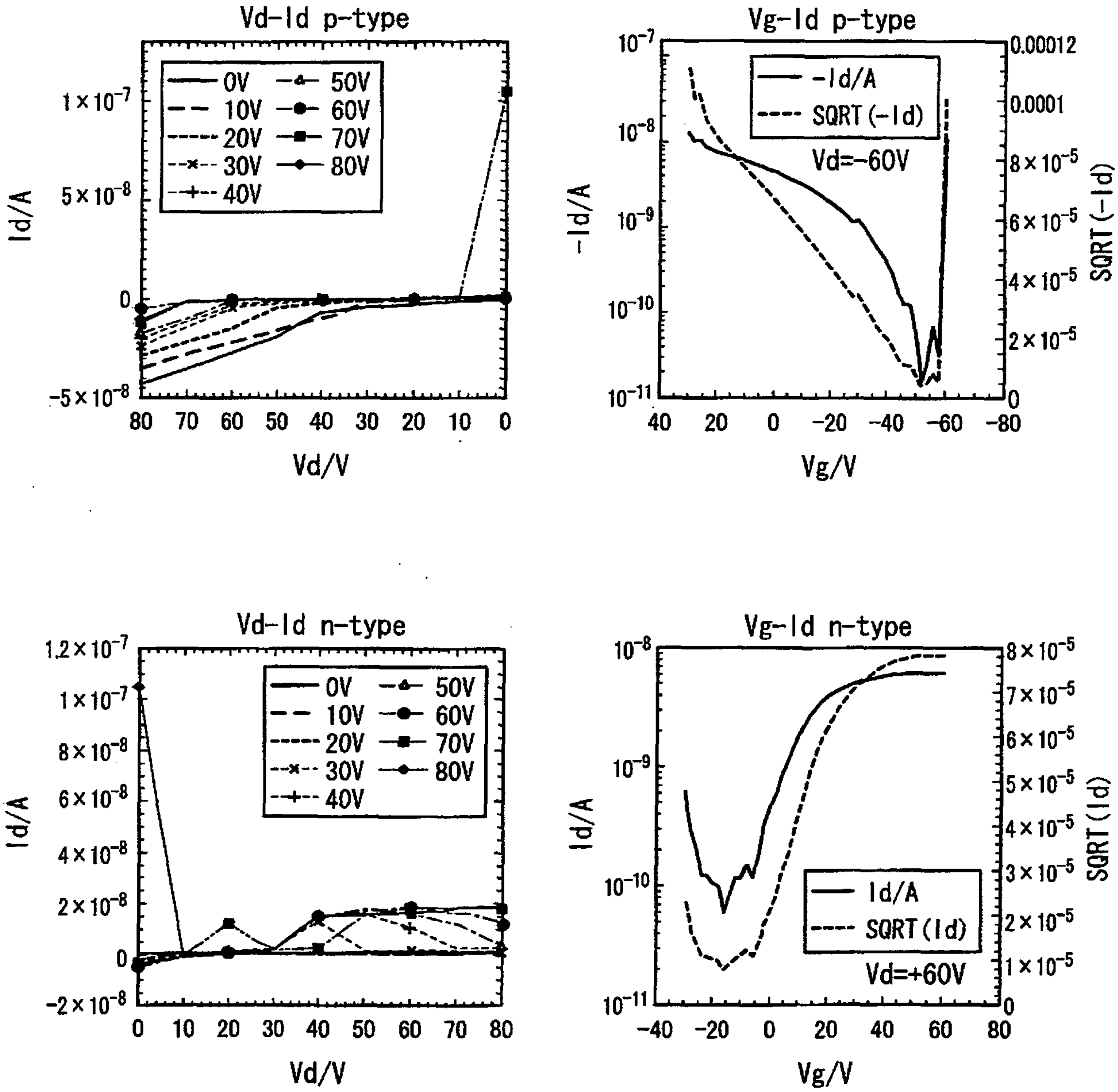

Fig. 12

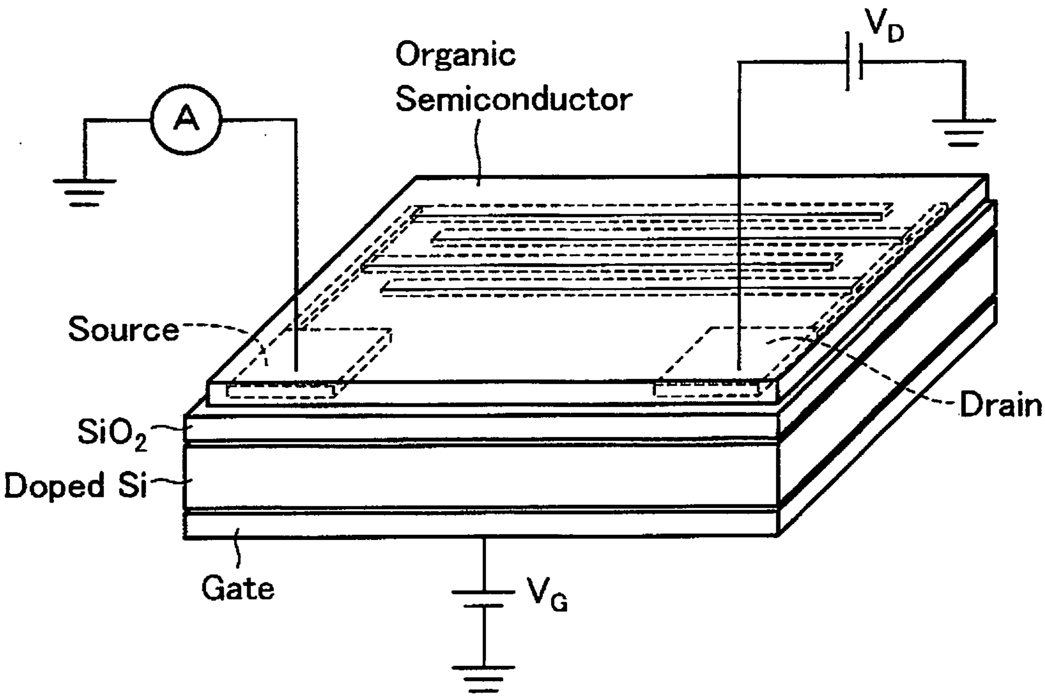
Organic Semiconductor
A
$V_D$
Source
Drain
$SiO_2$
Doped Si
Gate
$V_G$

Fig. 13

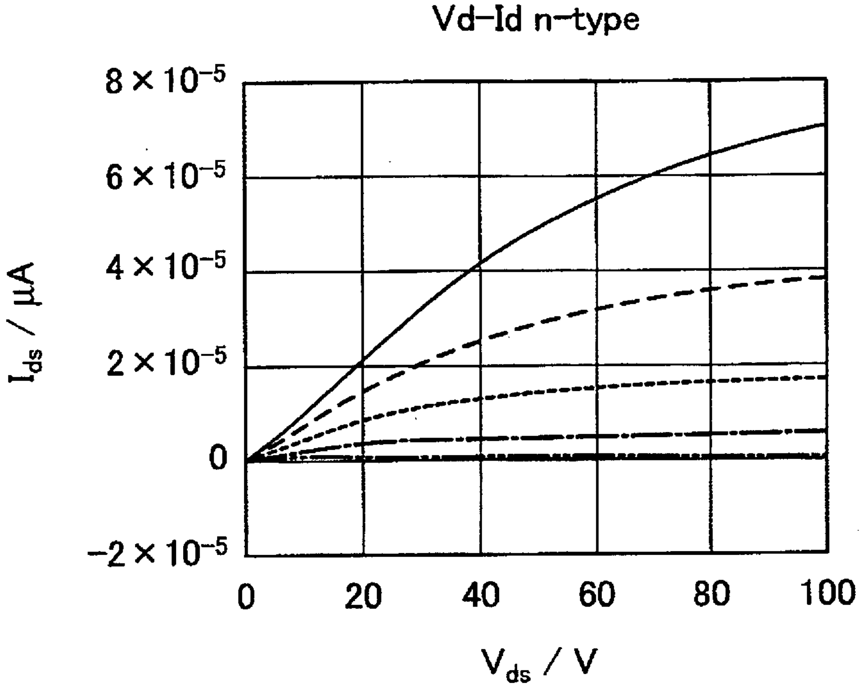
Vd-Id n-type
8×10⁻⁵
6×10⁻⁵
4×10⁻⁵
2×10⁻⁵
0
−2×10⁻⁵
0
20
40
60
80
100
Ids / μA
Vds / V

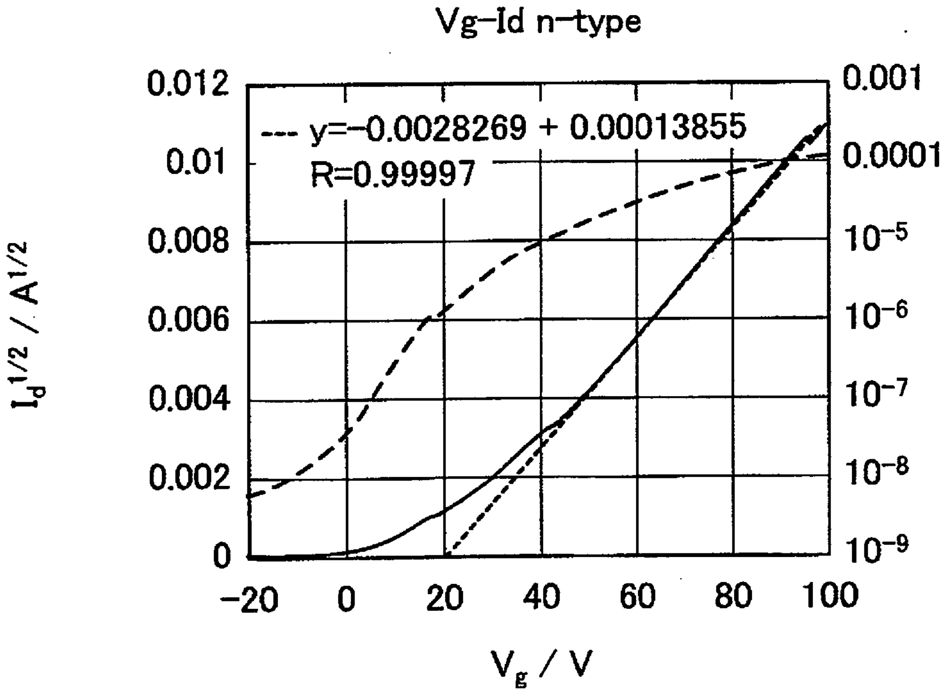
Vg-Id n-type
y=−0.0028269 + 0.00013855
R=0.99997
0.012
0.01
0.008
0.006
0.004
0.002
0
0.001
0.0001
10⁻⁵
10⁻⁶
10⁻⁷
10⁻⁸
10⁻⁹
−20
0
20
40
60
80
100
Id1/2 / A1/2
Id / A
Vg / V

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006028055 A
- JP 2006114701 A
- JP 10214044 A
- JP 2002289878 A
- JP 2005150705 A
- JP 2006060104 A
- JP 2004047752 A
- JP 2006199909 A


### Non-patent literature cited in the description


- **PAPPENFUS, T.M. ; CHESTERFIELD, R.J. ; FRIESBIE, C.D. ; MANN, K.R. ; CASADO, J. ; RAFF, J.D. ; MILLER, L.L.** *J. Am. Chem. Soc.,* 2002, vol. 124, 4184-4185
- **CHESTERFIELD, R.J. ; NEWMAN, C.R. ; PAPPENFUS, T.M. ; EWBANK, P.C. ; HAUKAAS, M.H. ; MANN, K.R. ; MILLER, L.L. ; FRISBIE, C.D.** *Adv. Mater.,* 2003, vol. 15, 1278-1282
- **T. TAKAHASHI ; K. MATSUOKA ; K. TAKIMIYA ; T. OTSUBO ; Y. ASO.** Extensive Quinoidal Oligothiophenes with Dicyanomethylene Groups at Terminal Positions as Highly Amphoteric Redox Molecules. *J. Am. Chem. Soc.,* 2005, vol. 127 (25), 8928-8929